(19) Europäisches Patentamt
European Patent Office
Office européen des brevets

(11) EP 1 358 183 B1

(12) EUROPEAN PATENT SPECIFICATION

(45) Date of publication and mention of the grant of the patent:
**12.04.2006 Bulletin 2006/15**

(21) Application number: **01273876.1**

(22) Date of filing: **16.11.2001**

(51) Int Cl.:
*C07D 407/12* (2006.01) *C07D 409/12* (2006.01)
*C07D 309/14* (2006.01) *C07D 307/22* (2006.01)
*A61K 31/365* (2006.01) *A61K 31/343* (2006.01)
*A61P 19/10* (2006.01)

(86) International application number:
**PCT/IB2001/002906**

(87) International publication number:
**WO 2002/088106 (07.11.2002 Gazette 2002/45)**

(54) **CYSTEINE PROTEASE INHIBITORS**
CYSTEIN PROTEASE INHIBITOREN
INHIBITEURS DE LA CYSTEINE PROTEASE

(84) Designated Contracting States:
**AT BE CH CY DE DK ES FI FR GB GR IE IT LI LU MC NL PT SE TR**
Designated Extension States:
**AL LT LV MK RO SI**

(30) Priority: **17.11.2000 US 252802 P**
**17.11.2000 US 252840 P**

(43) Date of publication of application:
**05.11.2003 Bulletin 2003/45**

(73) Proprietor: **MEDIVIR AB**
**141 44 Huddinge (SE)**

(72) Inventors:
- **QUIBELL, Martin**
**Hinton CB1 Cambridgeshire (GB)**
- **TAYLOR, Steven**
**Didcot, Oxon, OX11 7AU (GB)**
- **GRABOWSKA, Urszula,**
**Medivir UK Ltd.**
**Cambridge CB1 9PT (GB)**
- **NILSSON, Magnus,**
**Medivir AB**
**14144 Huddinge (SE)**
- **MORISSON, Veronique,**
**Medivir UK Ltd.**
**Cambridge CB1 9PT (GB)**

(74) Representative: **Dehmel, Albrecht**
**Herzogspitalstrasse 11**
**80331 München (DE)**

(56) References cited:
**WO-A-00/29408** **WO-A-00/49011**
**WO-A-00/69855** **WO-A-98/50533**
**WO-A-99/53039**

- **HANZLIK R P ET AL: "VINYLOGOUS AMINO ACID ESTERS: A NEW CLASS OF INACTIVATORS FOR THIOL PROTEASES" JOURNAL OF MEDICINAL CHEMISTRY, AMERICAN CHEMICAL SOCIETY. WASHINGTON, US, vol. 27, no. 6, 1 June 1984 (1984-06-01), pages 711-712, XP002037365 ISSN: 0022-2623**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### Field of the invention.

This invention relates to inhibitors of cysteine proteases, especially those of the papain superfamily. The invention provides novel compounds useful in the prophylaxis or treatment of disorders stemming from misbalance of physiological proteases such as cathepsin K, or pathogenic proteases such as malarial falcipain.

### Description of the related art.

The papain superfamily of cysteine proteases is widely distributed in diverse species including mammals, invertebrates, protozoa, plants and bacteria. A number of mammalian cathepsin enzymes, including cathepsins B, F, H, K, L, N and S, have been ascribed to this superfamily, and inappropriate regulation of their activity has been implicated in a number of metabolic disorders including arthritis, muscular dystrophy, inflammation, glomerulonephritis and tumour invasion. Pathogenic cathepsin like enzymes include the bacterial gingipains, the malarial falcipains I, II, III et seq and cysteine proteases from Pneumocystis carinii, Trypanosoma cruzei and brucei, Crithidia fusiculata, Schistosoma spp.

The inappropriate regulation of cathepsin K has been implicated in a number of disorders including osteoporosis, gingival diseases such as gingivitis and periodontitis, Paget's disease, hypercalcaemia of malignancy and metabolic bone disease. In view of its elevated levels in chondroclasts of osteoarthritic synovium, cathepsin K is implicated in diseases characterised by excessive cartilege or matrix degradation, such as osteoarthritis and rheumatoid arthritis. Metastatic neoplastic cells typically express high levels of proteolytic enzymes that degrade the surrounding matrix and inhibition of cathepsin K may thus assist in treating neoplasias.

WO 98/50533 describes the use of compounds according to the formula (I).

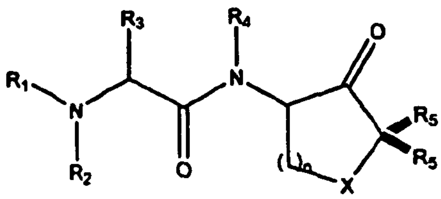

**(I)**

It is suggested the compounds of this formula, are useful as inhibitors to proteases, in particular the papain superfamily; specifically those of the Cathepsin family; and particularly Cathepsin K. The ketone bearing ring structure in these compounds has a tendency to spontaneously racemise, limiting their clinical utility. Other SKB applications describing ketone cathepsin K inhibitors include WO 98 46582, WO9964399, WO0029408, WO0038687 and W00049011. However, none of these applications disclose $\alpha$-ring substituents adjacent the linkage to the peptidomimetic chain.

Shenai et al, J Biol. Chem. 275 37 29000-29010 describes the isolation of a major cysteine protease, denoted falcipain 2 from trophozoites of Plasmodium falciparium. The enzyme appears inter alia to hydrolyse erythrocyte haemoglobin in acidic food vacuoles. This publication also describes the isolation of the corresponding gene using an N-terminus tag, which is autocatalytically removed during folding.

SmithKline Beecham's WO 99/53039 describes the cysteine protease inhibitory activity of a diverse range of peptidomimetics on a trophozoite preparation from Plasmodium falciparium. No guidance is provided as to which cysteine protease in being inhibited. Although most of the peptidomimetics are linear structures, one compound (R,S)-3-[N-(3-benzyloxybenzoyl)-L-leucinylamino]tetrahydrofuran-4-one belongs to the furanones of formula I depicted above. As would be expected of such structures, the ketone bearing ring is racemic.

Our copending PCT application WO00/69855 published after the present priority date, discloses cathepsin S inhibitors comprising a monocyclic P3 filling group.

Summary of the invention

A first aspect of the invention provides compounds of the formula (IVa) as identified in accompanying claim 1.

Compounds of the invention have utility in the treatment or prophylaxis of various disorders characterised by the presence or inappropriate activity of cysteine proteases of the papain superfamily, such as cathepsins B, F, L, S

and especially cathepsin K or falcipain.

'C1-7-alkyl' as applied herein is meant to include straight and branched chain aliphatic carbon chains such as methyl, ethyl, n-propyl, isopropyl, n-butyl, isobutyl, t-butyl, pentyl, isopentyl, hexyl, heptyl and any simple isomers thereof, Additionally, any C1-7-alkyl may optionally be substituted by one or two halogens and/or a heteroatom S. O, NH. If the heteroatom is located at a chain terminus then it is appropriately substituted with one or 2 hydrogen atoms, for example hydroxymethyl. Sulphur heteroatoms may further be oxidised to sulphones.

'C1-3-alkyl' as applied herein includes methyl, ethyl, propyl, isopropyl, cyclopropyl, any of which may be optionally substituted as described in the paragraph above.

'Amine' includes NH2, NHC1-3-alkyl or N(C1-3-alkyl)2.

'Halogen' as applied herein is meant to include F, Cl, Br, I, particularly chloro and preferably fluoro.

'C3-6-cycloalkyl' (or C3-7-cycloalkyl) as applied herein is meant to include any variation of 'C1-7-alky)' which additional contains a C3-6 (or C3-7) carbocyclic ring such as cyclopropyl, cyclobutyl, cyclopentyl, cyclohexyl. Alternatively the C3-6 or C3-7 cyclopropyl may be spiro bound to the adjacent carbon without an intervening C1-C7 alkyl.

'Ar- C1-7-alkyl' as applied herein is meant to include a phenyl, pyrazolyl, pyridyl, imidazolyl, oxazolyl, isoxazolyl, thiazinolyl, isothiazinolyl, thiazolyl, oxadiazolyl, 1,2,3-triazolyl, 1,2,4-triazolyl, furanyl or thienyl aromatic ring (Ar) attached through a 'C1-7-alkyl' (defined above) to the dihydro-(3H)-furanone ring system or in the case of R2, R3 or R4 linked directly to the molecule backbone. Optionally, the aromatic ring Ar may be substituted with halogen, C1-3-alkyl, OH, OC1-3-alkyl, SH, SC1-3-alkyl, amine and the like.

The cyclic substituent a) to R' may be saturated, unsaturated or aromatic and have 0 to 4 hetero atoms including monocyclic rings such as phenyl, cycloalkenyl, such as cyclohexenyl or cyclopentenyl, furyl, thienyl, pyranyl, pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolidinyl, isoxazolyl, isoxazolidinyl, morpholinyl, thiazolyl, thiazolidinyl, isothiazolyl, isothiazolidinyl, and the like or bicyclic rings such as napthyl and especially any of the above fused to a phenyl ring such as indolyl, quinolinyl, isoquinolinyl, benzimidazolyl, benzothiazolyl, benzoxazolyl, benzothienyl etc. The carbo or heterocyclic ring substituent may be bonded via a carbon or via a hetero atom, typically a nitrogen atom, such as N-piperidyl, N-morpholinyl etc. The ring substituent a) may itself be substituted with substituents as for Ar above. The ring substituent a) excludes cycloalkyl as defined in subgroup b).

The optional alkyl, alkylether, alkylthioether, alkylamine, alkylamide, alkylsulphonamide, alkylsulphone, alkylurea, alkyketone or alkylester linkage between R' and ring substituent a) may comprise up to 6 carbon atoms, typically up to four carbon atoms, for instance 1 or 2. If present, the ether, thioether, amine, amide, sulphonamide, sulphone, urea, ketone or ester component may be located adjacent the R' ring, (for example a morpholinoethoxy substituent) adjacent the substituent ring (for example a phenylsulphonethyl substituent) or intermediate two alkyl groups, (for example a benzoyloxymethyl substituent).

'C1-3-alkyl-CONR''', $R^{iv}$' as applied herein is meant to include straight or branched carbon chain substituted with a 1°, 2° or 3° carboxamide wherein R''', $R^{iv}$ includes H and Me.

'C1-3-alkyl-$SO_2$-$R^{ix}$, as applied herein is meant to include straight or branched carbon chain substituted with a sulphone wherein $R^{ix}$ includes 'C1-7-alkyl', 'Ar-C 1-7-alkyl', 'C3-6-cycloalkyl'.

'C1-3-alkyl-C(O)-NH$R^{ix}$, as applied herein is meant to include straight or branched carbon chain substituted with a secondary carboxamide wherein $R^{ix}$ includes 'C1-7-alkyl', 'Ar- C1-7-alkyl', 'C3-6-cycloalkyl'.

Preferred R' groups include bicyclic rings such as napthyl, quinoloyl, benzofuranyl, benzothienyl, indolyl and indolinyl, particularly where the linkage is to the 2 position of the R' ring.

Additional bicyclic groups include naphthalenyl, especially naphthylen-2-yl; benzo[1,3]dioxolyl, especially benzo [1,3]dioxol-5-yl, benzofuranyl, especially benzofuran-2-yl, and especially Cl-6 alkoxy substituted benzofuranyl, more especially 5-(2-piperazin-4-carboxylic acid tert-butyl ester- etboxy) benzofuran-2-yl, 5-(2-morpholino-4-yl-ethoxy)-benzofuran-2-yl, 5-(2-piperazin-1-yl-ethoxy)benzofuran-2-yl, 5-(2-cyclohexyl-ethoxy)-benzofuran-2-yl; 7-methoxy-benzofuran-2-yl, 5-methoxy-benzofuran-2-yl, 5,6-dimethoxy-benzofuran-2-yl, especially halogen substituted benzofuranyl, more especially 5-fluoro-benzofuran-2-yl, 5,6-difluoro-benzofuran-2-yl, especially C 1-6alkyl substituted benzofuranyl, most especially 3-methyl-benzofuran-2-yl; benzo[b]thiophenyl, especially benzo[b]thiophen-2-yl; especially Cl-6alkoxy substituted benzo[b]thiopheny], more especially 5,6-dimethoxybenzo[b]thiophen-2-yl quinolinyl, especially quinolin-2-yl, quinolin-3-yl, quinolin-4-yl, quinolin-6-yl, and quinolin-S-yl; quinoxalinyl, especially quinoxalin-2-yl; 1,8 naphthyridinyl, especially 1,8 naphthyridin-2-yl; indolyl, especially indol-2-yl, especially indol-6-yl, indol-5-yl, especially Cl-6alkyl substituted indolyl, more especially N-methylindol-2-yl; furo[3,2-b]pyridinyl, especially furo[3,2-b]pyridin-2-yl, and Cl-6alkyl substituted furo[3,2-b]pyridinyl, especially 3-methyl-furo[3,2-blpyridin-2-yl; thieno[3,2-b]thiophene, especially thieno[3,2-b]thiophene-2-yl, more especially Cl 6alkyl substituted thieno[3,2-b]thiophene-2-yl, more especially 5-tert-buty]-3-methylthieno[3,2-b]thiophene-2-yl.

Monocyclic R' groups include substituted pyridyl, substitute pyrimidyl, substituted phenyl, particularly phenyl substituted with a cyclic group such as pyrrolidine-1-yl, piperidine-1-yl, morpholin-4-yl, 4-methylpiperazin-1-yl, 2-morpholin-4-yl-ethylamino, and piperazin-1-yl. A phenyl R' is conveniently substituted at the 3 or 4 position with such a cyclic

group.

If a chiral centre is present, all isomeric forms are intended to be covered. Both (R) and (S) stereochemistries at the position corresponding to the furan 5-position (ie R5 adjacent the linkage to the peptidomimetic chain) are encompassed by the invention with (R) being convenient in some cases, for instance in cathepsin K or falcipain inhibitors, particularly in conjunction with R stereochemistry at the pyranone 4 bond. Alternatively R5 and the furanone/pyranone C4-bond conveniently both have the S stereochemistry.

The compounds of the invention are cysteine protease inhibitors, notably against cathepsins or cathepsin-like proteases of the papain superfamily.

Ideally the compound displays selective inhibition of a single protease in the complex mixture of proteolytic enzymes characterising the physiological environment, for example a greater than 10-fold selectivity, preferably greater than 100 fold. Most preferably inhibitory specificity is exhibited over other members of the same enzyme class or family, such as the Cathepsin family, which have a high degree of homology, as incorrect regulation of proteolytic, activity can lead to unwanted pathological conditions such as hypertension, blood clotting or worse. This is especially desirable for disorders such as autoimmune disorders where administration of the drug is likely to be protracted.

However, compounds can be useful notwithstanding that they exhibit a degree of promiscuity in relation to inhibition of physiological proteases. For example the physiological functions of many cathepsins are redundant, that is inhibition of a particular cysteine protease can be compensated by the presence or upregulation of other non-inhibited proteases or alternative metabolic routes. Alternatively, treatments of short duration can result only in transient toxicity or other side effects.

The cross-specificity of cysteine proteases for a given putative inhibitor (ie the selectivity of the inhibitor) is readily ascertained with conventional enzyme and cell culture assays performed in parallel with the respective enzymes.

The invention further provides the use of the compounds of formula ($IV^a$) in the manufacture of a medicament for the treatment or prophylaxis of diseases or conditions alleviated or moderated by inhibition of cathepsin K,

A further aspect of the invention provides the use of the compounds of formula (IVa) in the manufacture of a medicament for the treatment or prophylaxis of a parasitical infection such as a protozoal or bacterial infection.

Conveniently the protozoal or bacterial parasite is a Plasmodium, Leishmania, Schistosoma, Giardia, Entamoeba, Trypasoma, Crithidia, Pneumocystis or Porphyromonas species.

Suitably, the treatment or prophylaxis of Plasmodium falciparium comprises inhibition of a falcipain II enzyme.

Preferred R3 groups for parasite treatment and prophylaxis include 2-methylpropen-1-yl; or isobutyl or benzyl, especially with the stereochemistry corresponding to the side chain of L-leucine and L-phenylalanine.

Preferred R3 groups for cathepsin K inhibition include the sidechain corresponding to L-leucine.

The R5 substituent confers many beneficial qualities to molecules of general formula (IVa) including improvements in potency and offers the potential to append inhibitor molecules with a basic functionality to improve solubility and pharmacokinetic properties, It should be remembered that many cathepsins such as cathepsin K and falcipain are active in acidic vacuoles or physiological microenvironments which may favour basic functionality at this position Additionally, motecules of formula (IVa) where R5 is alkyl or other substituent and not simply hydrogen tend to show good chiral stability at the furanone (or corresponding for the pyranone) $\alpha$-carbon (denoted ring position 4 or C4 herein, unless the context requires otherwise). By chirally stable is meant that the compounds of the invention exist as a predominant stereoisomer rather than an equal mixture of stereoisomers differing in stereochemistry at C4. Preferably the compounds of the invention are at least 90% diastereomically pure.

Note particularly the presence of the substituent R5 in formula (IVa) in comparison with the absence of any substituent in the same position in formula (I) according to WO 98/50533, WO 98/46582. WO99/64399, WO00/29408, WO00/38687 and WO00/49011.

Interesting compounds of formula (IVa), particularly in the context of cathepsin K inhibition include:

*N*-[3-Methyl-1S-(2R-methyl-4-oxo-tetrahydro-furan-3S-ylcarbamoyl)-butyl]-2-phenethyl-benzamide
Benzofuran-2-carboxylic-acid-[3-methyl-1S-(2R-methyl-4-oxo-tetrahydro-furan-3S-ylcarbamoyl)-pentyl]-amide
Benzofuran-2-carboxylic acid [1S-(2R-methyl-4-oxo-tetrahydro-furan-3S-ylcarbamoyl)-cyclohexyl]-amide
Benzofuran-2-carboxylic-acid-[1S-(2R-methyl-4-oxo-tetrahydro-furan-3S-ylcarbamoyl)-cyclopentyl]-amide
Naphthalene-2-carboxylic-acid-[1S-(2R-methyl-4-oxo-tetrahydro-furan-3S-ylcarbamoyl)-cyclohexyl-amide
Benzofuran-2-carboxylic-acid-[2-cyclopropyl- 1S-(2R-methyl-4-oxo-tetrabydrofuran-3S-ylcarbamoyl)-ethyl]-amide
*N*-[3-Methyl-1S-(2R -methyl-4-0xo-tetrahydro- furan-3S-ylcarbamoyl)-butyl]-4-pyrrol-1-yl-benzamide
*N*-[3-Methyl-1S-(2R-methyl-4-oxo-tetrahydro-furan-3S-ylcarbamoyl)-butyl]-4-ptperidin-1-yl-benzamide
*N*-[3-Methyl-1S-(2R-methyl-4-oxo-tetrahydro-furan-3S-ylcarbamoyl)-butyl]-4-morpholin-4-yl-benzamide
*N*-[3-Methyl-1S-(2R-methyl-4-oxo-tetrahydro-furan-3S-ylcarbamoyl)-butyl]-4-piperazin-1-yl-benzamide
*N*-[3-Methyl-1S-(2R-methyl-4-oxo-tetrabydro-furan-3S-ylcarbarnoyl)-butyl]-4-(4-methyl-piperazin-l-yl)-benzamide
*N*-[3-Methyl-1S-(2R-methyl-4-oxo-tetrahydro-furan-3S-ylcarbamoyl)-butyl]4-pyrrolidin-1-yl-benzamide
4-(3,3-Dimethyl-piperazin-1-yl)-*N*-[3-methyl- S-(2R-methyl-4-oxo-tetrahydrofuran-3 S-ylcarbamoyl)-butyl]-benzamide
4-(2,2-Dimethyl-piperazin-1-yl)-*N*-[3-methyl-1S-(2R-methyl-4-oxo-tetrahydrofuran-3S-ylcarbamoyl)-butyl]-benzamide
4-(4-Allyl-piperazin-1-yl)-*N*-[3-methyl-1S-(2R-methyl-4-oxo-tetrahydrofuran-3S-ylcarbamoyl)-butyl]-benzamide
4-(4-Cyclopropylmethyl-piperazin-1-yl)-*N*-[3-methyl-1S-(2R-methyl-4-oxo -tetrahydro-furan-3S-ylcarbamoyl)-butyl]-benzamide
1,2,3,4-Tetrahydro-quinoline-6-carboxylic-acid-[3-methyl-1S-(2R-methyl-4-oxo-tetrahydro-furan-3S-ylcarbamoyl)-butyl]-amide
Benzothiazole-5-carboxylic-acid-[3-methyl-1S-(2R-methyl-4-oxo-tetrahydrofuran-3S-ylcarbamoyl)-butyl]-amide
4 Azepan-1-yl-*N*-[3-methyl-1S-(2R-methyl-4-oxo-tetrahydro-furan-3S-ylcarbamoyl)-butyl]-benzamide
4-[1,4]Diazepan-1-yl-*N*-[3-methyl-1S-(2R-methyl-4-oxo-tetrahydro-furan-3S-ylcarbamoyl)-butyl]-benzamide
4-(2-Methylamino-ethylamino)-*N*-[3-methyl-1S-(2R-methyl-4-oxo-tetrahydrofuran-3S-ylcarbamoyl)-butyl]-benzamide
Naphthalene-1-carboxylic-acid-[3-methyl-1S-(2R-methyl-4-oxo-tetrahydrofuran-3S-ylcarbamoyl)-butyl]-amide
Benzofuran-2-carboxylic-acid-[3-methyl-1S-(2R-methyl-4-oxo-tetrahydrofuran-3S-ylcarbamoyl)-butyl]-amide
Benzo[*b*]thiophene-2-carboxylic-acid-[3-methyl-1S-(2R-methyl-4-oxotetrahydro-furan-3S-ylcarbamoyl)-butyl]-amide
5-Methoxy-benzofuran-2-carboxylic-acid-[3-methyl-1S-(2R-methyl-4-oxotetrahydro-furan-3S-ylcarbamoyl)-butyl]-amide
5-Methoxy-benzofuran-2-carboxylic-acid-[3-methyl-1S-(2R-methyl-4-oxotetrahydro-furan-3-ylcarbamoyl)-but-3S-enyl]-amide
4-Acetylamino-*N*-[3-methyl-1S-(2R-methyl-4-oxo-tetrahydrofuran-3S-ylcarbamoyl)-butyl]-benzamide
*N*-[3-Methyl-1S-(2R-methyl-4-oxo-tetrahydro-furan-3S-ylcarbamoyl)-butyl]4-morpholin-4-ylmethyl-benzamide
*N*-[3-Methyl-1S-(2R-methyl-4-oxo-tetrahydro-furan-3S-ylcarbamoyl)-butyl]-4-piperidin-1-ylmethyl-benzamide
Piperidine-1-carboxylic-acid-{4-[3-methyl-1S-(2R-methyl-4-oxo-tetrahydrofuran-3S-ylcarbamoyl)-butylcarbamoyl]-phenyl}-amide
*N*-[3-Methyl-1S-(2R-methyl-4-oxo-tetrahydro-furan-3S-ylcarbamoyl)-but-3-enyl]-*N'*-phenyl-terephthalamide
*N*-[3-Methyl-1S-(2R-methyl-4-oxo-tetrahydro-furan-3S-ylcarbamoyl)-butyl]-*N'*-phenyl-terephthalamide
*N*-Ethyl-*N'*-[3-methyl-1S-(2R-methyl-4-oxo-tetrahydro-furan-3*S*-ylcarbamoyl)-but-3-enyl]-terephthalanude
*N*-Ethyl-*N'*-[3-methyl-1S-(2R-methyl-4-oxo-tetrahydro-furan-3S-ylcarbamoyl)-butyl]-terephthalamide
4-Hydroxy-*N*-[3-methyl-1S-(2R-methyl-4-oxo-tetrahydro-furan-3S-ylcarbamoyl)-butyl]-3-morpholin-4-ylmethyl-benzamide
4-Hydroxy-*N*-[3-methyl-1S-(2R-methyl-4-oxo-tetrahydro-furan-3S-ylcarbamoyl)-but-3-enyl]-3-morpholin-4-ylmethyl-benzamide
Biphenyl-4-carboxylic-acid-[3-methyl-1S-(2R-methyl-4-oxo-tetrahydrofuran-3S-ylcarbamoyl)-butyl]-amide
4-*tert*-Butyl-*N*-[3-methyl-1S-(2R-methyl-4-oxo-tetrahydro-furan-3S-ylcarbamoyl)-butyl]-benzamide
4-*tert*-Butyl-*N*-[3-methyl-1S-(2R-methyl-4-oxo-tetrahydro-furan-3S-ylcarbamoyl)-but-3-enyl]-benzamide
4-Guanidino-*N*-[3-methyl-1S-(2R-methyl-4-oxo-tetrahydro-furan-3S-ylcarbamoyl)-butyl]-benzamide
4-Guanidino-*N*-[3-methyl-1S-(2R-methyl-4-oxo-tetrahydro-furan-3S-ylcarbamoyl)-but-3-enyl]-benzamide
5-(2-Morpholin-4-yl-ethoxy)-benzofuran-2-carboxylic-acid-[3-methyl-1S-(2R-methyl-4-oxo-tetrahydro-furan-3S-ylcarbamoyl)-butyl]-amide
5-(2-Morpholin-4-yl-ethoxy)-benzofuran-2-carboxylicacid-[3-methyl-1S-(2R-methyl-4-oxo-tetrahydro-furan-3S-ylcarbamoyl)-but-3-enyl]-amide
Naphthalene-2-carboxylic-acid-[3-methyl-1S-(2R-methyll-4-oxo-tetrahydrofuran-3S-ylcarbamoyl)-butyl]-amide
4-Benzenesulfonylamino-*N*-[3-methyl-1S-(2R-methyl-4-oxo-tetrahydro-furan-3 S-ylcarbamoyl)-butyl]-benzamide
3,4,5,6-Tetrahydro-2*H*-[1,4']bipyridinyl-4-carboxylic-acid-[3-methyl-1S-(2R-methyl-4-oxo-tetrahydro-furan-3S-ylcarbamoyl)-butyl]-amide
4-(1-Methyl-4,5-dihydro-1*H*-imidazol-2-yl)-*N*-[3-methyl-1S-(2R-methyl-4-oxo-tetrahydro-furan-3S-ylcarbamoyl)-butyl]-benzamide
4-(Benzyl-methyl-amino)-*N*-[3-methyl-1S-(2R-methyl-4-oxo-tetrahydrofuran-3 S-ylcarbamoyl)-butyl]-benzamide
*N*-[3-Methyl-1S-(2R-methyl-4-oxo-tetrahydro-furan-3S-ylcarbamoyl)-butyl]-4-phenylamino-benzamide
4-Benzylamino-*N*-[3-methyl-1S-(2R-methyl-4-oxo-tetrahydro-furan-3S-ylcarbamoyl)-butyl]-benzamide
1-Methyl-1,2,3,4-tetrahydro-quinoline-6-carboxylic-acid-[3-methyl-1S-(2R-methyl-4-oxo-tetrahydro-furan-3S-ylcarbamoyl)-butyl]-amide

and the corresponding R5 hydroxymethyl compounds;
and pharmaceutically acceptable salts thereof.

The compounds of the invention can form salts which form an additional aspect of the invention. Appropriate

pharmaceutically acceptable salts of the compounds of Formula (IVa) include salts of organic acids, especially carboxylic acids, including but not limited to acetate, trifluoroacetate, lactate, gluconate, citrate, tartrate, maleate, malate, pantothenate, isethionate, adipate, alginate, aspartate, benzoate, butyrate, digluconate, cyclopentanate, glucoheptanate, glycerophosphate, oxalate, heptanoate, hexanoate, fumarate, nicotinate, palmoate, pectinate, 3-phenylpropionate, picrate, pivalate, proprionate, tartrate, lactobionate, pivolate, camphorate, undecanoate and succinate, organic sulphonic acids such as methanesulphonate, ethanesulphonate, 2-hydroxyethane sulphonate, camphorsulphonate, 2-napthalenesulphonate, benzenesulphonate, p-chlorobenzenesulphonate and p-toluenesulphonate; and inorganic acids such as hydrochloride, hydrobromide, hydroiodide, sulphate, bisulphate, hemisulphate, thiocyanate, persulphate, phosphoric and sulphonic acids. The compounds of Formula (IVa) may in some cases be isolated as the hydrate.

It will be appreciated that the invention extends to prodrugs solvates, complexes and other forms releasing a compound of formula (IVa) in vivo.

While it is possible for the active agent to be administered alone, it is preferable to present it as part of a pharmaceutical formulation. Such a formulation will comprise the above defined active agent together with one or more acceptable carriers/excipients and optionally other therapeutic ingredients. The carrier(s) must be acceptable in the sense of being compatible with the other Ingredients of the formulation and not deleterious to the recipient.

The formulations include those suitable for rectal, nasal, topical (including buccal and sublingual), vaginal or parenteral (including subcutaneous, intramuscular, intravenous and intradermal) administration, but preferably the formulation is an orally administered formulation. The formulations may conveniently be presented in unit dosage form, e.g. tablets and sustained release capsules, and may be prepared by any methods well known in the art of pharmacy.

Such methods include the step of bringing into association the above defined active agent with the carrier. In general, the formulations are prepared by uniformly and intimately bringing into association the active agent with liquid carriers or finely divided solid carriers or both, and then if necessary shaping the product. The invention extends to methods for preparing a pharmaceutical composition comprising bringing a compound of Formula (IVa) or its pharmaceutically acceptable salt in conjunction or association with a pharmaceutically acceptable carrier or vehicle. If the manufacture of pharmaceutical formulations involves intimate mixing of pharmaceutical excipients and the active ingredient in salt form, then it is often preferred to use excipients which are non-basic in nature, i.e. either acidic or neutral:

Formulations for oral administration in the present invention may be presented as discrete units such as capsules, cachets or tablets each containing a predetermined amount of the active agent; as a powder or granules; as a solution or a suspension of the active agent in an aqueous liquid or a non-aqueous liquid; or as an oil-in-water liquid emulsion or a water in oil liquid emulsion and as a bolus etc.

With regard to compositions for oral administration (e.g. tablets and capsules), the term suitable carrier includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavouring agents such as peppermint, oil of wintergreen, cherry flavouring or the like can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may be optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

The appropriate dosage for the compounds or formulations of the invention will depend upon the indication and the patient and is readily determined by conventional animal trials. Dosages providing intracellular (for inhibition of physiological proteases of the papain superamily) concentrations of the order 0.01-100 $\mu$M, more preferably .01-10 $\mu$M, such as 0.1-25$\mu$M are typically desirable and achievable. Ex vivo or topical administration against parasites will typically involve higher concentrations.

The term "N-protecting group" or "N-protected" and the like as used herein refers to those groups intended to protect the N-terminus of an amino acid or peptide or to protect an amino group against undesirable reactions during synthetic procedures. Commonly used N-protecting groups are disclosed in Greene, "Protective Groups in Organic Synthesis" (John Wiley & Sons, New York, 1981), which is hereby incorporated by reference. N-protecting groups include acyl groups such as formyl, acetyl, propionyl, pivaloyl, t-butylacetyl, 2-chloroacetyl, 2-bromoacetyl, trifluoracetyl, trichloroacetyl, phthalyl, o-nitrophenoxyacetyl, α-chlorobutyryl, benzoyl, 4-chlorobenzoyl, 4-bromobenzoyl, 4-nitrobenzoyl, and the like; sulfonyl groups such as benzenesulfonyl, p-toluenesulfonyl, and the like, carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butoxycarbonyl, diisopropylmethoxycarbonyl, isopropyloxycarbonyl, ethoxycarbonyl, methoxycarbonyl, allyloxycarbonyl, 2,2,2-trichloroethoxycarbonyl, phenoxycarbonyl, 4-nitrophenoxycarbonyl, fluorenyl-9-methoxycarbonyl, cyclopentyloxycarbonyl, adamantyloxycarbonyl, cyclohexyloxycarbonyl, phenylthiocarbonyl, and the like; alkyl gropus such as benzyl, triphenylmethyl, benzyloxymethyl and the like; and silyl groups such as trimethylsilyl and the like. Favoured N-protecting groups include formyl, acetyl, allyl, Fmoc, benzoyl, pivaloyl, t-butylacetyl, phenylsulfonyl, benzyl, t-butoxycarbonyl (Boc) and benzyloxycarbonyl (Cbz).

Hydroxy and/or carboxy protecting groups are also extensively reviewed in Greene ibid and include ethers such as methyl, substituted methyl ethers such as methoxymethyl, methylthiomethyl, benzyloxymethyl, t-butoxymethyl, 2-methoxyethoxymethyl and the like, silyl ethers such as trimethylsilyl (TMS), 1-butyldimethylsilyl (TBDMS) tribenzylsilyl, triphenylsilyl, t-butyldiphenylsilyl (TBDPS), triisopropyl silyl and the like, substituted ethyl ethers such as 1-ethoxymethyl, 1-methyl-1-methoxyethyl, t-butyl, allyl, benzyl, p-methoxybenzyl, dipehenylmethyl, triphenylmethyl and the like, aralkyl groups such as trityl, and pixyl (9-hydroxy-9-phenylxanthene derivatives, especially the chloride). Ester hydroxy protecting groups include esters such as formate, benzylformate, chloroacetate, methoxyacetate, phenoxyacetate, pivaloate, adamantoate, mesitoate, benzoate and the like. Carbonate hydroxy protecting groups include methyl vinyl, allyl, cinnamyl, benzyl and the like.

Compounds of the invention are synthesised by a combination of chemistries, performed either in solution or on the solid phase. The synthesis methodology described in schemes 1-8 of our copending PCT/GB00/01894 but employing the appropriate R' capping group is convenient for the compounds of the invention.

Scheme 1. Preparation of dihydro-2(3H)-5-alkyl furanone ring system

## Scheme 1

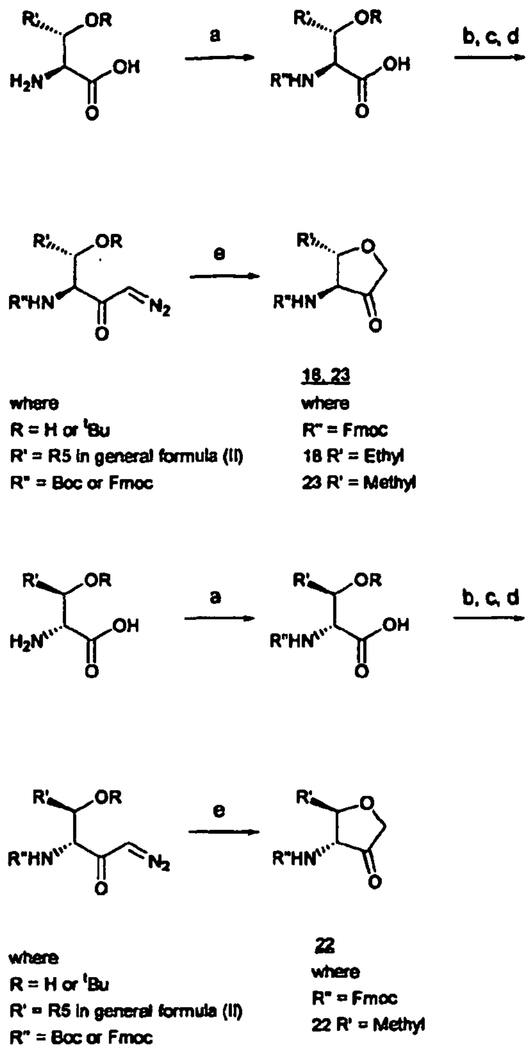


a) Fmoc-Cl, $Na_2CO_3$ or $Boc_2O$; b) $^1$BuOCOCl, NMM, THF; c) $CH_2N_2$ in $Et_2O$; d) AcOH; e) LiCl in 80% AcOH

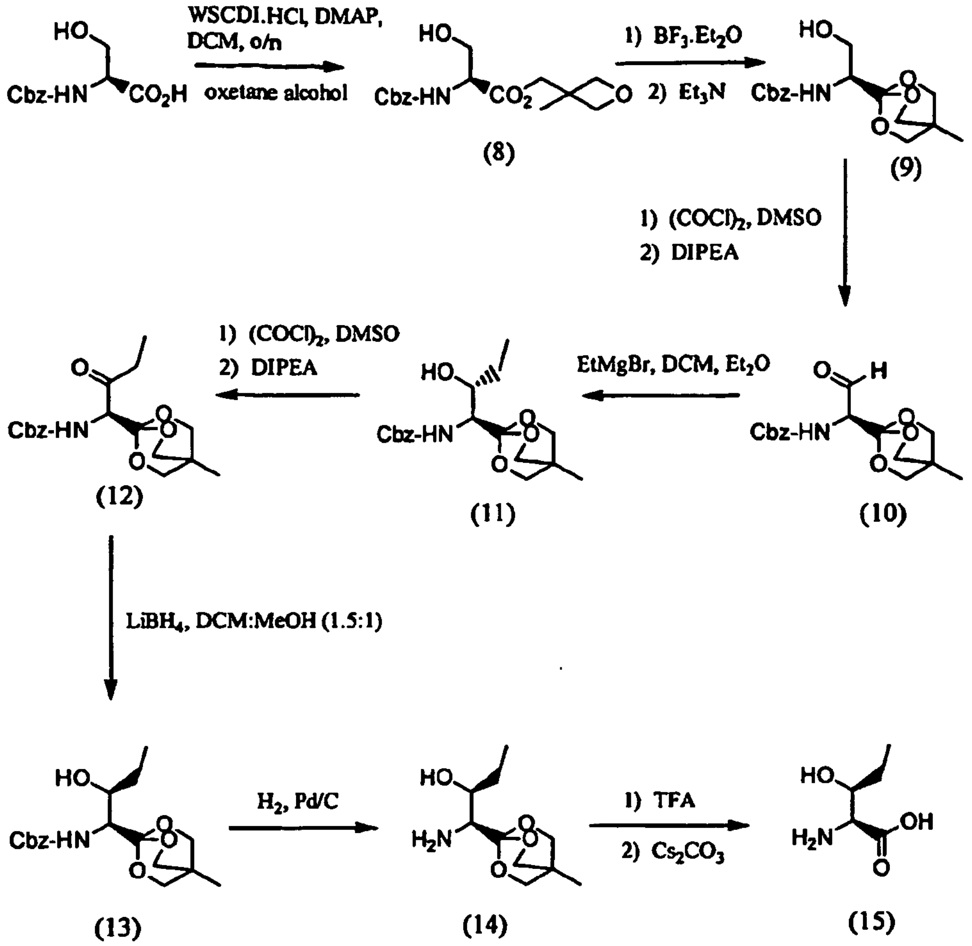


(8) (9) (10) (11) (12) (13) (14) (15)

**Scheme 2. Preparation of chiral β-alkylserine aminoacids, exemplified by (2S, 3S)-β-ethylserine (15)**

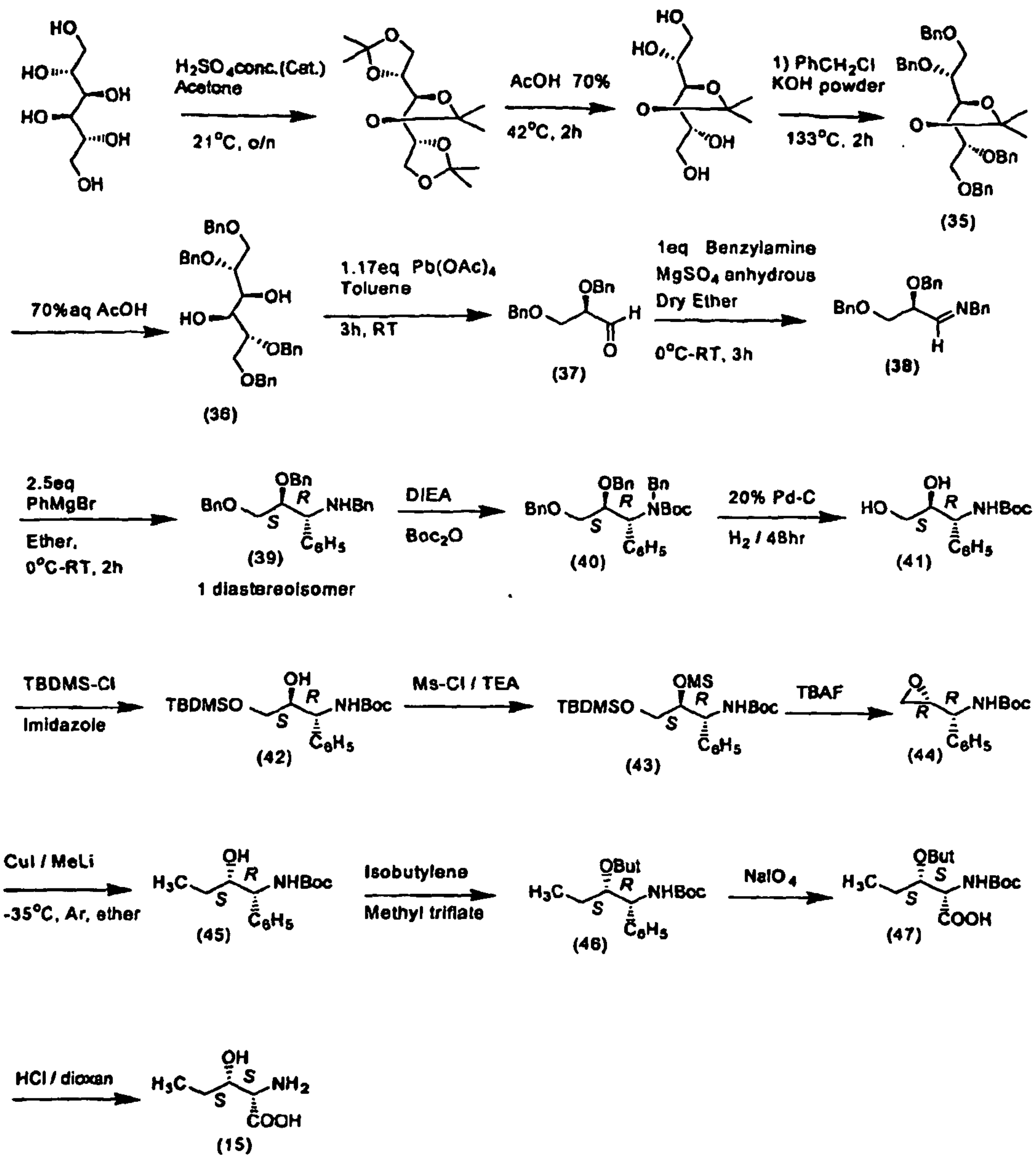


Scheme 3. Sugar route for the preparation of chiral -alkylserine aminoacids, exemplified by (2S, 3S)-β-ethylserine (15)

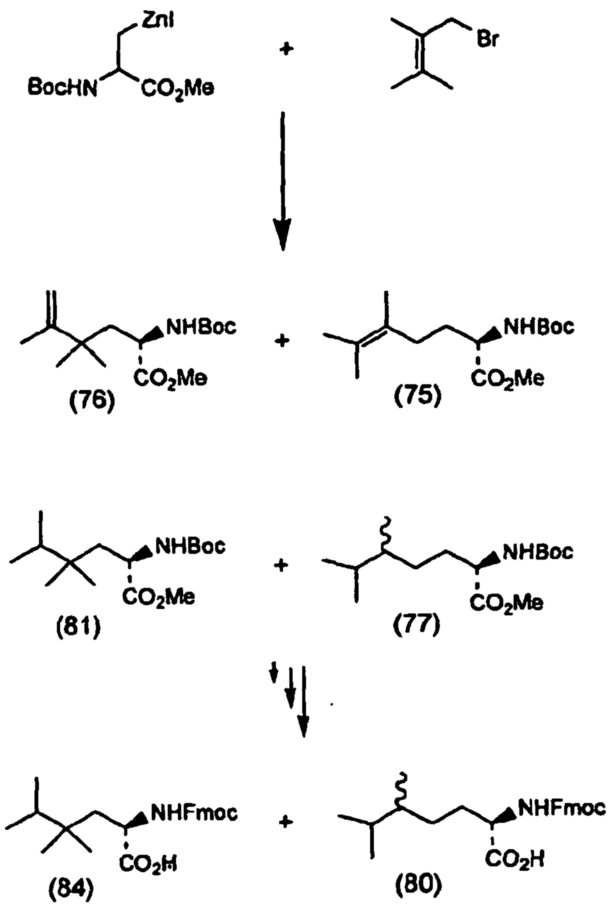

(76) (75)

(81) (77)

(84) (80)

Scheme 4. Novel P2 hybrids by the CuCN catalysed cross coupling of Zn activated -iodoalanine with allyl bromides (with permission from Dexter & Jackson ibid)

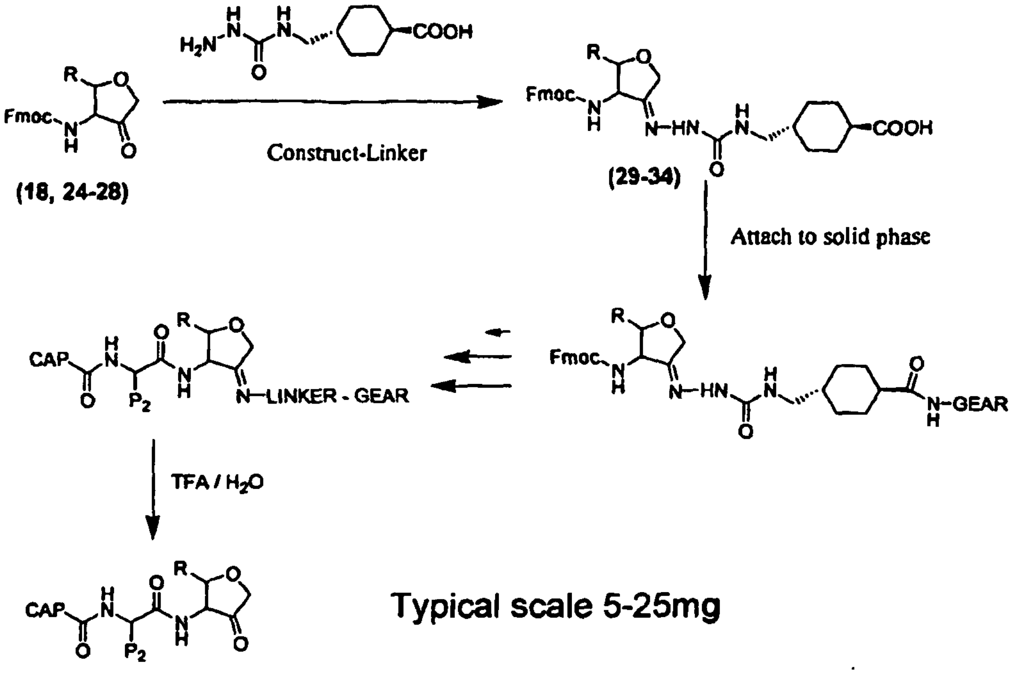


Scheme 5. Solid phase synthesis of dihydro-2(3H)-5-alkyl furanone inhibitors of cathepsin (also applicable to the corresponding pyranones)

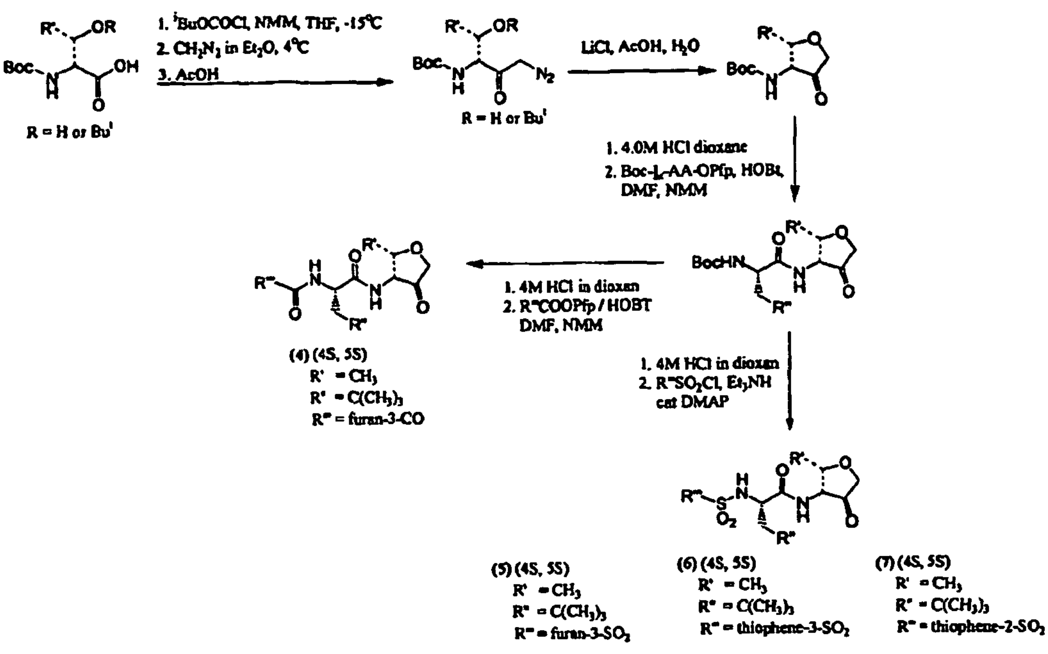


Scheme 6. Solution phase preparation of 3(2H)-furanone inhibitors

Additional routes to building blocks include:

Scheme 7

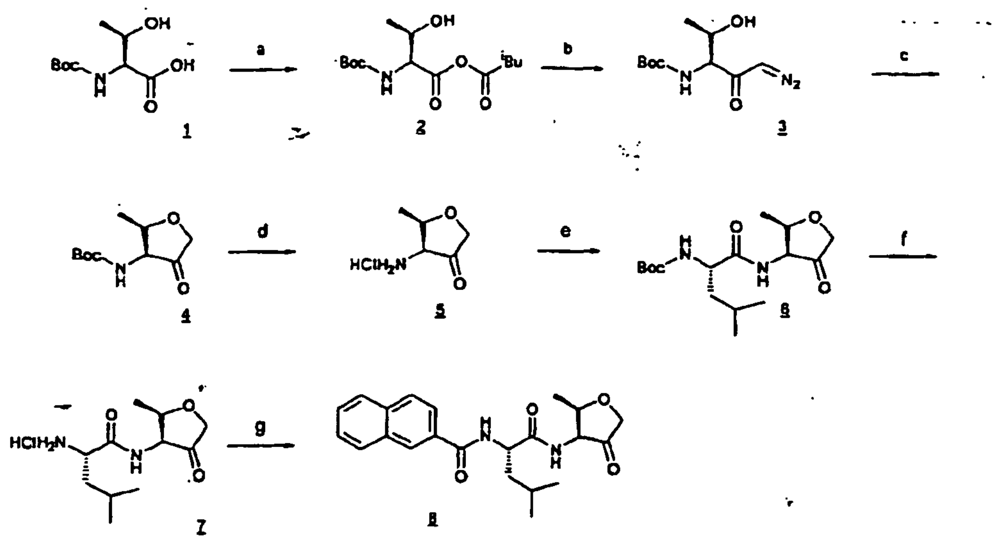


a) [1]BuOCOCl, NMM; b) diazomethane in $Et_2O$; c) LiCl (10eq) in 80 % acetic acid; d) 4M HCl in dioxane; e) Boc-Leu-Opfp, HOBt, NMM, DMF; f) 4M HCl in dioxane: g) 2-naphthoic acid, HBTU, HOBt, NMM, DMF.

Compounds of the general formula (IVa) are prepared by methods shown in Scheme 7. Activation of the known Boc-aminoacid 1-Scheme-7 with isobutyl chloroformate and 4-methylmorpholine provides 2-Scheme-7. Subsequent treatment of 2-Scheme-7 with diazomethane provides the diazoketone 3-Scheme-7. Cyclization of diazoketone 3-Scheme-7 can be effected by lithium chloride/aqueous acetic acid to give the dihydro-3(2H)-furanone 4-Scheme-7. The *tert*-butoxycarbonyl group may be removed from 4-Scheme-7, by treatment with acid, and provides the amine salt 5-Scheme-7. The amine salt 5-Scheme-7 may be coupled with a carboxylic acid by methods that are known in the art, such as coupling with a pentafluorophenol derivative in the presence of HOBT and NMM, to provide the amide 6-Scheme-7. The *tert*-butoxycarbonyl group may be removed from 6-Scheme-7 by treatment with an acid, such as hydrogen chloride in dioxane, to provide the amine salt 7-Scheme-7. The amine salt 7-Scheme-7 may be coupled with a carboxylic acid by methods that are known in the art, such as coupling with an acid in the presence of HBTU and HOBT, to provide the amide 8-Scheme-7.

Additional routes to 5-methyl and ethyl furanones as building blocks toward inhibitors or as intermediates to access other R5 functionalities are as shown in schemes 12 and 13.

## Scheme 12

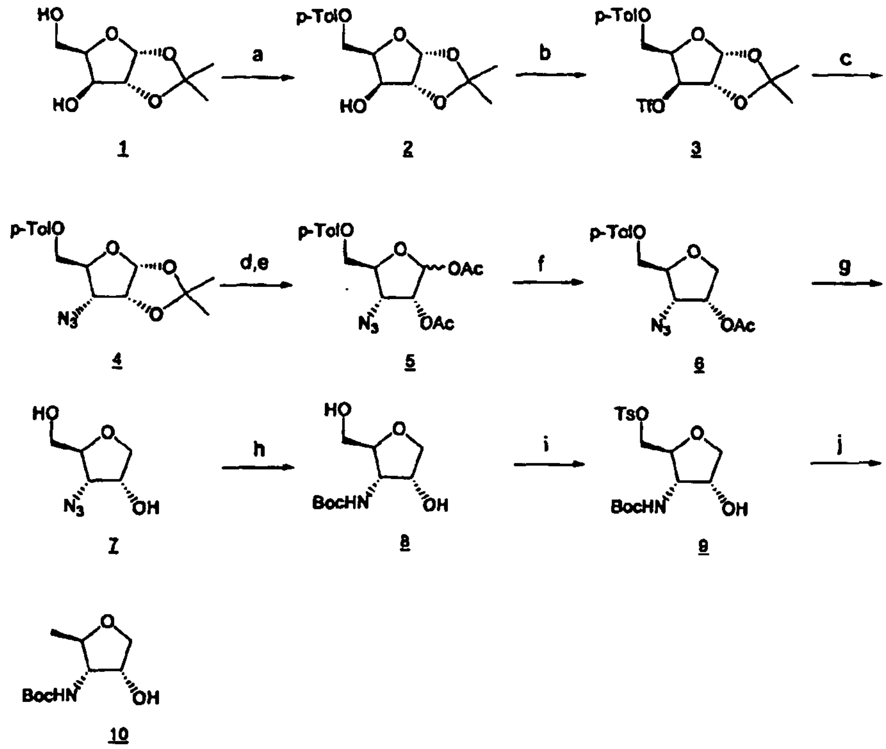


1 2 3 4 5 6 7 8 9 10

a) *p*-TolCl, pyridine; b) $(CF_3SO_2)_2O$, pyridine, DCM; c) $NaN_3$, DMF; d) 75% HCOOH; e) $Ac_2O$, pyridine; f) TMSOTf, $Et_3SiH$; g) $K_2CO_3$, MeOH; h) $H_2$, 10% Pd/C, MeOH, $Boc_2O$; i) TsCl, pyridine; j) $LiAlH_4$, $Et_2O$

An alternative synthesis of methyl furanones is shown in Scheme 12. 1,2-Isopropylidene-D-xylofuranoside 1-Scheme-12 is first converted to the p-toluoyl ester 2-Scheme-12 with p-toluoyl chloride and pyridine. The secondary alcohol 2-Scheme-12 may be converted to the triflate 3-Scheme-12. The triflate 3-Scheme-12 may be displaced with sodium azide to provide the corresponding azide 4-Scheme-12. Deprotection of the 1,2-isopropylidene of 4-Scheme-12 and subsequent acetylation of the residue provides diacetate 5-Scheme-12. Reduction of the anomeric centre of 5-Scheme-12 with trimethylsilyl triflate and triethylsilane provides monoacetate 6-Scheme-12. Removal of the two ester groups from 6-Scheme-12 with potassium carbonate affords alcohol 7-Scheme-12. Reduction of the azide 7-Scheme-12 in the presence of Boc anhydride affords the key intermediate furanol 8-Scheme-12. Furanol 8-Scheme-12 can be transformed to the methyl furanol 10-Scheme-12 by converting the primary alcohol functionality of 8-Scheme-12 to the tosylate 9-Scheme-12, which in turn can be reduced with lithium aluminium hydride to provide the methyl furanol 10-Scheme-12. As described herein, furanol 10-Scheme-12 can be used to build up inhibitors of the invention in solution or on solid phase. Solid phase chemistry would typically require conversion of the Boc protection to Fmoc chemistry. The ultimate synthetic step involves oxidation of the furanol functionality to the corresponding furanone using an oxidant such as Dess-Martin periodinane. Alternatively, the oxidation may be carried out prior to subsequent modifications at the N-terminus. Importantly, furanol 8-Scheme-12 also provides an opportunity for introduction of diverse functionality at C-5 as the hydroxmethylene can be used for subsequent transformations known to those skilled in the art.

**Scheme 13**

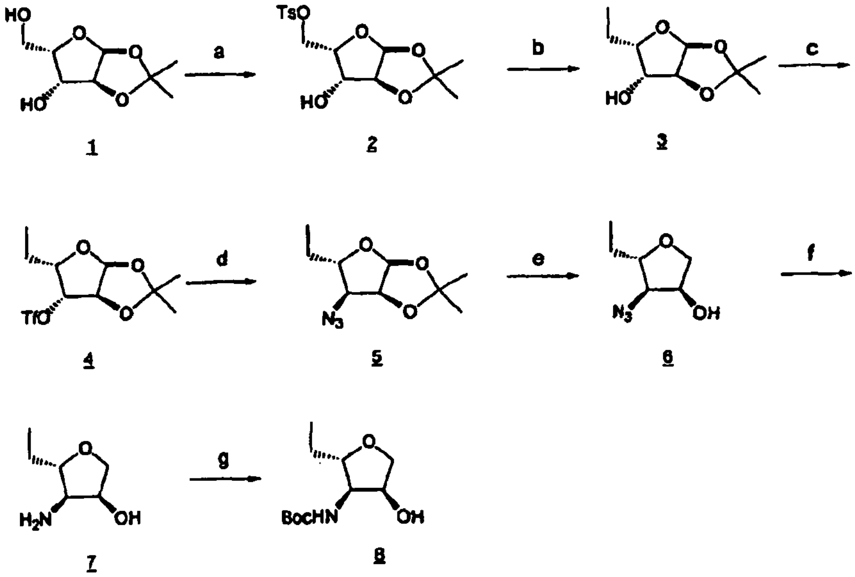


1 2 3 4 5 6 7 8

a) TsCl, pyridine; b) $Me_2CuLi$, $Et_2O$,THF; c) $(CF_3SO_2)_2O$, pyridine, DCM; d) $NaN_3$, DMF; e) TMSOTf, $Et_3SiH$; f) $H_2$, 10% Pd/C, MeOH; g) $Boc_2O$

An alternative synthesis of ethyl furanones is shown in Scheme 13. 1,2-Isopropylidene-L-xylofuranoside 1-Scheme-13 is used as the starting material and is first converted to the tosylate 2-Scheme-13. The tosylate 2-Scheme-13 is readily displaced using cuprate chemistry to provide the ethyl furanoside 3-Scheme-13. The secondary alcohol 3-Scheme-13 may be converted to the triflate 4-Scheme-13 using triflic anhydride and pyridine. The triflate 4-Scheme-13 may be displaced with sodium azide to provide the corresponding azide 5-Scheme-13. Reduction of the anomeric centre of 5-Scheme-13 with trimethylsilyl triflate and triethylsilane provides alcohol 6-Scheme-13. Reduction of the azide 6-Scheme-13 with hydrogen in the presence of 10% palladium on carbon provides amine 7-Scheme-13. Protection of the amine 7-Scheme-13 with Boc anhydride provides the ethyl furanol 8-Scheme-13. As described previously, furanol 8-Scheme-13 can be used to build up potential inhibitors in solution or on solid phase. Solid phase chemistry would require conversion of the Boc protection to Fmoc chemistry. The ultimate synthetic step involves oxidation of the furanol functionality to the corresponding furanone using an oxidant such as Dess-Martin periodinane. Alternatively, the oxidation may be carried out prior to subsequent modifications at the N-terminus.

Many R3 groups are accessed from commercially available amino acid residues such as L-leucine, L-norleucine, L-phenylaianine etc. Other branched and unsaturated amino acid building blocks are as shown in Medivir UK's PCT/GB01/02162 claiming priority from British patent application GB 00025386-4 filed 17 May 2000 the contents of which are incorporated by reference.

Access to sulphonyl bearing C1-C7alkyl or ArC1-C7alkyl R3 groups, for instance arylalkylCO-2sulphonylmethyl functionalities can come from the suitably protected amino acid cysteine. Mitsunobu coupling of the cysteinyl thiol with aryl alcohols such as phenol yield the protected amino acid containing the phenylthiomethyl R3 sidechain that is readily oxidised using m-chloroperbenzoic acid to provide the R3 sidechain phenylsulphonylmethyl. The benzylsulphonylmethyl and phenethylsulphonylmethyl R3 sidechain containing amino acids can be prepared by nucleophilic substitution of the cysteinyl thiol with benzyl bromide and phenethyl bromide respectively.

Oxidation of the resulting sulphides with m-chloroperbenzoic acid provides the suitably protected amino acids with the benzylsulphonylmethyl and phenethylsulphonylmethyl R3 sidechain.

Detailed Description of the Embodiments

Solution phase chemistry

Example 1

Following general chemistry scheme 14:

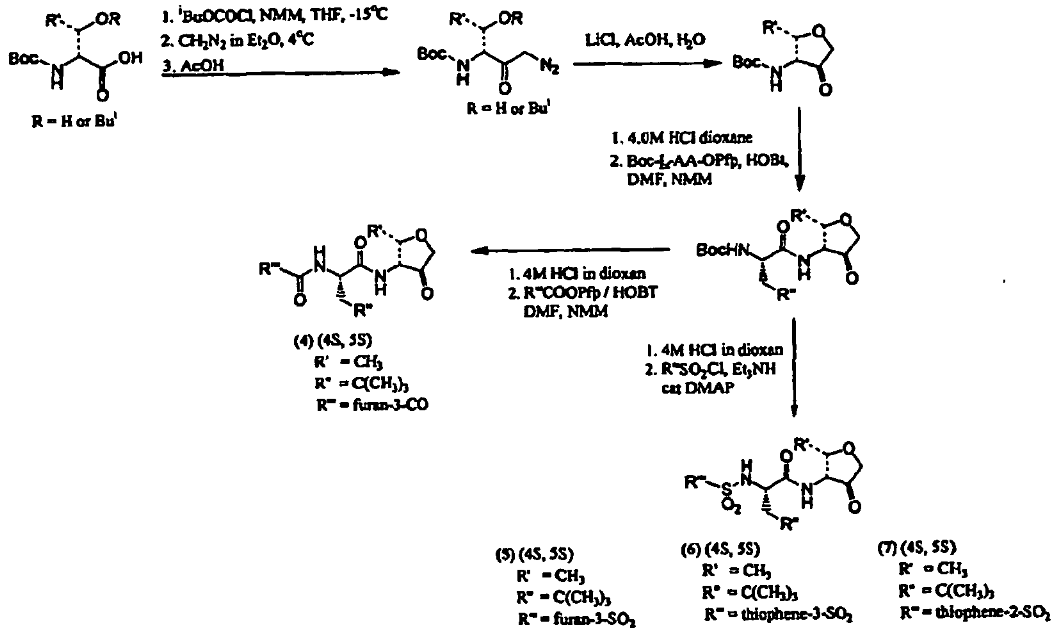


**(a)** General method for the synthesis of N-Boc protected diazoketones, exemplified by (2S, 3S)-N-Boc-O-t-butyl-L-threonyldiazomethane **(1)**

(2S. 3S)- N-Boc-O-t-butyl-L-threonine (1.2g, 4.2mmol) was dissolved in dry DCM (20mL) and N-methylmorpholine (1mL, 2.2eq) added. The reaction mixture was cooled to -15°C and stirred under an atmosphere of argon. Isobutyl chloroformate (0.56mL, 4.3mmol) was added and the mixture stirred for 10mins at -15°C. A solution of diazomethane in diethyl ether (45mL, approx 40mmol) was added and the reaction allowed to warm to room temperature over 1 hr, then acetic acid was added dropwise until effervescence had ceased. The reaction mixture was diluted with DCM (100mL) and washed successively with saturated aqueous sodium bicarbonate (2 x 75mL), water (75mL) and brine (75mL) and dried over sodium sulphate. The solvent was removed *in* vacuo to give crude (2S, 3S)-N-Boc-O-t-butyl-L-threonyldiazomethane (1.2g, ~100%) as a pale yellow oil. The above synthesis was repeated 9 times and the total crude product pooled (12g) and used without purification for the next stage.

**(b)** General method for the synthesis of Boc-3(2H)-furanones, exemplified by dihydro-(4S-amino-[N-Boc])-5S-methyl-3(2H)-furanone **(2)**

A solution of lithium chloride (13.6g, 320mmol) in 80% aqueous acetic acid (400mL) was cooled to 5°C and added to crude (2S, 3S)-N-Boc-O-t-butyt-L-threonyldiazomethane **(1)** (9.6g) with stirring. The oil dissolved over 10mins and stirring continued for a further 1 hr slowly warming to room temperature, with evolution of gas. The solvents were removed in vacuo and the residue taken into EtOAc (250mL) and washed successively with water (250mL), saturated aqueous sodium bicarbonate (2 x 100mL) and brine (75mL), then dried over sodium sulphate. The solvent was removed *in vacuo* and the crude product purified by flash chromatography over silica gel (150g) eluting with EtOAc / heptane (1: 2, v/v). Two fractions were pooled and the quicker eluting fraction reduced *in vacuo* to approx 50mL heptane and left to crystallise to give dihydro-(4S-amino-[N-Boc])-5S-methyl-3(2H)-furanone **(2)** as a white solid, yield 4.05g, 18.8mmol, 58%. Electrospray-MS m/z 216 ($MH^+$), 160 ($MH^+$ - 56), elemental analysis $C_{10}H_{17}O_4N$ (req) %C 55.80, %H 7.96, %N 6.51, (fnd) %C 55.82, %H 7.86, %N 6.44.

$\delta_H$(500 MHz; $CDCl_3$); 1.41 (9H, s, $C(C\underline{H}_3)_3$), 1.49 (3H, d, *J* 6, 5S-$C\underline{H}_3$), 3.72 (1H, bm, furanone $C\underline{H}\alpha$), 3.90-4.02 (2H, 5S-H + 1 x furanone $COC\underline{H}_2O$), 4.22 (1H, d, *J* 17.4, 1 x furanone $COC\underline{H}_2O$), 4.85 (1H, bs, furanone, N$\underline{H}$).

$\delta_c$ (125 MHz; $CDCl_3$); 19.34 (5S-$\underline{C}H_3$), 28.45 ($C(\underline{C}H_3)_3$), 62.79 (furanone $\underline{C}H\alpha$), 71.06 (furanone $CO\underline{C}H_2O$), 77.96 (5S-$\underline{C}HCH_3$), 80.88 ($\underline{C}(CH_3)_3$), 155.6 ($(CH_3)_3$ CO-$\underline{C}O$), 212.6 (furanone $\underline{C}O$).

(c) General method for N-terminal extension, exemplified by dihydro-(4S-amino-[N-Boc-L-*tert*-butylalanyl])-5S-methyl-3(2H)-furanone **(3)**

Dihydro-(4S-amino-[N-Boc])-5S-methyl-3(2H)-furanone **(2)** (1.0g, 4.6mmol) was treated with a solution of 4.0M HCl in dioxan (25mL) at room temperature for 1hr. The solvents were removed *in vacuo* and the residue azeotroped with 2 x toluene to give the hydrochloride salt as a white solid.
Boc-L-*tert*-butylalanine pentafluorophenyl ester (2.0g, 1.05eq) and 1-hydroxybenzotriazole hydrate (0.735g, 1.05eq) were dissolved in DMF (20mL) and after 5mins added to the above salt. The clear solution was then treated with N-methylmorpholine (0.51g, 0.56mL, 1.1eq) and left at room temperature for 2hrs. The solvents were removed *in vacuo* and the crude product purified by flash chromatography over silica gel (50g) eluting with EtOAc / heptane (1:3, v/v), then EtOAc / heptane (1:2, v/v). Fractions were pooled and reduced *in vacuo* to give dihydro-(4S-amino-[N-Boc-L-*tert*-butylalanyl])-5S-methyl-3(2H)-furanone **(3)** as a white solid, yield 1.31g, 3.82mmol, 83%.
Electrospray-MS m/z 343 ($MH^+$), 287 ($MH^+$ - 56).

This methodology is readily applicable to the corresponding N-Boc-protected pyranone or (1,5-anhydro-3-[(tert-butoxycarbonyl)amino]-3,4-dideoxy-4-ethyl-D-xylitol

(d) General method for addition of capping group, exemplified by benzofuran-2-carboxylic acid [3,3-dimethyl-1S-(2S-methyl-4-oxo-tetrahydrofuran-3S-ylcarbamoyl)butyl]amide **(4)**

Dihydro-(4S-amino-[N-Boc-L,-*tert*-butylalanyl])-5S-methyl-3(2H)-furanone **(3)** (1.03g, 3.0mmol)) was treated with a solution of 4.0M HCl in dioxan (25mL) at room temperature for 1 hr. The solvents were removed *in vacuo* and the residue azeotroped with 2 x toluene to give the hydrochloride salt as a white solid.
Benzofuran-2-carboxypentafluorophenyl ester (1.05eq) and 1-hydroxybenzotriazole hydrate (1.05eq) are dissolved in DMF (15mL) and after 5mins added to the above salt. The clear solution was then treated with N-methylmorpholine (1.1eq) and left at room temperature for 2hrs.. The solvents are removed *in vacuo* and the crude product purified by flash chromatography over silica gel (50g) eluting with EtOAc / heptane (3:2, v/v). Fractions are pooled and reduced *in vacuo* to give the title compound.

Example 2. 4,4-Dimethyl-2S-(benzofuran-2-sulfonylamino)pentanoic acid (2S-methyl-4-oxo-tetrahydrofuran-3S-yl) amide **(5)**

(a) General method for addition of sulphonyl capping group, exemplified by 4,4-Dimethyl-2S-(benzofuran-2-sulfonylamino)pentanoic acid (2S-methyl-4-oxo-tetrahydrofuran-3S-yl)amide **(5)**

Dihydro-(4S-amino-[N-Boc-L-tertbutylalanyl])-5S-methyl-3(2H)-furanone **(3)** (34mg, 0.1mmol) was treated with a solution of 4.0M HCl in dioxan (5mL) at room temperature for 1 hr. The solvents were removed *in vacuo* and the residue azeotroped with 2 x toluene to give the hydrochloride salt as a white solid.
Hydrochloride salt was dissolved in dry DCM (2mL) and benzofuran-2-sulphonylchloride added followed by diisopropylethylamine (3eq) and catalytic N,N-dimethylaminopyridine (2mg). After 2hr at room temperature, the solution was diluted with DCM (15mL) and washed successively with 0.1N HCl (25mL), water (2 x 25mL) and brine (25mL), then dried over sodium sulphate. The solvent was removed *in vacuo* and the crude product purified by flash chromatography over silica gel (15g) eluting with EtOAc / heptane (1:1, v/v). Fractions were pooled and reduced *in vacuo* to give the title compound.

**General Synthesis of Chiral β-alkyl serine aminoacids**

Adapted from Blaskovich, M.A., Evinder, G., Rose, N. G. W., Wilkinson, S., Luo, Y. and Lajoie, G. A. *J. Org. Chem,* 63, 3631-3646, 1998. (Following scheme 2).

Example 5. (2S, 3S)β-hydroxynorvaline **(15)**

(a) N-Benzyloxycarbonyl-L-serine 3-methyl-3-(hydroxymethyl)oxetane ester **(8)**

N-Cbz-L-serine (10 g, 41.8 mmol) was dissolved in DCM (450 mL) and DMF (14 mL) and added dropwise over 2.5 h to a stirred solution of WSC. HCl (12 g, 62.7 mmol), N'N-dimethylaminopyridine (260 mg, 2.1 mmol) and 3-methyl-3-oxetane methanol (84 mL, 0.84 mmol) cooled to 0 °C. The reaction was warmed to room temperature and allowed to stir overnight. The mixture was washed with 0.1M HCl (200 mL), water (200 mL), 10 % $Na_2CO$ (200 mL x 2) and water (200mL x 2), dried ($Na_2SO_4$) and the solvent evaporated *in vacuo* to afford a pale yellow oil. Purification by column chromatography (4:1, EtOAc:heptane) and subsequent recrystallisation (1:1, EtOAc:heptane) yielded the target intermediate as a white crystalline solid, 8.07 g, 60 %; TLC (4:1, EtOAc:heptane), R*f*= 0.28, electrospray-MS m/z 324.1 ($MH^+$). δ (400 MHz; $CDCl_3$) 1.28 (3H, s, $C\underline{H}_3$), 3.04 (1H, t, *J* 6.2, CHNH), 3.90-3.91 (1H, br m, $O\underline{H}$), 4.10-4.13 (2H, m, $C\underline{H}_2OH$), 4.41-4.55 (6H, m, 3 x $C\underline{H}_2$), 5.13 (2H, s, $OC\underline{H}_2$), 5.82 (1H, d, *J* 7.7, $N\underline{H}$). 7.35-7.36 (5H, m, $C_6\underline{H}_5$).
$\delta_c$ (100 MHz; $CDCl_3$) 20.75 ($\underline{C}H_3$). 39.67 ($\underline{C}H_2OH$), 56.39 ($\underline{C}HNH$), 63.37 ($\underline{C}H_2$), 67.19 ($\underline{C}H_2$), 68.94 ($\underline{C}H_2$). 79.50 ($O\underline{C}H_2$), 128.16 ($\underline{C}_6H_5$), 128.27 ($\underline{C}_6H_5$), 128.58 ($\underline{C}_6H_5$), 136.14 ($\underline{C}_6H_5$), 156.25 ($\underline{C}O_2NH$), 170.74 ($\underline{C}O_2$).

(b) 1-[*N*-Benzyloxycarbonyl-(1S)-1-amino-2-hydroxyethyl]-4-methyl-2,6,7-trioxabicyclo[2.2.2]oxetane **(9).**

Compound **(8)** (10.23 g, 28.6 mmol) was dissolved in anhydrous DCM (150 mL) and cooled to 0 °C under $N_2$. A solution of boron trifluoride etherate (0.10 mL, 0.77 mmol) in anhydrous DCM (10 mL) was added and the mixture stirred for 30 minutes at 0 °C, then at room temperature overnight. Triethylamine (1.2 mL, 8.30 mmol) was added and the reaction mixture stirred for 30 minutes before being concentrated to a thick colourless oil. Purification by column chromatography (4:1, EtOAc:heptane) and subsequent recrystallisation (1:1, EtOAc:heptane) yielded **(9)** as a white crystalline solid, 8.06 g, 80 %; TLC (4:1, EtOAc:heptane) R*f* = 0.27, electrospray-MS m/z 324.1 ($MH^+$).
δ (400 MHz; $CDCl_3$) 0.78 (3H, s, $CH_3$), 2.67 (1H, m, $C\underline{H}NH$), 3.64-3.69 (1H, m, $C\underline{H}_2OH$). 3.80-3.83 (1H, m, $C\underline{H}_2OH$). 3.88 (6H, s, $C\underline{H}_2$ x 3), 5.09 (2H, dd, *J* 18.9,12.3, $OC\underline{H}_2$), 5.38 (1H, d, *J* 8.7, $N\underline{H}$), 7.26-7.34 (5H, m, $C_6\underline{H}_5$),
$\delta_c$ (100 MHz; $CDCl_3$) 14.11 ($\underline{C}H_3$), 30.39 ($\underline{C}CH_3$), 55.26 ($\underline{C}HNH$), 61.75 ($\underline{C}H_2OH$), 66.76 ($\underline{C}H_2O$), 72.53 ($\underline{C}H_2$ x 3), 108.29 ($\underline{C}O_3$), 127.98 ($\underline{C}_6H_5$), 128.32 ($\underline{C}_5H_5$), 136.31 ($\underline{C}_6H_5$), 156.31 ($\underline{C}O_2NH$).

(c) 1-[*N*-Benzyloxycarbonyl-(1S)-1-amino-2-oxoethyl]-4-methyl-2,6,7-trioxabicyclo[2.2.2]oxetane **(10).**

Compound **(9)** (6.45 g, 20.0 mmol) was dissolved in anhydrous DCM (55 mL) under $N_2$ and cooled to -78°C in flask 1. Oxalyl chloride (2.8 mL, 31.9 mmol) was added to anhydrous DCM (85 mL) in a separate flask (flask 2) under $N_2$ and cooled to -78 °C. Anhydrous dimethylsulphoxide (4.7 mL, 65.8 mmol) was added to the oxalyl chloride solution and the mixture stirred at -78 °C for 15 minutes. The alcohol solution was transferred over 20 minutes by cannula to flask 2 and rinsed with anhydrous DCM (35 mL). The resulting cloudy, white mixture was stirred for 1.5 hours at -78 °C. Diisopropylethylamine (17.4 mL, 99.7 mmol) was added and the solution stirred for 30 minutes at -78 °C and 10 minutes at 0 °C. Ice-cold DCM (140 mL) was added and the solution washed with ice-cold $NH_4Cl$ (20 % saturated solution; 3 x 140 mL) and saturated NaCl (140 mL), dried ($MgSO_4$) and the solvent evaporated *in vacuo* to afford a yellow solid (10), 5.08 g, 79 %; TLC (3:1, EtOAc:heptane), R*f* = 0.56, electrospray-MS m/z 322.1 (40%) ($MH^+$), 340.2 (100%) ($MH^+ + H_2O$).
$\delta_H$ (400 MHz; $CDCl_3$); 0.82 (3H, s, $CH_3$), 3.93 (6H, s, $C\underline{H}_2$ x 3), 4.60 (1H, d, *J* 8.8, CHNH), 5.12 (2H, dd, *J* 14.9,12.4, $OC\underline{H}_2$), 5.35 (1H, br d, *J* 8.0, $N\underline{H}$), 7.30-7.36 (5H, m, $C_6\underline{H}_5$), 9.68 (1H, s, $\underline{H}CO$).
$\delta_c$ (100 MHz; $CDCl_3$) 14.25 ($\underline{C}H_3$), 30.86 ($\underline{C}CH_3$), 63.25 ($\underline{C}HNH$), 67.22 ($\underline{C}H_2O$), 72.88 ($\underline{C}H_2$ x 3), 107.16 ($\underline{C}O_3$), 128.13 ($\underline{C}_6H_5$), 128.46 ($\underline{C}_6H_5$), 136.13 ($\underline{C}_6H_5$), 156.17 ($\underline{C}O_2NH$), 195.66 ($\underline{C}HO$).

(d) 1-[*N*-(Benzyloxycarbonyl)-(1S,2R)-1-amino-2-hydroxybutyl]-4-methyl-2,6,7- trioxabicyclo[2.2.2]oxetane **(11).**

Compound **(10)** (2 g, 5.73 mmol) was dissolved in anhydrous DCM:$Et_2O$ (1:1) under $N_2$. A solution of EtMgBr (3M solution in $Et_2O$; 7.6 mL, 22.9 mmol) was added quickly at - 78 °C and stirred vigorously. After 30 minutes the reaction was quenched by pouring into 5 % $NH_4Cl$ (500 mL). DCM (500 mL) was added, the organic layer separated and washed-with 3 % $NH_4Cl$ (500 mL) and brine (500 mL), dried ($Na_2SO_4$) and the solvent evaporated *in vacuo* to afford a yellow oil. Purification by column chromatography (1:10, EtOAc:DCM) and subsequent recrystallisation (EtOAc:heptane) yielded a white crystalline solid (11), 1.32 g, 60 %; TLC (1:10, EtOAc:DCM) Rf= 0.25, electrospray-MS m/z 352.2 (20%) ($MH^+$), 370.3 (100%) ($MH^+ + H_2O$).
8 (400 MHz; $CDCl_3$) 0.82 (3H, s, $C\underline{H}_3$), 0.94 (3H, t, *J* 7.4, $CH_2C\underline{H}_3$), 1.36-1.53 (2H, m, $C\underline{H}_2$CH3), 3.85 (1H, d, *J* 10.3, $C\underline{H}$), 3.93 (6H, s, $C\underline{H}_2$ x 3), 4.05 (1H, t, *J* 6.8, $C\underline{H}$), 5.13-5.14 (2H, dd, *J* 16.4, 12.7, $OC\underline{H}_2$), 5.32 (1H,d, *J* 10.2, $N\underline{H}$), 7.30-7.36 (5H, m, $C_6\underline{H}_5$).
$\delta_c$ (100 MHz; $CDCl_3$). 10.11 ($CH_2\underline{C}H_3$), 14.34 ($\underline{C}H_3$), 25.98 ($\underline{C}H_2CH_3$), 30.65 ($\underline{C}CH_3$), 56.05 ($\underline{C}HOH$), 66.83 ($\underline{C}H_2O$), 70.81 ($\underline{C}HNH$), 72.76 ($\underline{C}H_2$ x 3), 108.93 ($\underline{C}O_3$), 127.65 ($\underline{C}_6H_5$), 128.45 ($\underline{C}_6H_5$), 136.65 ($\underline{C}_6H_5$), 156.83 ($\underline{C}O_2NH$).

(e) 1-[*N*-Benzyloxycarbonyl-(1S)-1-amino-2-oxobutyl]-4-methyl-2,6,7-trioxabicyclo[2.2.2]oxetane **(12).**

Compound **(11)** (1.32 g, 3.8 mmol) was dissolved in anhydrous DCM (10 mL) under $N_2$ and cooled to -78 °C in flask 1. Oxalyl chloride (2M solution in DCM; 3 mL, 6.0 mmol) was diluted with anhydrous DCM (10 mL) in a separate flask (flask 2) under $N_2$ and cooled to -78 °C. Anhydrous dimethylsulphoxide (0.88 mL, 12.4 mmol) was added to the oxalyl chloride solution and the mixture stirred at -78 °C for 15 minutes. The alcohol solution was transferred over 20 minutes by cannula to flask 2 and rinsed with anhydrous DCM (10 mL). The resulting cloudy, white mixture was stirred for 2 hr 15 min at-78 °C. DIPEA (3.3 mL, 18.8 mmol) was added and the solution stirred for 30 minutes at -78 °C and

10 minutes at 0 °C. Ice-cold DCM (25 mL) was added and the solution washed with ice-cold $NH_4Cl$ (5 % saturated solution; 3 x 25 mL) and saturated NaCl (25 mL), dried ($Na_2SO_4$) and the solvent evaporated *in vacuo* to afford an orange oil. Purification by column chromatography (2:3, EtOAc:heptane) yielded a colourless oil **(12),** 556 mg, 45 %; TLC (2:3, EtOAc:heptane) Rf= 0.25, ,electrospray-MS m/z 350.2 (60%) ($MH^+$), 368.2 (100%) ($MH^++H_2O$).

8 (400 MHz; $CDCl_3$) 0.80 (3H, s, C$\underline{H}_3$), 1.06 (3H, t, *J* 7.2, $CH_2C\underline{H}_3$), 2.48-2.56 (1H, m, C$\underline{H}CH_3$), 2.80-2.88 (1H, m, C$\underline{H}CH_3$), 3.90 (6H, s, C$\underline{H}_2$ x 3), 4.60 (1H, d, *J* 8.8, C$\underline{H}$NH), 5.09 (2H, s, OC$\underline{H}_2$), 5.66 (1H, d, *J* 8.5, N$\underline{H}$), 7.30-7.35 (5H, m, $C_6\underline{H}_5$).

$\delta_c$ (100 MHz; $CDCl_3$) 7.53 ($CH_2\underline{C}H_3$), 14.25 ($\underline{C}H_3$), 30.57 ($\underline{C}CH_3$), 35.74 ($\underline{C}H_2CH_3$), 62.33 ($\underline{C}$HNH), 67.05 ($\underline{C}H_2O$), 72.94 ($\underline{C}H_2$ x 3), 106.98 ($\underline{C}O_3$), 128.10 ($\underline{C}_6H_5$), 128.46 ($\underline{C}_6H_5$), 136.31 ($\underline{C}_6H_5$), 155.99 ($\underline{C}O_2NH$).

(f) 1-[*N*-(Benzyloxycarbonyl)-(1S,2S)-1-amino-2-hydroxybutyl]-4-methyl-2,6,7- trioxabicyclo[2.2.2]oxetane **(13).**

Compound (12) (2.77 g, 7.9 mmol) and $LiBH_4$ (1.73 g, 79 mmol) were cooled to -78 °C under $N_2$. A solution of DCM:$CH_3OH$ (1.5:1; 332 mL cooled to -78 °C) was added and the solution stirred at -78°C overnight. After being warmed to room temperature, the solution was poured into 5% $NH_4Cl$ solution (500 mL) and DCM (300 mL) added. The organic layer was separated, washed with 5 % $NH_4Cl$ solution (500 mL) and brine (400 mL), dried ($Na_2SO_4$) and the solvent evaporated *in vacuo* to afford a white solid (13), 2.51 g, 90 %; TLC (1:1, EtOAc:heptane) Rf= 0.23, electrospray-MS m/z 352.2 (40%) ($MH^+$), 370.3 (100%) ($MH^++H_2O$).

δ (400 MHz; $CDCl_3$) 0.82 (3H, s, $CH_3$), 0.97 (3H, t, *J* 7.4, $CH_2C\underline{H}_3$), 1.44-1.45 (1H, m, $CHCH_3$), 1.63-1.68 (1H, m, $CHCH_3$), 3.44 (1H, d, *J* 4.0, C$\underline{H}$OH), 3.66-3.69 (1H, m, CHNH), 3.92 (6H, s, C$\underline{H}_2$ x 3). 5.04 (1H, d. *J* 9.8, N$\underline{H}$). 5.16 (2H, d, *J* 6.1, $OCH_2$), 7.36 (5H, d, *J* 4.3, $C_6H_5$),

$\delta_C$ (100 MHz; $CDCl_3$) 9.79 ($CH_2\underline{C}H_3$), 14.27 ($CH_3$), 26.10 ($\underline{C}H_2CH_3$), 30.56 ($\underline{C}CH_3$), 57.57 ($\underline{C}$HOH), 66.94 ($\underline{C}H_2O$), 69.80 ($\underline{C}$HNH), 72.66 ($\underline{C}H_2$ x 3), 108.89 ($\underline{C}O_3$), 128.06 ($\underline{C}_6H_5$), 128.47 ($\underline{C}_6H_5$), 136.51 ($\underline{C}_6H_5$), 156.49 ($\underline{C}O_2NH$).

(g) (1S,2S)-(1-amino-2-hydroxybutyl)-4-methyl-2,6,7-trioxabicyclo[2.2.2]oxetane **(14).**

Compound **(13)** (2.51g, 7.1mmol) was dissolved in ethanol (220 mL) and 10 % Pd/C (218 mg) added. The reaction mixture was stirred overnight in the presence of $H_2$. The catalyst was removed by filtration through celite and the solvent evaporated *in vacuo* to afford a thick oil. Purification by column chromatography (20:1, DCM:MeOH) yielded a pale yellow oil **(14),**1.24 g, 92 %, which crystallised on standing; TLC (5:1, DCM:MeOH) Rf= 0.51, electrospray-MS m/z 218.1 ($MH^+$).

(400 MHz; $CDCl_3$) 0.83 (3H, s, C$\underline{H}_3$), 0.98 (3H, t, *J* 7.4, $CH_2C\underline{H}_3$), 1.38-1.48 (1H, m, C$\underline{H}CH_3$), 1.71-1.78 (1H, m, C$\underline{H}CH_3$), 2.77 (1H, d, *J* 7.1, C$\underline{H}NH_2$), 3.62-3.66 (1H, m, C$\underline{H}$OH), 3.93 (6H, s, C$\underline{H}_2$ x 3).

c (100 MHz; $CDCl_3$) 9.57 ($CH_2\underline{C}H_3$), 14.37 ($\underline{C}H_3$), 26.01 ($\underline{C}H_2CH_3$), 30.52 ($\underline{C}CH_3$), 58.52 ($\underline{C}$HOH), 72.59 ($\underline{C}HNH_2$), 72.67 ($\underline{C}H_2$ x 3), 109.62 ($\underline{C}O_3$).

(h) (2S,3S)β-hydroxynorvaline **(15)**

Compound **(14)** (1.24 g, 5.5 mmol) was dissolved in DCM (68 mL) and trifluoroacetic acid (1.58 mL) and $H_2O$ (1.13 mL) added. The resulting cloudy, white solution was stirred at room temperature for 30 minutes and the solvent evaporated *in vacuo.* The colourless residue was dissolved in MeOH (66 mL) and $H_2O$ (17 mL) and 10 % $Cs_2CO_3$ (9.2 g in 92 mL $H_2O$) added. After stirring overnight at room temperature, the solution was acidified with 2 M HCl (~35 mL) to pH <3. The solution was loaded onto a cation exchange column (Bio-Rad AG 50W-X8 100-200 mesh, hydrogen form, 4.5 x 20 cm) washed with 0.01 M HCl (500 mL) and $H_2O$ (500 mL) and eluted with 2M $NH_4OH$ (2 L) then lyopholised to afford a pale yellow solid. The solid was washed with MeOH to yield an off-white solid **(15),** 227 mg, 30 %; TLC (4:1:1, butan-2-ol : AcOH : $H_2O$) Rf= 0.26, electrospray-MS m/z 134.1 ($MH^+$), elemental analysis $CsH_{11}O_3N$ (req) %C 45.10. %H 8.33, %N 10.52, (fnd) %C 44.67, %H 8.03, %N 9.92.

δ (400 MHz; $CDCl_3$) 92:8 *erythro* (2S, 3S) : *threo* (2S, 3R), 1.00 (3H, t, *J* 7.4, C$\underline{H}_3$), 1.40-1.54 (2H, m, C$\underline{H}_2$), 3.41 (0.08H, d, *J* 4.2, C$\underline{H}$), 3.61 (0.92H, d, *J* 4.2, C$\underline{H}$), 3.60-3.65 (0.08H, m, C$\underline{H}$), 3.66-3.69 (0.92H, m, C$\underline{H}$),

$\delta_c$ (100 MHz; $CDCl_3$) 11.02 ($CH_2\underline{C}$H3), 25.26 ($\underline{C}H_2CH_3$), 61.26 ($\underline{C}$HOH), 72.09 ($\underline{C}HNH_2$), 172.03 ($\underline{C}O_2H$).

**General method for the synthesis of Fmoc-3(2H)-furanones**

Exemplified by dihydro-(4S-amino-[N-Fmoc])-5S-ethyl-3(2H)-furanone (18), following the general chemistry detailed in scheme 1.

(a) Preparation of Fmoc-(2S,3S)-β-ethylserine **(16)**

(2S,3S) -hydroxynorvaline **(15)** (277mg, 2.07mmol) and sodium carbonate (2.1eq, 460mg) were dissolved with stirring and ice-cooling in water (25mL) and THF (10mL). 9-Fluorenylmethyl chloroformate (1.05eq, 560mg) in THF (15mL) was added over 45mins and the mixture stirred for a further 1 hr at room temperature. Chloroform (100mL) and water (50mL) were added and the mixture acidified to pH2 with 0.1 N HCl. The organic layer was collected and the aqueous washed with a further 2 x 100mL chloroform. The combined organics were backwashed with brine (1 x 300mL) and dried over magnesium sulphate. The chloroform was reduced *in vacuo* to yield a fine white solid. The solid was dissolved in *tert*-butyl methylether (25mL) with heating and heptane (75mL) added to give a cloudy solution. The mixture was cooled to -20°C and each 30mins further heptane (75mL) added for 4 cycles. The precipitate was filtered off and dried *in vacuo* to a fine white solid **(16)** 590mg, 80.6 %; TLC ($CHCl_3$ ; MeOH 3:1) R*f* = 0.40, electrospray-MS m/z 356.2 ($MH^+$).

(b) Preparation of (2S, 3S)-N-Fmoc-β-ethylserinydiazomethane **(17)**

Following the general method detailed in example **1**.(a) for compound **(1),** Fmoc-(2S, 3S)- -ethylserine **(16)** (560mg) was converted to a yellow solid (600mg) **(17)** used without purification.

(c) Preparation of dihydro-(4S-amino-[N-Fmoc])-5S-ethyl-3(2H)-furanone **(18)**

A solution of lithium chloride (1.0g, 23.5mmol) in 80% aqueous acetic acid (10mL) was cooled to 5°C and added to crude (2S, 3S)-N-Fmoc-ethylserinydiazomethane **(17)** (0.6g) with stirring. The oil dissolved over 10mins and stirring continued for a further 1 hr slowly warming to room temperature, with evolution of gas. The solvents were removed in vacuo and the residue taken into EtOAc (50mL) and washed successively with water (50mL), saturated aqueous sodium bicarbonate (2 x 100mL) and brine (75mL), then dried over sodium sulphate. The solvent was removed *in vacuo* and the crude product purified by flash chromatography over silica gel (25g) eluting with EtOAc / heptane (1:3, v/v). Desired fractions were pooled and reduced *in vacuo* to give dihydro-(4S-amino-[N-Fmoc])-5S-ethyl-3(2H)-furanone **(18)** as a white solid, yield 320mg, 0.91mmol, 58%. Electrospray-MS m/z 352 ($MH^+$), HRMS $C_{21}H_{21}O_4NNa$ requires M, 374.1368, found: $MNa^+$, 374.1368. (. - 1.49 ppm), analytical HPLC Rt = 13.61 mins (98.4%), elemental analysis $C_{21}H_{21}O_4N$ (req) %C 71.78, %H 6.02, %N 3.99, (fnd) %C 70.95, %H 6.22, %N 3.81.
δ (500 MHz; $CDCl_3$); 1.05 (3H, m, $CH_2\underline{C}\underline{H}_3$), 1.76, 1.94 (2H, bm, $C\underline{H}_2CH_3$), 3.83 (1H, bm, furanone C$\underline{H}$β), 3.88 (1H, bm, furanone C$\underline{H}$α), 4.02 (1H, d, *J* 17.3, 1 x furanone $COC\underline{H}_2O$), 4.23 (2H, m, 1 x furanone $COC\underline{H}_2O$ + Fmoc $C\underline{H}CH_2O$), 4.42 (2H, b, Fmoc $CHC\underline{H}_2O$). 5.05 (1H, b, furanone, N$\underline{H}$), 7.35 (2H, t, *J* 7.4, Fmoc aromatic), 7.42 (2H, t, *J* 7.3, Fmoc aromatic), 7.58 (2H, t, *J* 7.4, Fmoc aromatic), 7.77 (2H, t, *J* 7.4, Fmoc aromatic).
$\delta_C$ (125 MHz; $CDCl_3$); 8.90 (5S-$CH_2\underline{C}H_3$), 26.14 (5S-$\underline{C}H_2CH_3$), 46.90 (Fmoc $\underline{C}HCH_2O$), 60.50 (furanone $\underline{C}$Hα), 66.99 (Fmoc $CH\underline{C}H_2O$), 70.43 (furanone $CO\underline{C}H_2O$), 81.65 (furanone $\underline{C}$Hβ), 119.76 (Fmoc aromatic), 124.72 (Fmoc aromatic), 126.85 (Fmoc aromatic), 127.53 (Fmoc aromatic), 141.09 (Fmoc aromatic). 143.37 (Fmoc aromatic), 155.76 (O$\underline{C}$ONH), 211.72 (furanone $\underline{C}$O).

(d) Preparation of (2S, 3R)-N-Fmoc-*O*-*t*-butyt-L-threonyldiazomethane **(19)**

Following the general method detailed in example 1. (a) for compound **(1),** Fmoc-(2S,3R)-*O*-*t*-butyl-L-threonine (1.99 g, 5 mmol) was converted to (2S, 3R)-N-Fmoc-*O*-t-butyl-L-threonyldiazomethane **(19)** (2.11g, 100%) as a pale yellow immobile oil. This compound was carried through to the next stage without further purification. Electrospray-MS m/z 444 ($MNa^+$, 20%), 394 ($MH^+$-$N_2$, 70%) and 338 ($MH^+$-*t*butyl- $N_2$, 100%).

(e) Preparation of (2R, 3S)-N-Fmoc-O-*t*-butyl-D-threonyldiazomethane **(20)**

Following the general method detailed in example 1. (a) for compound **(1),** Fmoc-(2R,3S)-*O*-*t*-butyl-D-threonine (0.4 g, 1 mmol) was converted to (2S, 3R)-N-Fmoc-O-*t*-butyl-L-threonyldiazomethane **(20)** (0.48g, 111%) as a pale yellow immobile oil. This compound was carried through to the next stage without further purification. Electrospray-MS m/z 394 ($MH^+$ - $N_2$, 60%) and 338 ($MH^+$ - tbutyl - $N_2$, 100%).

(f) Preparation of (2S, 3S)-N-Fmoc-*O*-*t*-butyl-L-*allo*-threonyldiazomethane **(21)**

Following the general method detailed in example 1.(a) for compound **(1),** Fmoc-(2S,3S)-O-*t*-butyl-L-*allo*-threonine (0.4 g, 1 mmol) was converted to (2S, 3S)-N-Fmoc-O-*t* butyl-L-*allo*-threonyldiazomethane **(21)** (0.53g, 123%) as a pale yellow immobile oil.. Electrospray-MS m/z 394 ($MH^+$- $N_2$, 90%) and 338 ($MH^+$ - *t*butyl - $N_2$, 60%).

(i) Preparation of dihydro-(4R-amino-[N-Fmoc])-5R-methyl-3(2H)-furanone **(22)**

Following the general method detailed for cyclisation of **(17)** to **(18),** diazoketone (19) cyclised to give dihydro-(4R-amino-[N-Fmoc])-5R-methyl-3(2H)-furanone (22) isolated as a white solid, yield 69%, electrospray-MS m/z 338 ($MH^+$, 100%), analytical HPLC Rt = 14.59 mins (97.7%).
δ (500 MHz; $CDCl_3$); 1.50 (3H, brd, C<u>H</u>$_3$), 3.80 (1H, brt, furanone C<u>H</u>α), 3.97 (1H, brm, furanone C<u>H</u>), 3.99 (1H, d, *J* 17.7, 1 x furanone COC<u>H</u>$_2$O), 4.22 (1H, t, *J* 6.7, Fmoc C<u>H</u>CH$_2$O), 4.25 (1H, d, *J* 17.7, 1 x furanone COC<u>H</u>$_2$O), 4.44 (2H, b, Fmoc C<u>H</u>CH$_2$O), 5.11 (1H, b, N<u>H</u>), 7.32 (2H, t, *J* 7.4, Fmoc aromatic), 7.41 (2H, t, *J* 7.4, Fmoc aromatic), 7.58 (2H, t, *J* 7.4, Fmoc aromatic), 7.76 (2H, t, *J* 7.4, Fmoc aromatic).
$\delta_c$ (125 MHz; $CDCl_3$); 19.1 (<u>C</u>H$_3$), 47.2 (Fmoc <u>C</u>HCH$_2$O), 62.7 (furanone <u>C</u>Hα), 67.3 (Fmoc CH<u>C</u>H$_2$O), 70.8 (furanone CO<u>C</u>H$_2$O), 77.4 (furanone <u>C</u>Hβ), 120.1 (Fmoc aromatic), 125.0 (Fmoc aromatic), 127.1 (Fmoc aromatic), 127.8 (Fmoc aromatic), 141.4 (Fmoc aromatic), 143.7 (Fmoc aromatic), 156.1 (O<u>C</u>ONH), 211.8 (furanone <u>C</u>O).

(j) Preparation of dihydro-(4S-amino-[N-Fmoc])-5S-methyl-3(2H)-furanone **(23)**

Following the general method detailed for cyclisation of **(17)** to **(18),** diazoketone **(20)** cyclised to give dihydro-(4S-amino-[N-Fmoc])-5S-methyl-3(2H)-furanone **(23)** isolated as a white solid, yield 70%, electrospray-MS m/z 338 ($MH^+$, 75%), analytical HPLC Rt = 14.62 mins (98.9%).

(k) Preparation of dihydro-(4S-amino-[N-Fmoc])-5S-methyl-3(2H)-furanone **(23)**

Following the general method detailed for cyclisation of **(17)** to **(18),** diazoketone **(21)** cyclised to give dihydro-(4R-amino-[N-Frnoc])-SR-methyl-3(2H)-furanone **(23)** isolated as a white solid, yield 64%, electrospray-MS m/z 338 ($MH^+$, 100%), analytical HPLC Rt = 14.68 mins (97.5%).

**<u>Alternative Route Towards Chiral β-Alkyl Serines</u>**

Following the chemistry detailed in scheme 3. Exemplified by the synthesis of (2S, 3S)-β-hydroxynorvaline (15) (also termed of (2S, 35)-β-ethylserine)

(a) Tri-acetone-D-mannitol

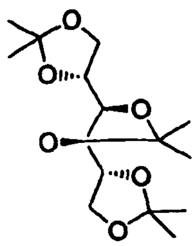

D-Mannitol (49.5g, 0.27mol) was suspended in acetone (600mL, 99.9% purity). To the suspension $H_2SO_4$ (4.95mL) was added and the mixture shaken at 21°C overnight. The solution was then filtered and the clear solution neutralised with a saturated solution of $NaHCO_3$ until pH=6. The solvent was concentrated *in vacuo,* affording tri-acetone-<u>D</u>-mannitol as a white solid, yield 78g, 96%. Electrospray-MS m/z 303 ($MH^+$).

(b) 3,4-Isopropylidine-D-mannitol

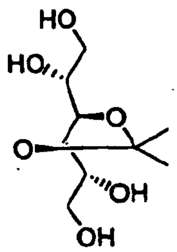

Tri-acetone-D-mannitol (78g, 0.26mol) was dissolved in the minimum amount of 70% acetic acid (400mL) and stirred in water bath at 42.7°C for 1.5hrs. The solvent was quickly evaporated *in vacuo* to give 3,4-Isopropylidine-D-mannitol as a colourless oil, yield 57.6g, 99.8%. Electrospray-MS m/z 223 ($MH^+$).

(c) 1,2,5,6-tetra-O-benzyl-3,4-O-isopropylidine-D-mannitol **(35)**

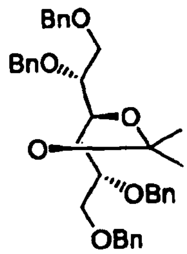

3,4-Isopropylidine-D-mannitol (57.64g, 0.26mol) was dissolved in benzylchloride (543mL). To the stirred solution powdered KOH (500g) was added and the solution heated in an oil bath at 133°C for 2hrs. The mixture was allowed to cool to room temperature and poured into a 3000mL beaker. Ice and water (1400mL) were carefully added, the mixture extracted with DCM (800mL) and the aqueous phase further extracted with DCM (300mL). The organic extracts were dried over sodium sulphate and the filtered solution concentrated *in vacuo.* The residue was purified by flash chromatography over silica gel eluting with EtOAc/heptane (1:15 to 1:10, v/v) to afford compound (35) as a colourless oil, yield 77g, 51%.
Electrospray-MS m/z 583 ($MH^+$). Analytical HPLC Rt = 29.16mins (91.8%).
$_H$ (500 MHz, $CDCl_3$) 1.35 (6H, s, $C(C\underline{H}_3)_2$), 3.62 (2H, dd, *J* 6, 10,2 x $C\underline{H}OC$), 3.75 (4H, m, 2 x $C\underline{H}_2OBn$), 4.15-4.20 (1H, m, $C\underline{H}OBn$), 4.46 (1H, dd, *J* 12.5, 14.5, $C\underline{H}OBn$), 4.77 (4H, d, *J* 11.5, 4 x $C\underline{H}_{2A}C_6H_5$), 4.73 (4H, d, *J* 11.5, 4 x $C\underline{H}_{2B}C_6H_5$) and 7.25-7.34 (20H, m, 4 x $C_6\underline{H}_5$).

(d) 1,2,5,6-Tetra-O-benzyl-D-mannitol **(36)**

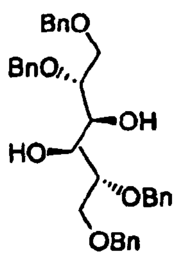

In a 2000mL flask fitted with a condenser compound **(35)** (41.11g, 0.071mol) was dissolved in 70% acetic acid (700mL) and the solution stirred at 100°C in an oil bath for 1.5hrs. After concentration *in vacuo,* the residue was purified by flash chromatography over silica gel eluting with EtOAc/heptane (3:7, v/v) to afford compound **(36)** as a pale yellow oil, yield 21.8g, 57%.

Electrospray-MS m/z 543 ($MH^+$). Analytical HPLC Rt = 25.8 (100%).
$\delta_H$ (500 MHz, $CDCl_3$) 3.01 (2H, d, *J* 6.0, 2 x OH), 3.65 -3.70 (2H, m, 2 x C$\underline{H}$OBn), 3.72-3.78 (4H, m, 2 x C$\underline{H}_2$OBn), 3.93-3.97 (2H, m, 2 x C$\underline{H}$OH), 4.55 (4H, s, 2 x C$\underline{H}_2C_6H_5$), 4.73 (2H, d, *J* 11.5, 2 x C$\underline{H}_{2A}C_6H_5$), 4.77 (2H, d, *J* 11.5, 2 x C$\underline{H}_{2B}C_6H_5$) and 7.25-7.34 (20H, m, 4 x $C_6H_5$).

(e) (2R)-2,3-Di-O-benzylglyceraldehyde **(37)**

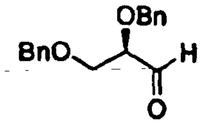

Compound **(36)** (10.78g, 0.02mol) was dissolved in anhydrous toluene (150mL). While vigorously stirring lead tetraacetate (9.83g, 0.023mol, 1.1eq) was added as a solid and the mixture stirred for 3hrs at room temperature. The mixture was then filtered and the filtered concentrated *in vacuo* to afford compound **(37)** as a colourless oil, yield 10.2g, 95%.
$\delta_H$ (500 MHz, $CDCl_3$) 3.75-3.83 (2H, m, C$\underline{H}_2$OBn), 3.97 (1H, t, *J* 4, C$\underline{H}$OBn), 4.55 (2H, d, *J* 5.5, 2 x C$\underline{H}_{2A}C_6H_5$), 4.70 (2H, d, *J* 12, 2 x C$\underline{H}_{2B}C_6H_5$), 7.20-7.40 (10H, m, 2 x $C_6\underline{H}_5$) and 9.70 (1H, s, C$\underline{H}$O).

(f) (2S)-N-(2,3-Dibenzyloxypropylidene)benzylamine **(38)**

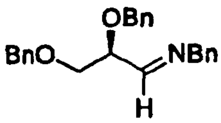

Benzylamine (4.06mL, 0.037mol, 1eq) was dissolved in anhydrous diethyl ether (150mL) and the solution cooled to 0°C. To a solution of compound **(37)** (9.9g, 0.037mol, 1eq) in anhydrous diethyl ether (100mL) at 0°C, was added anhydrous magnesium sulphate (7.3g) and the solution transferred *via* cannula under argon to the solution of the amine. After stirring for 3 hrs the reaction mixture was concentrated *in vacuo* to give compound **(38)** as a crude colourless oil, yield 12.2g, 96%.
$\delta_H$ (500 MHz, $CDCl_3$) 3.75-3.83 (2H, m, C$\underline{H}_2$OBn), 4.17-4.25 (1H, m, C$\underline{H}$OBn), 4.57 (2H, s, NC$\underline{H}_2C_6H_5$), 4.62 (2H, m, 2 x C$\underline{H}_{2A}C_6H_5$), 4.70 (2H, m, C$\underline{H}_{2B}C_6H_5$), 7.20-7.40 (15H, m, 3 x $C_6\underline{H}_5$) and 7.70 (1H, m, C$\underline{H}$N).

(g) (1R,2S)-N-Benzyl-2,3-dibenzyloxy-1 -phenyl-1-propylamine **(39)**

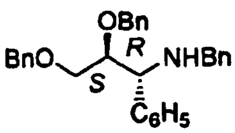

Phenylmagnesium bromide (29.17mL, 0.087mol, 3.0M, 2.5eq) was dissolved in anhydrous diethyl ether (124mL) and the solution cooled to 0°C under argon. A solution of compound **(38)** (12.5g, 0.035mol) in anhydrous diethyl ether (140mL) was transferred via cannula to the solution of the phenylmagnesium bromide and the reaction mixture stirred at room temperature for 2hrs. The solution was poured into an aqueous solution of $NH_4Cl$ (200mL) and extracted with *tert*-butyl methyl ether (2 x 100mL). The combined extracts, dried over anhydrous sodium sulphate, were concentrated *in vacuo*. The crude oil obtained was purified by flash chromatography over silica gel eluting with EtOAc/heptane (1:4, v/v) to afford compound **(39)** as a pale yellow oil, yield 8.5g, 56%. Electrospray-MS m/z 438 ($MH^+$). Analytical HPLC Rt = 24.0mins (98%).

$\delta_H$ (500 MHz, $CDCl_3$) 2.45 (1H, br s, NH), 3.32 (1H, dd, *J* 10, 4.5, $CH_{2A}OBn$), 3.43 (1H, d, *J* 13, $C_6H_5CH_{2A}NH$) 3.50-3.54 (1H, dd, *J* 10, 3, $CH_{2B}OBn$), 3.55-3.61 (1H, d, *J* 13, $C_6H_5CH_{2B}NH$), 3.65-3.78 (1H, m, CHOBn), 3.90 (1H, d, *J* 7, $C_6H_5CHNH$), 4.40 (2H, s, $OCH_2C_6H_5$), 4.62 (1H, d, *J* 11, $OCH_{2A}C_6H_5$), 4.70 (1H, d, *J* 11, $OCH_{2B}C_6H_5$) and 7.18-7.40 (20H, m, 4 x $C_6H_5$).

(h) (1R,2S)-N-Benzyl-*tert*-butoxycarbonyl-2,3-dibenzyloxy-1-phenyl-1-propylamine **(40)**

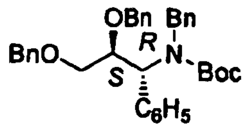

Compound **(39)** (9.26g, 0.02mol) was dissolved in dioxane (66mL) and diisopropylamine (0.37mL, 0.0021 mol, 0.11 eq) was added. To the stirred solution di-*tert*-butyl dicarbonate (11.25g, 0.0516mol, 2.6eq) was added as a solid and the solution stirred at 50°C in an oil bath overnight. The mixture was treated with *tert*-butyl methyl ether (300mL), washed with 1.0M $KHSO_4$ aqueous solution (60mL) and the organic extracts were dried over anhydrous sodium sulphate and concentrated *in vacuo.* The crude oil was purified by flash chromatography over silica gel eluting with EtOAc/heptane (1:9, v/v) to afford compound **(40)** as a colourless oil, yield 7.7g, 71%. Electrospray-MS m/z 538 ($MH^+$). Analytical HPLC Rt = 30.0mins (95%).

$\delta_H$ (500 MHz, $CDCl_3$) 1.30 (9H, s, $C(CH_3)_3$), 3.44 (1H, dd, *J* 10, 4.5, $CH_{2A}OBn$), 3.61 (1H, dd, *J* 10, 2, $CH_{2B}OBn$), 4.30 (1H, m, $CH_{2A}N$) 4.37 (2H, d, *J* 12, $OCH_{2A}C_6H_5$), 4.43 (2H, d, *J* 12, $OCH_{2B}C_6H_5$), 4.50-4.63 (1H, m, $CH_{2B}N$), 4.85 (1H, m, CHOBn) 5.25 (1H, d, *J* 9, $C_6H_5CHN$) and 7.00-7.45 (20H, m, 4 x $C_6H_5$).

(i) (1R,2S)-N-*tert*-Butoxycarbonyl-2,3-hydroxy-1-phenyl-1-propylamine **(41)**

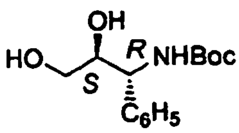

Compound (40) (7.67g, 0.014mol) was dissolved in anhydrous methanol (80mL). After having flushed the flask with argon, 20%$Pd(OH)_2$/C (10.00g. Degussa type, E101 NE/W, wet) was carefully added and the mixture stirred under $H_2$ for 48 hrs. The mixture was carefully filtered through a pad of Celite and the catalyst washed with a solution of aqueous methanol (10:100 $H_2O$:$CH_3OH$, v/v). The filtered solution was concentrated *in vacuo* and the residue purified by flash chromatography over silica gel eluting with EtOAc/heptane (3:1, v/v) to afford compound **(41)** as a colourless oil, yield 2.7g, 72%. Electrospray-MS m/z 268 ($MH^+$). Analytical HPLC Rt = 15.3mins (100%).

$\delta_H$ (500 MHz, $CDCl_3$) 1.44 (9H, s, $C(CH_3)_3$), 2.6 (2H, br s, OH), 3.56 (2H, d, *J* 5.5, $CH_2OH$), 3.97 (1H, s, $C_6H_5CHNH$), 4.83 (1H, s, $C_6H_5CHCHOH$), 5.28 (1H, d, *J* 8, NH) and 7.20-7.45 (5H, m, $C_6H_5$)

(i) (1R,2S)-N-*tert*-Butoxycarbonyl-3-tert-butyldimethylsilyloxy-2-hydroxy-1-phenyl-1-propylamine **(42)**

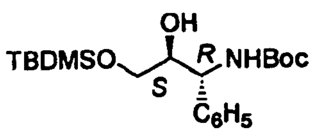

Compound **(41)** (2.67g, 0.01mol) was dissolved in anhydrous DMF (60mL) and stirred under argon. Imidazole (1.5g, 0.022mol, 2.2eq) was added followed by the addition of TBDMSCl (1.66g, 0.011 mol, 1.1eq). The reaction mixture

was stirred overnight at room temperature. The mixture was diluted with ether (240mL), washed with saturated $NH_4Cl$ (120mL) and $H_2O$ (40mL) and the aqueous layer extracted with ether (4 x 100mL). The combined extracts were dried over anhydrous sodium sulphate, filtered and concentrated *in vacuo.* Purification of the residue by flash chromatography over silica gel eluting with EtOAc/heptane (3:1, v/v) afforded compound **(42)** as a colourless oil, yield 3.31g, 87%. Electrospray-MS m/z 382 ($MH^+$).
$\delta_H$ (500 MHz, $CDCl_3$) 0.05 (3H, s, $C\underline{H}_{3A}SiCH_3$), 0.06 (3H, s, $CH_3SiC\underline{H}_{3B}$), 0.89 (9H, s, $Si(CH_3)_3$), 1.39 (9H, br s, $C(C\underline{H}_3)_3$), 2.45 (1H, br s, OH), 3.51 (1H, dd, *J* 10, 7, $TBDMSOC\underline{H}_{2A}$), 3.65 (1H, dd, *J* 10, 4.5, $TBDMSOC\underline{H}_{2B}$), 3.85 (1H, m, $C\underline{H}OH$), 4.66 (1H, m, $C_6H_5C\underline{H}NH$), 5.45 (1H, br s, $N\underline{H}$) and 7.23-7.35 (5H, m, $C_6\underline{H}_5$).

(k) (1R,2S)-N-*tert*-butoxycarbonyl-3-*tert* butyldimethylsilyloxy-2-mesyloxy-1-phenyl-1-propylamine **(43)**

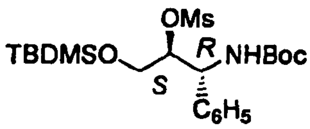

Compound **(42)** (1.30g, 3.40mmol, 1.0eq) was dissolved in anhydrous DCM (30mL). To the solution TEA (0.57mL, 4.09mmol, 1.2eq) was added and the mixture was cooled to 0°C in an ice-water bath. At this temperature and under argon, a solution of MsCl (0.32ml, 4.09mmol, 1.2eq) in anhydrous DCM (3mL) was added. The mixture was stirred for 1.5hrs. The reaction mixture was treated with water (20mL) and extracted with DCM (20mL). The aqueous phase was further extracted with DCM (4 x 60mL) and the combined organic layers were dried over anhydrous sodium sulphate and concentrated *in vacuo.* The residue was purified by flash chromatography over silica gel eluting with EtOAc/heptane (1:3, v/v) affording compound (43) as a colourless oil, yield 1.30g, 83%. Electrospray-MS m/z 460 ($MH^+$). Analytical HPLC Rt: 27.1mins (98%).
$\delta_H$ (500 MHz, $CDCl_3$) 0.06 (3H, s, $C\underline{H}_{3A}SiCH_3$), 0.07 (3H, s, $CH_3SiC\underline{H}_{3B}$), 0.91 (9H, s, $Si(CH_3)_3$), 1.42 (9H, br s, $C(C\underline{H}_3)_3$), 2.54 (3H, s, $SO_2CH_3$), 3.77 (2H, d, *J* 6.0, $TBDMSOC\underline{H}_2$), 4.7 (1H, m, $C\underline{H}OH$). 5.1 (1H, m, $C_6H_5C\underline{H}NH$), 5.4 (1H, br s, NH) and 7.26-7.38 (5H, m, $C_6H_5$).

(l) (1R,2R)-N-*tert*-Butoxycarbonyl-2,3-epoxy-1-propylamine **(44)**

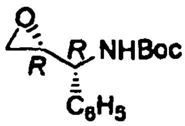

Compound **(43)** (3.79g, 8.26mmol, 1.0eq) was dissolved in THF anhydrous (78mL) and the solution cooled to 0°C in an ice water bath. TBAF (16.52mL, 1.0M sol in THF, 16.52mmol, 2eq) was added dropwise *via* syringe and once the addition was complete the ice bath was removed. The reaction mixture was stirred at room temperature overnight and then treated with water (40mL), extracted with diethyl ether (40mL) and the aqueous phase further extracted with diethyl ether (3 x 75mL). The combined extracts were dried over anhydrous sodium sulphate, filtered and concentrated *in vacuo.* The residue was purified by flash chromatography over silica gel eluting with TBME /heptane (1:6 to 2:1, v/v) affording compound **(44)** a white solid, yield 1.0g, 48%. Electrospray-MS m/z 250 ($MH^+$).
$\delta_H$ (500 MHz, $CDCl_3$) 1.42 (9H, s, $C(CH_3)_3$), 2.50 (1H, dd, *J* 5, 2.2, $CHC\underline{H}_{2A}O$), 2.76 (1H, dd, *J* 5, 4, $CHC\underline{H}_{2B}O$), 3.20-3.30 (1H, m, $C\underline{H}CH_2O$), 4.72 (1H, br s, $C_6H_5C\underline{H}CHO$), 5.00 (1H, br s, NH) and 7.27-7.38 (5H, m, $C_6H_5$).

(m) (1R,2S)-*N*-tert-butoxycarbonyl-2-hydroxy-1-phenyl-1-butytamine **(45)**

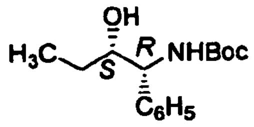

Copper(I)iodide (0.574g, 3.01mmol, 5eq) was dispersed in anhydrous diethyl ether (17mL). After cooling the suspension to -35°C under argon, $CH_3Li$ in diethyl ether (3.76mL, 1.6M, 6.02mmol, 10eq) was added dropwise. After stirring at -35°C for 30 mins a solution of compound **(44)** (0.15g, 0.60mmol, 1.0eq) dissolved in diethyl ether (1.5mL) was added dropwise to the solution of the organocuprate and the reaction mixture was stirred at -35°C for 1.5 hrs. Ethyl acetate (12.5mL) was added followed by the careful addition of a saturated solution of $NH_4Cl$ (10mL) and water (3mL). The mixture was allowed to warm up to room temperature and the organic phase extracted. The aqueous phase was further extracted with ethyl acetate (3 x 15mL) and the combined extracts dried over anhydrous sodium sulphate, filtered and concentrated *in vacuo.* The crude oil was purified by flash chromatography over silica gel eluting with TBME /heptane (2:3, v/v) affording compound **(45)** as a white solid, yield 0.14g, 88%. Electrospray-MS m/z 266 ($MH^+$). Analytical HPLC Rt = 17.6mins (100%).
$\delta_H$ (500 MHz, $CDCl_3$) 0.97 (3H, t, *J* 7.5, $C\underline{H}_3CH_2$), 1.10-1.25 (1H, m, $CH_3C\underline{H}_{2A}$), 1.25-1.50 (1H, m, $CH_3C\underline{H}_{2B}$), 1.50 (9H, s, $C(CH_3)_3$), 3.78 (1H, br s, $C\underline{H}OH$), 4.73 (1H, br s, $C_6H_5C\underline{H}NH$), 5.28 (1H, br s, NH) and 7.25-7.38 (5H, m, $C_6H_5$).

(n) (1R,2S)-N-*tert*-Butoxycarbonyl-2-*tert*-butoxy-1-phenyl-1-butylamine **(46)**

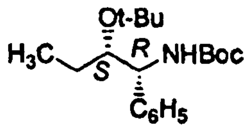

In a sealed tube, compound **(45)** (0.114g, 0.43mmol) was dissolved in anhydrous DCM (11mL). Whilst stirring was maintained, the tube was immersed in a dry ice-acetone bath and cooled to -60°C. Isobutylene (11 mL) was condensed into the tube and methyltriflate (55 L) was carefully added. The tube was capped tightly and the bath removed to allow the reaction to proceed at room temperature for 4 days. The tube was cooled to -60°C, the lid removed and then the bath removed to allow the excess of isobutylene to slowly evaporate whilst warming up to room temperature. At about 10°C, TEA (0.7mL) was added to neutralise the excess acid. The residue obtained after removal of the solvents *in vacuo* was purified by flash chromatography over silica gel eluting with EtOAc/heptane (2:8, v/v) affording compound (46) as a white solid, yield 0.02g, 14.% Electrospray-MS m/z 322 ($MH^+$). Analytical HPLC Rt = 24.1mins (90%).
$\delta_H$ (500 MHz, $CDCl_3$) 0.84 (3H, t, *J* 7.5, $CH_3CH_2$), 1.15-1.30 (1H, m, $CH_3C\underline{H}_{2A}$) 1.24 (9H, s, $CHOC(C\underline{H}_3)_3$), 1.35-1.40 (1H, m, $CH_3C\underline{H}_{2B}$), 1.41 (9H, s, $CO_2C(C\underline{H}_3)_3$), 3.72 (1H, m, $C\underline{H}O(CH_3)_3$), 4.78 (1H, m, $C_6H_5C\underline{H}NH$), 5.15 (1H, br s, NH) and 7.22-7.38 (5H, m, $C_6H_5$).

(o) (2S,3S)-N-tert-Butoxycarbonyl- -*tert*-butoxy-norvaline **(47)**

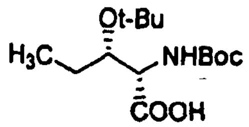

Compound **(46)** (0.024g, 0.074mmol, 1eq), was dissolved in a mixture of $CCl_4/CH_3CN/H_2O$ (1:1:2, v/v/v, 2.4mL). To the stirred biphasic solution $NaHCO_3$ (0.104g, 1.25mmol, 16.9eq) was added as a solid, followed by the careful addition of $NaIO_4$ (0.284g, 1.33mmol, 18eq). After 10 minutes $RuCl_3.3H_2O$ (1.5mg, 7.23 mol, 0.1eq) was added and the reaction mixture stirred for 48hrs. The solution was treated with EtOAc (15mL) and acidified to pH =3 by dropwise addition of citric acid (10%). The organic phase was further extracted with EtOAc (3 x 15mL) and the combined extracts were dried over anhydrous magnesium sulphate, filtered and concentrated *in vacuo.* The crude residue was purified by flash

chromatography over silica gel eluting with a gradient of MeOH /$CH_3Cl$ (0.1:10 to 1.0:10, v/v) to give compound **(47)** as a white solid, yield 0.009g, 42%.
Electrospray-MS m/z 290 ($MH^+$).

(p) (2S,3S)-β-Hydroxy-norvaline **(15)**

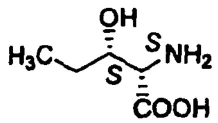

Compound (47) (9mg, 0.03mmol) was dissolved in a solution HCl in dioxane (1 mL, 4.0M). After stirring for 3hrs at room temperature, the solvent was removed *in vacuo* and the residue was lyophilised using $CH_3CN/H_2O$ (4:1, v/v) to yield (2S,3S)- -hydroxynorvaline (15) as a white solid, 3.0 mg, 75%.Electrospray-MS m/z 134 ($MH^+$).
$_H$ (500 MHz; $CD_3OD$) 1.00 (3H, t, *J* 7.5, $CH_3CH_2$), 1.50-1.65 (2H, m, $CH_3CH_2$), 3.88-3.95 (1H, m, CHOH) and 3.98 (1H, d, *J* 3, $C_6H_5CHNH_2$).

Chemistry Towards P2 Hybrid Aminoacids

The general chemistry depicted in scheme 4 will shortly be published in full in the academic literature, by its inventors CS Dexter and RFW Jackson at the University of Newcastle, England.

(a) General Procedure for the zinc coupling reactions

(b) Zinc activation

Zinc dust (150mg, 2.3mmol, 3.0eq, Aldrich) was weighed into a 25mL round bottom flask with a side arm and fitted with a three way tap. The zinc powder was heated with a heat gun under vacuum and the flask was flushed with nitrogen and evacuated and flushed a further three times. With the flask filled with nitrogen, dry DMF (1mL) was added. Trimethylsilylchloride (30μl, 0.23mmol, 0.3eq) was added and the zinc slurry was vigorously stirred for a further 30mins.

(c) Zinc insertion; N-(*tert*-Butoxycarbonyl)-3-iodozinc-L-alanine methyl ester **(61)**

N-(*tert*-Butoxycarbonyl)-3-iodo-L-alanine methyl ester (247mg, 0.75mmol, 1.0eq) dissolved in dry DMF (0.5mL) was added dropwise, via cannula, to the activated zinc slurry at 0°C prepared as described above. The reaction mixture was then allowed to warm up to room temperature and stirred for 1 hr to give the organozinc reagent.

(d) $CuBr.SMe_2$ preparation

Whilst the zinc insertion reaction was in progress, $CuBr.SMe_2$ (20mg, 0.1 mmol, 0.13eq) was weighed into a 25ml round bottom flask fitted with a three way tap and dried "gently" with a heat gun under vacuum until $CuBr.SMe_2$ changed appearance from a brown powder to give a light green powder. Dry DMF (0.5mL) was then added followed by addition of the electrophile (either 1-bromo-2-methylbut-2-ene, toluene-4-sulfonic acid-(E)-2-methyl-but-2-enyl ester or 1-bromo-2,3-dimethylbut-2-ene) (1.0mmol, 1.3eq). The reaction mixture was then cooled to -15°C.

(e) Coupling Reaction

Stirring of the organozinc reagent solution was stopped to allow the zinc powder to settle and the supernatant was carefully removed via cannula (care taken to avoid transferring too much zinc powder) and added dropwise to the solution of electrophile and copper catalyst. The cooling bath was removed and the solution was stirred at room temperature overnight. Ethyl acetate (20mL) was added and stirring was continued for a further 15mins. The reaction mixture was transferred to a separating funnel and a further aliquot of EtOAc (30mL) was added. The organic phase was washed successively with 1M $Na_2S_2O_3$ (20mL), water (2 x 20mL), brine (40mL), dried over sodium sulphate and filtered. The solvent was removed *in vacuo* and the crude product purified by flash chromatography on silica gel as described.

(f) Hydrogenation of alkene

The alkene (1.0mmol) was dissolved in ethanol (10mL), 10% palladium on carbon (80mg) added and hydrogen introduced. Once the reaction had been deemed to have reached completion, the hydrogen was removed, the reaction filtered through Celite and the catalyst washed with ethanol (30mL). The combined organic filtrate was concentrated *in vacuo* and the alkane used directly in the subsequent reaction.

(g) Saponification of methyl ester

The methyl ester (1.0mmol) was dissolved in THF (6mL) and whilst stirring, a solution of LiOH (1.2mmol, 1.2eq) in water (6mL) was added dropwise. Once the reaction was deemed to have reached completion, the THF was removed *in vacuo* and diethyl ether (10mL) added to the residue. The reaction mixture was then acidified with 1.0M HCl until pH =3. The organic phase was then removed and the aqueous layer extracted with diethyl ether (2 x 10mL). The combined organic extracts were dried over magnesium sulphate, filtered and the solvent removed *in vacuo* to give the carboxylic acid used directly in the subsequent reaction.

(h) Removal of N-Boc protecting group

The N-Boc protected material (1.0mmol) was dissolved in DCM (2mL) and cooled to 0°C. Trifluoroacetic acid (2mL) was added dropwise and when the reaction was deemed to have reached completion, the solvents were removed *in vacuo* to yield the amine used directly in the subsequent reaction. Alternatively, the N-Boc protected material (1.0mmol) was cooled to 0°C and 4M HCl in dioxane (5mL) added dropwise and when the reaction was deemed to have reached completion, the solvents were removed *in vacuo* to yield the amine used directly in the subsequent reaction.

(i) Fmoc protection of amine

The amine (1.0mmol) in 1,4-dioxane (2mL) was cooled to 0°C and 10% sodium carbonate (2.2mmol, 2.2eq, 2mL) added. The biphasic reaction mixture was stirred vigorously and Fmoc-Cl (1.1mmol, 1.1eq) added. Once the reaction was deemed to have reached completion, diethyl ether (10mL) added and the reaction mixture acidified to pH = 3 with 1M HCl. The organic phase was removed and the aqueous layer extracted with diethyl ether (2 x 10mL). The combined organic extracts were dried over sodium sulphate, filtered, the solvent removed *in vacuo* and the residue purified by flash chromatography over silica gel.

<u>Example Synthesis 1</u>

Preparation of 2S-2-(9*H*-fluoren-9-ylmethoxycarbonylamino)-4,4-dimethylhexanoic acid (68)

The following scheme explains how optically pure (S)-2-*tert*-Butoxycarbonylamino-4,4-dimethyl-hex-5-enoic acid methyl ester (62) was prepared and isolated.

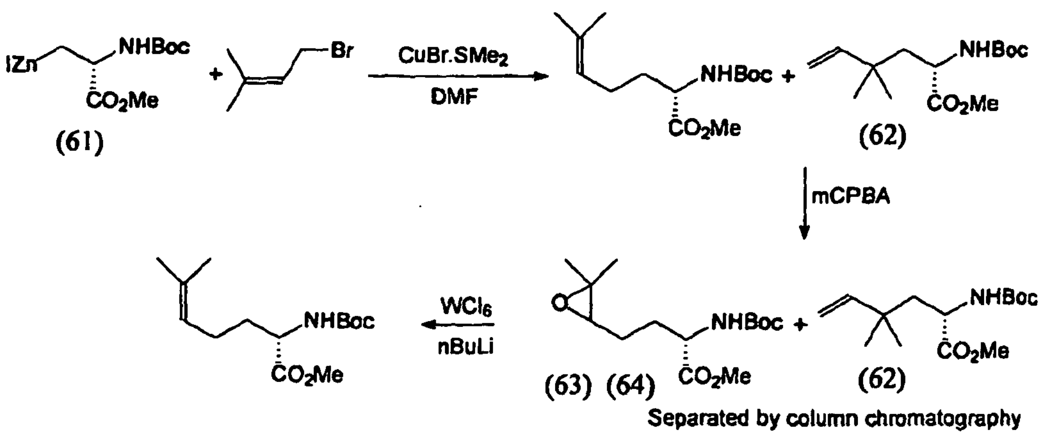

(a) 2S-2-*tert*-Butoxycarbonylamino-4, 4-dimethyl-hex-5-enoic acid methyl ester **(62),** 2S-2-*tert*-butoxycarbonylamino-4-(2S-3,3-dimethyl-oxiranyl)-butyric acid methyl ester **(63)** and 2S-2-*tert*-butoxycarbonylamino-4-(2R-3,3-dimethyloxiranyl)-butyric acid methyl ester **(64)**

Following the general procedure for zinc coupling reactions, 1-bromo-3-methylbut-2-ene (115μL, 1.Ommol) was coupled to compound (61) (247mg, 0.75mmol) in the presence of $CuBr.SMe_2$ (20mg, 0.1 mmol) to give a residue which was purified by flash column chromatography over silica gel eluting with EtOAc / 40:60 petroleum ether (1:9, v/v). Fractions were pooled and reduced *in vacuo* to give a mixture of regioisomers (2:1 formal SN2' *vs* SN2), inseparable by column chromatography, as a colourless oil, yield 190mg, 93%.
To a mixture of regioisomers (190mg, 0.7mmol) in chloroform (3mL) was added dropwise over 5mins, 3-chloroperbenzoic acid (156mg, 85% pure, 0.8mmol, 1.1eq) in chloroform (2mL). The reaction mixture was stirred at room temperature for a further 2hr. The reaction mixture was then washed successively with 1M $Na_2S_2O_5$ (5mL), saturated sodium bicarbonate solution (5mL) and brine (10mL). The organic phase was dried over sodium sulfate, filtered, the solvent removed *in vacuo* and the residue was purified by flash chromatography over silica gel eluting with EtOAc / 40:60 petroleum ether (2:8, v/v). Three products were obtained; compound (62) was eluted first and further elution afforded an inseparable mixture of compound (63) and compound **(64)**. Fractions of the initial component were pooled and reduced *in vacuo* to give 2S-2-*tert*-butoxycarbonylamino-4,4-dimethyl-hex-5-enoic acid methyl ester (62) as a clear oil, yield 93mg, 49%. Electrospray-MS m/z 272 ($MH^+$). Analytical HPLC Rt = 21.45mins (95%), HRMS $C_{10}H_{17}O_4N$ requires *M*, 215.1158, found: $M^+$-$C_4H_8$215.1152 (.- 2.8 ppm); IR (cap. film)/$cm^{-1}$ 3369 (s), 3084 (m), 2965 (s), 1748 (s), 1715 (s), 1517 (s), 1167 (s), 1007 (s), 914 (s)
$\delta_H$ (500 MHz; $CDCl_3$) 1.06 (6H, s, $CH_2$=CHC($C\underline{H}_3)_2$), 1.42 (9H, s, C($C\underline{H}_3)_3$) 1.55 (1H, dd, *J* 14, 9, $CH_2$=CHC($CH_3)_2C\underline{H}_2$A), 1.82 (1H, dd, *J* 14, 3, $CH_2$=CHC($CH_3)_2C\underline{H}_{28}$), 3.69 (3H, s, $OCH_3$), 4.30 (1H, m, NHC$\underline{H}$$CO_2CH_3$), 4.83 (1H, br d, *J* 7, NH), 4.97 (2H, m, $C\underline{H}_2$=CH) and 5.78 (1H, dd, $J_{trans}$ 17.5, $J_{cis}$ 11, $CH_2$=C$\underline{H}$)
$\delta_c$ (125 MHz; $CDCl_3$) 26.93 ($CH_2$=CHC($\underline{C}H_3)_2$), 28.34 (C($\underline{C}H_3)_3$), 36.33 ($CH_2$=CHC($CH_3)_2\underline{C}H_2$), 45.06 ($CH_2$=CH$\underline{C}$($CH_3)_2$), 51.25 (NH$\underline{C}$H$CO_2CH_3$), 52.15 ($OCH_3$), 79.77 ($\underline{C}$($CH_3)_3$), 111.39 ($\underline{C}H_2$=CH), 146.87 ($CH_2$=$\underline{C}$H), 154.97 (NH$\underline{C}O_2Bu^t$) and 174.04 ($\underline{C}O_2CH_3$).

(b) 2S-2-*tert*-Butoxycarbonylamino-4,4-dimethyl-hexanoic acid methyl ester **(65)**

Following the general procedure for alkene hydrogenation, compound **(62)** (93mg, 0.3mmol) yielded compound **(65)** as a colourless oil, yield 90mg, 96% and used directly in the subsequent reaction. Electrospray-MS m/z 274 ($MH^+$). Analytical HPLC Rt = 22.55mins (100%).

(c) 2S-2-*tert*-Butoxycarbonylamino-4,4-dimethyl-hexanoic acid **(66)**

Following the general procedure for methyl ester saponification, compound **(65)** (90mg, 0.3mmol) gave compound **(66)** as crystals, yield 79mg, 92% and used directly in the subsequent reaction. Electrospray-MS m/z 260 ($MH^+$). Analytical HPLC Rt = 20.90mins (100%).

(d) 2S-2-Amino-4,4-dimethyl-hexanoic acid trifluoroacetic acid salt **(67)**

Following the general procedure of N-Boc removal using TFA, compound **(66)** (79mg, 0.3mmol) gave compound **(67)** as a solid, yield 80mg, 96% and used directly in the subsequent reaction. Electrospray-MS m/z 274 ($MH^+$).

(e) 2S-2-(9*H*-Fluoren-9-ylmethoxycarbonylamino)-4,4-dimethyl-hexanoic acid **(68)**

Following the general procedure for Fmoc protection of an amine, compound **(67)** (80mg, 0.3mmol) gave on purification by flash chromatography over silica gel eluting with $CHCl_3$ / $CH_3OH$ (100:0 to 96:4, v/v) 2S-2-(9*H*-fluoren-9-ylmethoxycarbonylamino)-4,4-dimethyl-hexanoic acid **(68)** as a solid, yield 60mg, 54%. Electrospray-MS m/z 382 ($MH^+$). Analytical HPLC Rt = 23.63mins (100%); $[\alpha]_D^{17}$ -18.4 (c 0.25 in EtOH)
$\delta_H$ (500MHz, $CDCl_3$) 0.88 (3H, t, *J* 7, $C\underline{H}_3CH_2$), 0.95 (6H, s, $CH_3CH_2C(C\underline{H}_3)_2$), 1.31 (2H, m, $CH_3C\underline{H}_2$), 1.46 (1H, dd, *J* 14.5, 10, $CH_3CH_2C(CH_3)_2C\underline{H}_{2A}$), 1.85 (1H, br d, *J* 14.5, $CH_3CH_2C(CH_3)_2C\underline{H}_{2B}$), 4.21_(1H, t, *J* 6.5, C$\underline{H}$-Fmoc), 4.41 (3H, m, NHC$\underline{H}$$CO_2$H and $CH_2O$), 5.02 (1H, br d, *J* 8, N$\underline{H}$-Fmoc), 7.29 (2H, m, H-2' and H-7'), 7.38 (2H, m, H-3' and H-6'), 7.58 (2H, m, H-1' and H-8') and 7.74 (2H, d, *J* 7, H-4' and H-5').

Example Synthesis 2

Preparation of 2S,4RS-2-(9H-Fluoren-9-ylmethoxycarbonylamino)-4,5-dimethyl-hexanoic acid **(74)**

Optically pure 2S,4S-2-*tert*-Butoxycarbonylamino-4.5-dimethyl-hex-5-enoic acid methyl ester **(69)** and 2S,4R-2-*tert* Butoxy-carbonylamino-4,5-dimethyl-hex-5-enoic acid methyl ester **(70)** were obtained directly after zinc coupling reaction by flash chromatography.

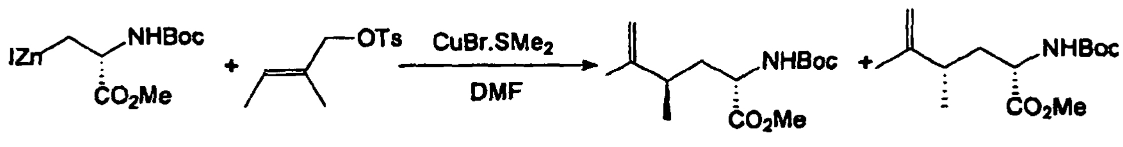


(61) (69) (70)

(a) 2S,4S-2-*tert*-Butoxycarbonylamino-4,5-dimethyl-hex-5-enoic acid methyl ester **(69)** and 2S,4R-2-*tert*-butoxy-carbonylamino-4,5-dimethyl-hex-5-enoic acid methyl ester **(70)**

Following the general procedure for zinc coupling reactions, toluene-4-sulfonic acid (E)-2-methyl-but-2-enyl ester (1.45mL, 1.0mmol) was coupled to compound **(61)** (247mg, 0.75mmol) in the presence of $CuBr.SMe_2$ (20mg, 0.10mmol) to give a residue which was purified by flash chromatography over silica gel eluting with EtOAc / 40:60 petroleum ether (1:9, v/v) to give two diastereoisomers. Analytical HPLC Rt = 22.49mins (60%) and Rt = 22.52mins (40%). Fractions of the first eluted component were pooled to give one of the diastereoisomers obtained as a colourless oil, yield 36mg, 18%. Next a mixture of the diastereomers as a colourless oil, yield 75mg, 37% was obtained. Pure fractions containing the later eluted component were pooled to give the other diastereoisomer as a colourless oil, yield 19mg, 9%. (The stereochemistry at the 4 position was not investigated). Spectral data obtained for the fast running diastereomer: Electrospray-MS m/z 272 ($MH^+$); $[\alpha]_D^{20}$ +12.3 (c 1.06 in $CHCl_3$); IR (cap. film)/$cm^{-1}$ 3382 (s), 3070 (m), 2966 (s), 1746 (s), 1716 (s), 1616 (w), 1507 (s), 886 (m)
$\delta_H$ (500 MHz, $CDCl_3$) 1.06 (3H, d, *J* 7, $CH_3CH$), 1.45 (9H, s, $C(CH)_3)_3$, 1.58 (1H, m, $CH_3CH$), 1.68 (3H, s, $CH_3C{=}CH_2$), 1.85 (1H. m, $CH_2CH$), 1.97 (1H. m, $CH_{2B}CH$), 3.73 (3H, s, $OCH_3$), 4.29 (1H. m, $NHCHCO_2CH_3$), 4.72 (1H. s, $CH_{2A}{=}CH$), 4.95 (1H, d, *J* 1.5, $CH_{2B}{=}CH$) and 5.04 (1H, d, J7, NH) $\delta_c$ (125 MHz, $CDCl_3$) 18.61 ($CH_3C{=}CH_2$), 21.64 ($CH_3CH$), 28.32 ($C(CH_3)_3$), 30.79 ($CH_3CHCH_2$), 38.06 ($CH_2CHNH$), 52.00 ($NHCHCO_2CH_3$), 52.22 ($OCH_3$), 79.53 ($C(CH_3)_3$), 110.19 ($CH_2{=}C(CH_3)$), 144.62 ($CH_2{=}C(CH_3)$), 155.18 (OCONH) and 173.30 ($CO_2CH_3$).

Spectral data obtained for the slow running diastereoismer: Electrospray-MS m/z 272 ($MH^+$); $[\alpha]_D^{20}$ +16.0 (c 0.60 in $CHCl_3$); IR (cap. film)/$cm^{-1}$ 3369 (s), 3073 (m), 2969 (s), 1747 (s), 1717 (s), 1617 (w), 1517 (s), 893 (m)
$\delta_H$ (500 MHz, $CDCl_3$) 1.04 (3H, d, *J* 7, $CH_3CH$), 1.44 (9H, s, $C(CH_3)_3$), 1.55 (1H, m, $CH_3CH$), 1.67 (3H, s, $CH_3C{=}CH_2$), 1.91 (1H, m, $CH_{2A}CH$), 2.37 (1H, m, $CH_{2B}CH$), 3.73 (3H, s, $OCH_3$), 4.26 (1H, m, $NHCHCO_2CH_3$), 4.75 (1H, d, *J* 1.5, $CH_{2A}{=}CH$), 4.79 (1H, d, *J* 1.5, $CH_{2B}{=}CH$) and 5.46 (1H, d, *J* 6.1, NH) $\delta_c$ (125 MHz, $CDCl_3$) 18.51 ($CH_3C{=}CH_2$), 20.14 ($CH_3CH$), 28.31 ($C(CH_3)_3$), 30.55 ($CH_3CHCH_2$), 37.64 ($CH_2CHNH$), 52.17 ($NHCHCO_2CH_3$), 52.22 ($OCH_3$), 79.74 ($C(CH_3)_3$), 111.27 ($CH_2{=}C(CH_3)$), 147.94 ($CH_2{=}C(CH_3)$). 155.36 (OCONH) and 173.83 ($CO_2CH_3$).

These diastereoisomers were not separated routinely and used as a mixture in subsequent reactions.

(b) 2S,4RS-2-*tert*-Butoxycarbonylamino-4,5-dimethyl-hexanoic acid methyl ester **(71)**

Following the general procedure for alkene hydrogenation, compounds **(69)** and compound **(70)** (130mg, 0.48mmol) yielded a mixture of two diastereoisomers **(71)** which were not separated, obtained as a colourless oil, yield 128mg, 98%. Analytical HPLC Rt 22.49mins, electrospray-MS m/z 274 ($MH^+$).

(c) 2S,4RS-2-*tert*-Butoxycarbonylamino-4,5-dimethyl-hexanoic acid **(72)**

Following the general procedure for methyl ester saponification, compounds **(71)** (128mg, 0.47mmol) gave a inseparable mixture of compounds **(72)** as a colourless oil, yield 106mg, 87%. Electrospray-MS m/z 260 ($MH^+$). Analytical HPLC Rt = 20.65mins (100%).

(d) 2S,4RS-2-Amino-4,5-dimethyl-hexanoic acid trifluoroacetic acid salt **(73)**

Following the general procedure of N-Boc removal using TFA, compounds **(72)** (106mg, 0.41mmol) gave an inseparable mixture of compounds **(73)** as a solid, yield 107mg, 96% and used directly in the subsequent reaction. Electrospray-MS m/z 160 ($MH^+$).

(e) 2S,4RS-2-(9H-Fluoren-9-ylmethoxycarbonylamino)-4,5-dimethyl-hexanoic acid **(74)**

Following the general procedure for Fmoc protection of an amine, compounds **(73)** (107mg, 0.39mmol) gave on purification by flash chromatography over silica gel eluting with $CHCl_3$ / $CH_3OH$ (100:0 to 95:5, v/v) 2S,4RS-2-(9H-fluoren-9-ylmethoxycarbonylamino)-4,5-dimethyl-hexanoic acid **(74)** as a solid, yield 60mg, 40% as a mixture of two diastereoisomers. Analytical HPLC Rt = 23.83mins (40%) and Rt = 24.06mins (60%). First eluted diastereomer: Electrospray-MS m/z 382 ($MH^+$). Later eluted diastereomer: Electrospray-MS m/z 382 ($MH^+$).

Example Synthesis 3

Preparation of 2S,5RS-2-(9H-Fluoren-9-ylmethoxycarbonylamino)-5,6-dimethyl-heptanoic acid (80) and 2S-2-(9H-Fluoren-9-ylmethoxycarbonylamino)-4,4,5-trimethyl-hexanoic acid (84)

(S)-2-*tert*-butyloxycarbonylamino-5,6-dimethyl-hept-5-enoic methyl ester **(75)** and (S)-2-*tert*-butyloxycarbonylamino-4,4,5-trimethyl-hex-5-enoic methyl ester **(76)** were obtained directly after zinc coupling reaction by flash chromatography.

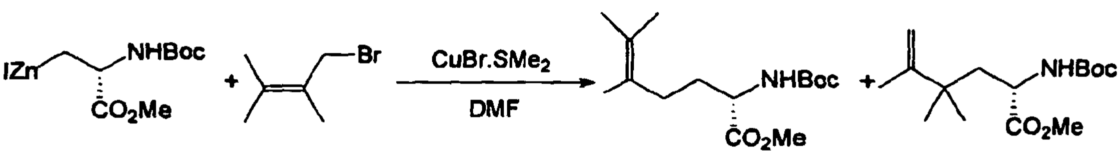


(61) (75) (76)

(a) 2S-2-*tert*-Butyloxycarbanylamino-5,6-dimethyl-hept-5-enoic methyl ester **(75)** and 2S-2-*tert*-butyloxycarbonylamino-4,4,5-trimethyl-hex-5-enoic methyl ester **(76)**

Following the general procedure for zinc coupling reactions, 1-bromo-2,3-dimethylbut-2-ene (163mg, 1.Ommol) was coupled to compound (61) (247mg, 0.75mmol) in presence of $CuBr.SMe_2$ (20mg, 0.10mmol) to give a residue which on purification by flash chromatography over silica gel eluting with EtOAc/ 40:60 petroleum ether (1:9) gave two regioisomers. The first eluted component compound **(75)** as a colourless oil, yield 60mg, 28% and the second eluted component was compound **(76)** as a colourless oil, yield 51mg, 24%.

Spectral data obtained for compound **(75);** Electrospray-MS m/z 285 ($MH^+$). Analytical HPLC Rt = 22.85mins (100%); HRMS $C_{15}H_{27}NO_4$ requires M, 285.1940, found: $M^+$ 285.1954 (- 4.9 ppm); $[\alpha]_D^{22}$ +26.1 (c 1.01 in $CH_2Cl_2$); elemental analysis $C_{15}H_{27}NO_4$ (req) %C 63.1, %H 9.5, %N 4.9, (fnd) %C 62.4, %H 9.6, %N 5.3; IR (cap. film)/cm$^{-1}$ 3366 (s), 3154 (m), 2978 (s), 1744 (s), 1718 (s), 1506 (s), 1366 (s), 1164 (s)

$\delta_H$ (500 MHz, $CDCl_3$) 1.45 (9H, s, $C(C\underline{H}_3)_3$), 1.62 (9H, m, $(C\underline{H}_3)_2=C(C\underline{H}_3)$), 1.87 (1H, m, $C\underline{H}_{2A}CH_2CH$), 2.03 (1H, m, $C\underline{H}_{2B}CH_2CH$), 2.09 (1H, dd, *J* 6, 10.5, $CH_2C\underline{H}_{2A}CH$), 2.12 (1H, dd, *J* 6.5, 10.5, $CH_2C\underline{H}_{2B}CH$), 3.74 (3H, s, $OCH_3$), 4.29 (1H, m, $NHC\underline{H}CO_2CH_3$) and 5.02 (1H, d, *J* 7, NH)

$\delta_C$ (125 MHz, $CDCl_3$) 18.19 ($(CH_3)_2C=C(\underline{C}H_3)$), 20.00 ($(\underline{C}H_3)_{2cis}C=C(CH_3)$), 20.61 ($(\underline{C}H_3)_{2trans}C=C(CH_3)$), 28.33 ($C(\underline{C}H_3)_3$), 30.07 ($CH_2\underline{C}H_2CH$), 30.92 ($\underline{C}H_2CH_2CH$), 52.20 ($NH\underline{C}HCO_2CH_3$), 53.47 ($OCH_3$), 80.00 ($\underline{C}(CH_3)_3$), 95.90 ($(CH_3)_2\underline{C}=C(CH_3)$), 96.49 ($(CH_3)_2C=\underline{C}(CH_3)$), 155.33 (OCONH) and 173.42 ($CO_2CH_3$).

Spectral data obtained for compound (76); Electrospray-MS m/z 285 ($MH^+$). Analytical HPLC Rt = 22.91mins (100%); HRMS $C_{11}H_{19}NO_4$ requires *M* 229.1314, found: $M^+$-$C_4H_8$ 229.1309 (- 2.2 ppm); $[\alpha]_D^{23}$ +4.8 (c 1.01 in $CH_2Cl_2$); elemental analysis $C_{15}H_{27}NO_4$ (req) %C 63.1, %H 9.5, %N 4.9, (fnd) %C 62.5, %H 9.5, %N; IR (cap. film)/cm$^{-1}$ 3368 (s), 3091 (m), 2934 (s), 1748 (s), 1717 (s), 1516 (s)

$\delta_H$ (500 MHz, $CDCl_3$) 1.10 (3H, s, $(CH_3)_{2A}C$), 1.12 (3H, s, $(CH_3)_{2B}C$), 1.43 (9H, s, $C(CH_3)_3$), 1.60 (1H, m, $C\underline{H}_{2A}CH$), 1.74 (3H, s, $C\underline{H}_3C=CH_2$), 1.92 (1H, dd, *J* 14.5, 4, $C\underline{H}_{2B}CH$), 3.70 (3H, s, $OCH_3$), 4.24 (1H, m, $NHC\underline{H}CO_2CH_3$), 4.79 (1H, s, $C\underline{H}_{2A}=C(CH_3)$), 4.82 (1H, s, $C\underline{H}_{2B}=C(CH_3)$) and 4.83 (1H, br d, *J* 11, NH) $\delta_c$ (125 MHz, $CDCl_3$) 19.38 ($CH_3$), 27.19 ($CH_3$), 27.61 ($CH_3$), 28.34 ($C(\underline{C}H_3)_3$). 38.50 ($\underline{C}H_2CH$), 38.95 ($(CH3)_2\underline{C}$). 51.34 ($NH\underline{C}HCO_2CH_3$), 52.13 ($OCH_3$), 79.71

($\underline{C}(CH_3)_3$), 110.95 ($\underline{C}H_2=C(CH_3)$), 150.62 ($CH_2=\underline{C}(CH_3)$), 155.00 (OCONH) and 174.24 ($\underline{C}O_2CH_3$).

(b) 2S,5RS-2-*tert*-Butoxycarbonylamino-5,6-dimethyl-heptanoic acid methyl ester **(77)**

Following the general procedure for alkene hydrogenation, 2S-2-*tert*-butyloxycarbonylamino-5,6-dimethyl-hept-5-enoic methyl ester **(75)** (60mg, 0.21 mmol) yielded compound **(77)** as a colourless oil, yield 54mg, 89%. Electrospray-MS m/z 288 ($MH^+$). Analytical HPLC Rt = 24.06mins (100%).

(c) 2S,5RS-2-*tert*-Butoxycarbonylamino-5,6-dimethyl-heptanoic acid **(78)**

Following the general procedure for methyl ester saponification, compounds **(77)** (54mg, 0.19mmol) gave compounds **(78)** as a colourless oil, yield 54mg, 100%. Electrospray-MS m/z 274 ($MH^+$). Analytical HPLC Rt = 21.44mins (100%).

(d) 2S,5RS-2-Amino-5,6-dimethyl-heptanoic acid hydrochloride salt (79)

Following the general procedure of N-Boc removal using 4M HCl in dioxane, compounds **(78)** (54mg, 0.20mmol) gave compounds **(79)** as a solid, yield 40mg, 97%. Electrospray-MS m/z 174 ($MH^+$).

(e) 2S,5RS-2-(9H-Fluoren-9-ylmethoxycarbonylamino)-5,6-dimethylheptanoic acid (80)

Following the general procedure for Fmoc protection of an amine, compounds **(79)** (40mg, 0.19mmol) gave on purification by flash chromatography over silica gel eluting with $CHCl_3$ / $CH_3OH$ (100:0 to 95:5, v/v) 2S,5RS-2-(9H-fluoren-9-ylmethoxycarbonylamino)-5,6-dimethyl-heptanoic acid **(80)** as a solid, yield 27mg, 36%. Electrospray-MS m/z 395 ($MH^+$). Analytical HPLC Rt = 24.52mins (100%), HRMS $C_{24}H_{29}O_4NNa$ requires *M* 418.1994, found: $MNa^+$, 418.1993. (- 0.38 ppm)
$\delta_H$ (500 MHz; $CDCl_3$) 0.73 (6H, m, ($C\underline{H}_3)_2CH$), 0.82 (3H, d, *J* 6.5, $(CH_3)_2CHCH(C\underline{H}_3)$), 1.23 (1H, m, $(CH_3)_2CHCH(CH_3)C\underline{H}_{2A}$, 1.39 (1H, m, $(CH_3)_2CHCH(CH_3)C\underline{H}_{2B}$), 1.55 (2H, m, $(CH_3)_2CHC\underline{H}(CH_3)$ and $(CH_3)_2CHCH(CH_3)CH_2C\underline{H}_{2A}$), 1.63 (1H, m, $(CH_3)_2C\underline{H}CH(CH_3)$), 1.90 (1H, m, $(CH_3)_2CHCH(CH_3)CH_2C\underline{H}_{2B}$), 4.18 (1H, t, *J* 6.5, C$\underline{H}$-Fmoc), 4.40 (3H, m, $NHC\underline{H}CO_2H$ and $CH_2O$), 5.30 (1H, br d, *J* 8, N$\underline{H}$-Fmoc), 7.27 (2H, m, H-2' and H-7'), 7.37 (2H, m, H-3' and H-6'), 7.56 (2H, m, H-1' and H-8') and 7.75 (2H, d, *J* 7, H-4' and H-5')
$\delta_c$ (125 MHz; $CDCl_3$) 14.91 $(CH_3)_2CHCH(\underline{C}H_3)$), 17.49 and 17.73 $((\underline{C}H_3)_{2A}CH)$, 19.93 and 20.05 $((\underline{C}H_3)_{2B}CH)$, 28.08 $((CH_3)_2\underline{C}H)$, 29.26 and 29.44 $((CH_3)_2CHCH(CH_3)CH_2\underline{C}H_2)$, 30.04 and 30.17 $((CH_3)_2CHCH(CH_3)\underline{C}H_2CH_2)$, 31.38 and 31.68 $((CH_3)_2CH\underline{C}H(CH_3))$, 37.89 and 38.07 $(NH\underline{C}HCO_2H)$, 46.88 (CH-1'), 66.84 ($CH_2O$), 119.72 (CH-5' and CH-10'), 124.80 (CH-4' and CH-11'), 126.81 (CH-6' and CH-9'), 127.46 (CH-3' and CH-12'), 141.05 (C-7' and C-8'), 143.47 (C-2' and C-13') and 155.89 (OCONH). The quaternary signal for the carboxylic acid was not observed.

(f) 2S-2-*tert*-Butoxycarbonylamino-4,4,5-trimethyl-hexanoic acid methyl ester **(81)**

Following the general procedure for alkene hydrogenation, 2S-2-*tert*-butyloxycarbonylamino-4,4,5-trimethyl-hex-5-enoic methyl ester **(76)** (51mg, 0.18mmol) yielded compound **(81)** as a colourless oil, yield 46mg, 90%. Electrospray-MS m/z 288 ($MH^+$). Analytical HPLC Rt = 22.91 mins (100%).

(g) 2S-2-*tert*-Butoxycarbonylamino-4,4,5-trimethyl-hexanoic acid **(82)**

Following the general procedure for methyl ester saponification, compound **(81)** (46mg, 0.16mmol) gave compound **(82)** as a colourless oil, yield 44mg, 100%. Electrospray-MS m/z 274 ($MH^+$).

(h) 2S-2-Amino-4,4,5-trimethyl-hexanoic acid hydrochloride salt **(83)**

Following the general procedure of N-Boc removal using 4M HCl in dioxane, compound **(82)** (44mg, 0.16mmol) gave compound **(83)** as a solid, yield 33mg, 99%. Electrospray-MS m/z 174 ($MH^+$).

(i) 2S-2-(9H-Fluoren-9-ylmethoxycarbonylamino)-4,4,5-trimethyl-hexanoic acid **(84)**

Following the general procedure for Fmoc protection of an amine, compound **(83)** (33mg, 0.16mmol) gave on purification by flash chromatography over silica gel eluting with $CHCl_3$/ $CH_3OH$ (100:0 to 95:5, v/v) 2S-2-(9H-fluoren-9-ylmethoxycarbonylamino)-4,4,5-trimethyl-hexanoic acid (84) as a solid, yield 20mg, 32%. Electrospray-MS m/z 396

$(MH^+)$. Analytical HPLC Rt = 24.28mins (100%), HRMS $C_{24}H_{29}O_4NNa$ requires M 418.1994, found: $MNa^+$, 418.1993. (- 0.38 ppm)

$\delta_H$ (500 MHz; $CDCl_3$) 0.93 (9H, m, $(C\underline{H}_3)_2CHC(C\underline{H}_3)_{2A}$), 0.98 (3H, s, $(CH_3)_2CHC(C\underline{H}_3)_{2B}$), 1.48 (1H, dd, *J* 14, 10, $(CH_3)_2CHC(CH_3)_2C\underline{H}_{2A}$), 1.57 (1H, m, $(CH_3)_2C\underline{H}$), 1.91 (1H, d, *J* 14, $(CH_3)_2CHC(CH_3)_2C\underline{H}_{2B}$), 4.21 (1H, t, *J* 6.5, $C\underline{H}$-Fmoc), 4.40 (3H, m, $NHC\underline{H}CO_2H$ and $CH_2O$), 5.10 (1H, br d, *J* 7.5, $N\underline{H}$-Fmoc), 7.27 (2H, m, H-2' and H-7'), 7.36 (2H, m, H-3' and H-6'), 7.57 (2H, m, H-1' and H-8') and 7.74 (2H, d, *J* 7, H-4' and H-5')

$\delta_c$ (125 MHz; $CDCl_3$) 17.01 ($(\underline{C}H_3)_{2A}CH$). 17.16 ($(\underline{C}H_3)_{2B}CH$), 23.69 ($(CH_3)_2CHC(\underline{C}H_3)_{2A}$), 24.27 ($(CH_3)_2CHC(\underline{C}H_3)_{2B}$), 35.27 ($(CH_3)_2CH\underline{C}(CH_3)_2$), 35.73 ($(CH_3)_2\underline{C}H$), 41.88 ($(CH_3)_2CHC(CH_3)_2\underline{C}H_2$), 46.93 (CH-1'), 54.20 ($NH\underline{C}HCO_2H$), 66.79 ($CH_2O$), 119.70 (CH-5' and CH-10'), 124.78 (CH-4' and CH-11'), 126.79 (CH-6' and CH-9'), 127.44 (CH-3' and CH-12'). 141.05 (C-7' and C-8'), 143.61 (C-2' and C-13') and 155.68 (OCONH). The quaternary signal for the carboxylic acid was hot observed.

General Solid Phase procedures

Molecules were assembled using the furanone and pyranone building blocks and novel protected aminoacids described earlier, by solid phase procedures on Chiron multipins following the protocols detailed below.

Preparation of Building Block-Linker Constructs

General method for the synthesis of dihydro-3(2H)-furanone Linker Constructs - See Scheme 5 above

Dihydro-3(2H)-furanone **(18, 24-28),** (1.0eq) was dissolved in a mixture of ethanol / water (7:1 v/v, 10mL per mmole compound) containing sodium acetate trihydrate (1.5eq). 4-[[(hydrazinocarbonyl)amino]methyl]-cyclohexanecarboxylic acid trifluoro acetate (mw 329.3, 1.0eq) (see Murphy, A. M., *et al, J. Am. Chem. Soc,* **114**, 3156-3157, 1992) was added and the mixture heated under reflux for 2hrs. The mixture was then cooled, poured into dichloromethane (100mL per mmole compound) and water (100mL) added. The organic layer was separated, backwashed with saturated brine (100mL). The organic layer was dried ($Na_2SO_4$), filtered and evaporated *in vacuo* to yield a white solid. Yield 85 - 105% crude weight. Constructs **(29-34)** were used without further purification

Preparation of Crown Assembly

The compounds were synthesised in parallel fashion using the appropriately loaded Fmoc-Building block-linker-DA/MDA derivatised macrocrowns (see above) loaded at approximately 3.5 - 9.1 μmoles per crown. Prior to synthesis each crown was connected to its respective stem and slotted into the 8 x 12 stem holder. Coupling of the amino acids employed standard Fmoc amino acid chemistry as described in Solid Phase Peptide Synthesis', E. Atherton and R.C. Sheppard, IRL Press Ltd, Oxford, UK, 1989.

Removal of Nα-Fmoc Protection

A 250 mL solvent resistant bath is charged with 200 mL of a 20% piperidine/DMF solution. The multipin assembly is added and deprotection allowed to proceed for 30 minutes. The assembly is then removed and excess solvent removed by brief shaking. The assembly is then washed consecutively with (200 mL each), DMF (5 minutes) and MeOH (5 minutes, 2 minutes, 2 minutes) and left to air dry for 15 minutes.

Quantitative UV Measurement of Fmoc Chromophore Release

A 1cm path length UV cell is charged with 1.2 mL of a 20% piperidine/DMF solution and used to zero the absorbance of the UV spectrometer at a wavelength of 290nm. A UV standard is then prepared consisting of 5.0 mg Fmoc-Asp(OBut)-Pepsyn KA (0.08 mmol/g) in 3.2 mL of a 20% piperidine/DMF solution. This standard gives $Abs_{290}$ = 0.55-0.65 (at room temperature). An aliquot of the multipin deprotection solution is then diluted as appropriate to give a theoretical $Abs_{290}$ = 0.6, and this value compared with the actual experimentally measured absorbance showing the efficiency of previous coupling reaction.

Standard Coupling Of Amino Acid Residues

Coupling reactions are performed by charging the appropriate wells of a polypropylene 96 well plate with the pattern of activated solutions required during a particular round of coupling. Macrocrown standard couplings were performed in DMF (500 μl).

Coupling of an Amino-acid Residue To Appropriate Well

Whilst the multipin assembly is drying, the appropriate $N_{\alpha}$-Fmoc amino acid pfp esters (10 equivalents calculated from the loading of each crown) and HOBt (10 equivalents) required for the particular round of coupling are accurately weighed into suitable containers. Alternatively, the appropriate $N_{\alpha}$-Fmoc amino acids (10 equivalents calculated from the loading of each crown), desired coupling agent e.g. HBTU (9.9 equivalents calculated from the loading of each crown) and activation e.g. HOBt (9.9 equivalents calculated from the loading of each crown), NMM (19.9 equivalents calculated from the loading of each crown) are accurately weighed into suitable containers.
The protected and activated Fmoc amino acid derivatives are then dissolved in DMF (500 μl for each macrocrown e.g. for 20 macrocrowns, 20 x 10 eq. x 7 μmoles of derivative would be dissolved in 10 mL DMF). The appropriate derivatives are then dispensed to the appropriate wells ready for commencement of the 'coupling cycle'. As a standard, coupling reactions are allowed to proceed for 6 hours. The coupled assembly was then washed as detailed below.

Washing Following Coupling

If a 20% piperidine/DMF deprotection is to immediately follow the coupling cycle, then the multipin assembly is briefly shaken to remove excess solvent washed consecutively with (200 mL each), MeOH (5 minutes) and DMF (5 minutes) and de-protected. If the multipin assembly is to be stored or reacted further, then a full washing cycle consisting brief shaking then consecutive washes with (200 mL each), DMF (5 minutes) and MeOH (5 minutes, 2 minutes, 2 minutes) is performed.

Addition of Capping Group

Whilst the multipin assembly is drying, the appropriate acid capping group (10 equivalents calculated from the loading of each crown), desired coupling agent e.g. HBTU (9.9 equivalents calculated from the loading of each crown) and activation e.g. HOBt (9.9 equivalents calculated from the loading of each crown), NMM (19.9 equivalents calculated from the loading of each crown) are accurately weighed into suitable containers. The acid derivatives coupling agents are then dissolved in DMF (500 μl for each macrocrown e.g. for 20 macrocrowns, 20 x 10 eq. of derivative would be dissolved in 10 mL DMF) and left to activate for 5 minutes. The appropriate derivatives are then dispensed to the appropriate wells ready for commencement of the 'capping cycle'. As a standard, capping reactions are allowed to proceed for 18 hours overnight. The capped assembly was then washed as detailed above.

Acidolytic Mediated Cleavage of Molecule-Pin Assembly

Acid mediated cleavage protocols are strictly performed in a fume hood. A polystyrene 96 well plate (1 mL/ well) is labelled and weighed to the nearest mg. Appropriate wells are then charged with a trifluoroacetic acid/water (95: 5, v/v, 600 μl) cleavage solution, in a pattern corresponding to that of the multipin assembly to be cleaved.
The multipin assembly is added, the entire construct covered in tin foil and left for 2 hours. The multipin assembly in then added to another polystyrene 96 well plate (1 mL/wett) containing trifluoroacetic acid/water (95:5, v/v, 600 l) (as above) for 5 minutes.

Work up of Cleaved Molecules

The primary polystyrene cleavage plate (2 hour cleavage) and the secondary polystyrene plate (5 minute wash) are then placed in the GeneVac evaporator and the solvents removed (minimum drying rate) for 90 minutes. The contents of the secondary polystyrene plate are transferred to their corresponding wells on the primary plate using an acetonitrile/ water (50: 50 v/v/v) solution (3 x 150 μl) and the spent secondary plate discarded. Aliqouts (5-20 L) are taken for analysis. The plate was covered in tin foil, pin-pricked over wells containing compounds, placed into the freezer for 1hr, then lyophilised.

Analysis and Purification of Molecules

The (5-20 L) aliquots are analysed by analytical HPLC and electrospray-MS. In virtually all cases, crude purities are >90% by HPLC with the desired m/z. Sample were purified by semi-preparative reverse phase HPLC, using Vydac $C_4$.Appropriate fractions are combined and lyophilised in tared 10mL glass vials, then re-weighed. Molecules were prepared on a 15-90μmole scale, yielding 2.0-26.0mg of purified products. The purity of each product was confirmed by analytical HPLC at >95% (215nm UV detection) and gave the appropriate $[MH]^+$ by electrospray mass spectrometry analysis.

Loading of Macrocrowns With Constructs

General method for the loading of multipins with Dihydro-3(2H)-Furanone - Linker Constructs (29-34) Amino functionalised DA/MDA macrocrowns (ex Chiron Mimotopes, Australia, 9.1 μmole loading) or amino functionalised HEMA gears (ex Chiron Mimotopes, Australia, 1.3μmole loading) were used for all loadings and subsequent solid phase syntheses.

Dihydro-3(2H)-Furanone- Linker Construct (29-34) (3eq compared to total surface functionalisation of crowns / gears) was carboxyl activated with 2-(1H-benzotriazole-1-yl)-1,1,3,3-tetramethyluronium hexafluorophosphate (3eq), 1-hydroxybenzotriazole (3eq) and N-methylmorpholine (6eq) in dimethylformamide (5mL) for 5mins. This mixture was added to the crowns /gears, additional DMF added to cover the reaction surface and the mixture left overnight.

Standard washing and Fmoc deprotection readings (see procedures above) indicated virtually quantitative loading.

Exemplar molecules prepared by the methods using the respective furanone, R3 amino acid and capping group in the method detailed above are shown in Table 1:

| Electrospray-MS m/z ($MH^+$) | NAME |
|---|---|
| 385 | Benzofuran-2-carboxylic acid [1S-(2R-methyl-4-oxo-tetrahydro-furan-3S-ylcarbamoyl)-cyclohexyl]-amide |
| 383 | Benzofuran-2-carboxylic acid [1-(2R-methyl-4-oxo-tetrahydro-furan-3S-ylcarbamoyl)-cyclohexyl]-amide |
| 371 | Benzofuran-2-carboxylic acid [2-cyclopropyl-1S-(2R-methyl-4-oxo-tetrahydro-furan-3S-ylcarbamoyl)-ethyl]-amide |
| 398. | *N*-[3-Methyl-1S-(2R-methyl-4-oxo-tetrahydro-furan-3S-ylcarbamoyl)-butyl]-4-pyrrol-1-yl-benzamide |
| 383 | Naphthalene-1-carboxylic acid [3-methyl-1S-(2R-methyl-4-oxo-tetrahydro-furan-3S-ylcarbamoyl)-butyl]-amide |
| 373 | Benzofuran-2-carboxylic acid [3-methyl-1S-(2R-methyl-4-oxo-tetrahydro-furan-3S-ylcarbamoyl)-butyl]-amide |
| 389 | Benzo[b]thiophene-2-carboxylic acid [3-methyl-1S-(2R-methyl-4-oxo-tetrahydro-furan-3S-ylcarbamoyl)-butyl]-amide |
| 403 | 5-Methoxy-benzofuran-2-carboxylic acid [3-methyl-1S-(2R-methyl-4-oxo-tetrahydro-furan-3S-ylcarbarmoyl)-butyl]-amide |
| 401 | 5-Methoxy-benzofuran-2-carboxylic acid [3-methyl-1S-(2R-methyl-4-oxo-tetrahydro-furan-3-ylcarbamoyl)-but-3S-enyl]-amide |
| 390 | 4-Acetylamino-*N*-[3-methyl-1S-(2R-methyl-4-oxo-tetrahydro-furan-3S-ylcarbamoyl)-butyl]-benzamide |
| 448 | 4-Hydroxy-*N*-[3-methyl-1S-(2R-methyl-4-oxo-tetrahydro-furan-3S-ylcarbamoyl)-butyl]-3-morpholin-4-ylmethyl-benzamide |
| 446 | 4-Hydroxy-*N*-[3-methyl-1S-(2R-methyl-4-oxo-tetrahydro-furan-3S-ylcarbamoyl)-but-3-enyl]-3-morpholin-4-ylmethyl-benzamide |
| 409 | Biphenyl-4-carboxylic acid [3-methyl-1S-(2R-methyl-4-oxo-tetrahydro-furan-3S-ylcarbamoy) -butyl]-amide |
| 389 | 4-*tert*-Butyl-*N*-[3-methyl-1S-(2R-methyl-4-oxo-tetrahydro-furan-3S-ylcarbamoyl)-butyl]-benzamide |
| 387 | 4-*tert*-Butyl-*N*-[3-methyl-1S-(2R-methyl-4-oxo-tetrahydro-furan-3S-ylcarbamoyl)-but-3-enyl]-benzamide |
| 390 | 4-Guanidino-*N*-[3-methyl-1S-(2R-methyl-4-oxo-tetrahydrofuran-3S-ylcarbamoyl)-butyl]-benzamide |
| 388 | 4-Guanidino-*N*-[3-methyl-1S-(2R-methyl-4-oxo-tetrahydro-furan-3S-ylcarbamoyl)-but-3-enyl]-benzamide |
| 502 | 5-(2-Morpholin-4-yl-ethoxy)-benzofuran-2-carboxylic acid [3-methyl-1S-(2R-methyl-4-oxo-tetrahydro-furan-3S-ylcarbamoyl)-butyl]-amide |
| 500 | 5-(2-Morpholin-4-yl-ethoxy)-benzofuran-2-carboxylic acid [3-methyl-1S-(2R-methyl-4-oxo-tetrahydro-furan-3S-ylcarbamoyl)-but-3-enyl]-amide |

Additional compounds of the invention were prepared as follows:

i) Preparation of N-[3-Methyl-1-(2-methyl-4-oxo-tetrahydro-furan-3-ylcarbamoyl)-butyl]-4-pyrrolidin-1-yl-benzamide

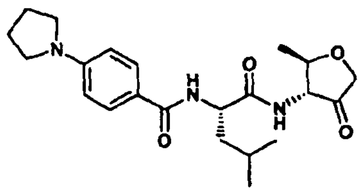

Following the procedure of Example 1 step d) except substituting "4-pyrrolidin-1-yl-benzoic acid" for the model carboxylic acid. These acids were prepared by employing two reactions: the initial esters were prepared using Buchwald type chemistry and subsequent standard hydrolysis of the esters provided the required acids.

a. 4-Pyrrolidin-1-yl-benzoic acid methyl ester

An oven-dried reaction tube was charged with cesium carbonate (2.12g, 6.51mmol) that had been finely ground with a pestle and mortar under an atmosphere of argon. Tris(dibenzylideneacetone)dipalladium(0) (42.5mg, 1.5mol%) and (S)-(-)-2,2'-bis(diphenylphosphino)-1,1'-binaphthyl (43.4mg, 1.5mol%) were added and the tube charged with argon. Pyrrolidine (0.39g, 5.58mmol), methyl-4-bromobenzoate (1.00g, 4.65mmol) and toluene (10ml) were added and the mixture heated to 100°C with vigorous stirring until the starting material had been consumed as judged by hplc. The mixture was cooled to room temperature, diluted with ether (20ml), filtered and concentrated. Purification by column chromatography gave the title compound (0.80g, 84%) as a solid. MS (M+H+): 206.

b. 4-Pyrrolidin-1-yl-benzoic acid salt

4-Pyrrolidin-1-yl-benzoic acid methyl ester (200mg, 0.98mmol) was dissolved in methanol (4ml) and sodium hydroxide (39mg, 0.98mmol) in water (2ml) was added. The mixture was heated to 60°C until the starting material had been consumed as judged by hplc. The mixture was cooled to room temperature, diluted with water (20ml), filtered and freeze dried to give the title compound (0.200g, 97%) as a solid. MS (M+H+): 192.
An alternative procedure is described below:
4-Pyrrolidin-1-yl-benzoic acid methyl ester (200mg, 0.98mmol) was dissolved in concentrated hydrochloric acid: water (1:1) (4ml). The mixture was heated at reflux until the starting material had been consumed as judged by hplc. The mixture was cooled to room temperature, diluted with water (10ml), filtered and freeze dried to give the title compound (0.190g, 97%) as a solid. MS (M+H+): 192.

Preparation of N-[3-Methyl-1-(2-methyl-4-oxo-tetrahydro-furan-3-ylcarbamoyl)-butyl]-4-piperidin-1-yl-benzamide

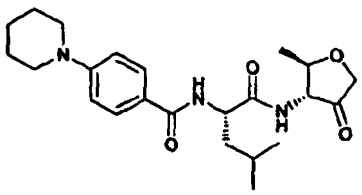

Following the procedure of Example 1 step d) except substituting "4-piperidin-1-yl-benzoic acid"

4-Piperidin-1-yl-benzoic acid methyl ester

Following the procedure above except substituting "piperidine" for "pyrrolidine" gave the title compound: MS (M+H+): 220.

a. 4-Piperidin-1-yl-benzoic acid salt

Following the procedure above except substituting "4-piperidin-1-yl-benzoic acid methyl ester" for "4-pyrrolidin-1-yl-benzoic acid methyl ester" gave the title compound: MS (M+H+): 206.

Preparation of N-[3-Methyl-1-(2-methyl-4-oxo- tetrahydro-furan-3-ylcarbamoyl)-butyl]-4-morpholin-4-yl-benzamide

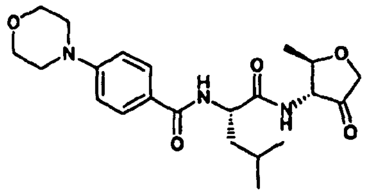

Following the procedure of Example 1 step d) except substituting "4-morpholin-4-yl-benzoic acid"

4-Morpholin-4-yl-benzoic acid methyl ester

Following the procedure above except substituting "morpholine" for "pyrrolidine" gave the title compound: MS (M+H+): 222.

a. 4-Morpholin-4-yl-benzoic acid salt

Following the procedure above except substituting "4-morpholin-4-yl-benzoic acid methyl ester" for "4-pyrrolidin-1-yl-benzoic acid methyl ester" gave the title compound: MS (M+H+): 208.

Preparation of N-[3-Methyl-1-(2-methyl-4-oxo-tetrahydro-furan-3-ylcarbamoyl)-butyl]-4-(4-methyl-piperazin-1-yl)-benzamide

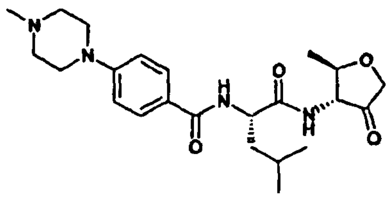

Following the procedure of Example 1 step d) except substituting "4-methyl-piperazin-1-yl-benzoic acid" for

4-Methyl-piperazin-1-yl-benzoic acid methyl ester

Following the procedure above except substituting "4-methyl-piperazine" for "pyrrolidine" gave the title compound: MS (M+H+): 235.

a. 4-Methyl-piperazin-1-yl-benzoic acid salt

Following the procedure above except substituting "4-methyl-piperazin-1-yl-benzoic acid methyl ester" for "4-pyrrolidin-1-yl-benzoic acid methyl ester" gave the title compound: MS (M+H+): 221.

Preparation of N-[3-Methyl-1-(2-methyl-4-oxo-tetrahydro-furan-3-ylcarbamoyl)-butyl]-4-(2-morpholin-4-yl-ethylamino)-benzamide

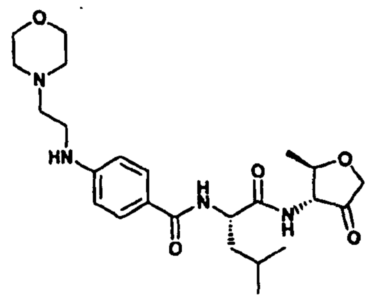

Following the procedure of Example 1 step d) except substituting "4-(2-morpholin-4-yl-ethylamino)-benzoic acid"

a. 4-(2-Morpholin-4-yl-ethylamino)-benzoic acid methyl ester

Following the procedure above except substituting "2-morpholin-4-yl-ethylamine" for "pyrrolidine" gave the title compound: MS (M+H+): 265.

b. 4-(2-Morpholin-4-yl-ethylamino)-benzoic acid salt

Following the procedure above except substituting "4-(2-Morpholin-4-yl-ethylamino)-benzoic acid methyl ester" for "4-pyrrolidin-1-yl-benzoic acid methyl ester" gave the title compound: MS (M+H+): 251.

Preparation of N-[3-Methyl-1-(2-methyl-4-oxo-tetrahydro-furan-3-ylcarbamoyl)-butyl]-4-piperazin-1-yl-benzamide

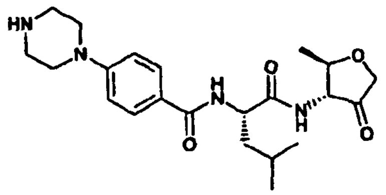

Following the procedure of Example 1 step d) except substituting "4-(4-carboxy-phenyl)-piperazine-1-carboxylic acid tert-butyl ester".

a. 4-(4-Methoxycarbonyl-phenyl)-piperazine-1-carboxylic acid tert-butyl ester

Following the procedure above except substituting "piperazine-1-carboxylic acid tert-butyl ester" for "pyrrolidine" gave the title compound: MS (M+H+): 321.

b. 4-(4-Carboxy-phenyl)-piperazine-1-carboxylic acid tert-butyl ester salt

Following the procedure above except substituting "4-(4-methoxycarbonylphenyl)-piperazine-1-carboxylic acid tert-butyl ester" for "4-pyrrolidin-1-yl-benzoic acid methyl ester" gave the title compound: MS (M+H+): 307.

Preparation of N-[3-Methyl-1-(2-methyl-4-oxo-tetrahydro-furan-3-ylcarbamoyl)-butyl]-3-pyrrolidin-1-yl-benzamide

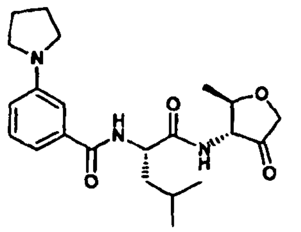

Following the procedure of Example 1 step d) except substituting "3-pyrrolidin-1-yl-benzoic acid" for "2-benzofuran-carboxylic acid."

a. 3-Pyrrolidin-1-yl-benzoic acid methyl ester

Following the procedure above except substituting "methyl-3-bromobenzoate° for "methyl-4-bromobenzoate" gave the title compound: MS (M+H+): 206.

b. 3-Pyrrolidin-1-yl-benzoic acid salt

Following the procedure above except substituting "3-pyrrolidin-1-yl-benzoic acid methyl ester" for "4-pyrrolidin-1-yl-benzoic acid methyl ester" gave the title compound: MS (M+H+): 192.

Preparation of N-[3-Methyl-1-(2-methyl-4-oxo-tetrahydro-furan-3-ylcarbamoyl)-butyl]-3-piperidin-1-yl-benzamide

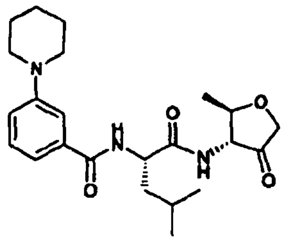

Following the procedure of Example 1 step d) except substituting "3-piperidin-1-yl-benzoic acid" for "2-benzofuran-carboxylic acid."

a. 3-Piperidin-1-yl-benzoic acid methyl ester

Following the procedure above except substituting "piperidine" for "pyrrolidine" and "methyl-3-bromobenzoate" for "methyl-4-bromobenzoate" gave the title compound: MS-(M+H+): 220.

b. 3-Piperidin-1-yl-benzoic acid salt

Following the procedure above except substituting "3-piperidin-1-yl-benzoic acid methyl ester" for "4-pyrrolidin-1-yl-benzoic acid methyl ester" gave the title compound: MS (M+H+): 206.

Preparation of N-[3-Methyl-1-(2-methyl-4-oxo-tetrahydro-furan-3-ylcarbamoyl)-butyl]-3-morpholin-4-yl-benzamide

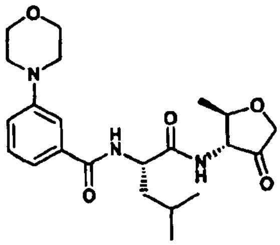

Following the procedure of Example 1 step d) except substituting "3-morpholin-4-yl-benzoic acid".

a. 3-Morpholin-4-yl-benzoic acid methyl ester

Following the procedure above except substituting "morpholine" for "pyrrolidine" and "methyl-3-bromobenzoate" for "methyl-4-bromobenzoate" gave the title compound: MS (M+H+): 222.

b. 3-Morpholin-4-yl-benzoic acid salt

Following the procedure above except substituting "3-morphotin-4-yl-benzoic acid methyl ester" for "3-pyrrolidin-1-yl-benzoic acid methyl ester" gave the title compound: MS (M+H+): 208.

Preparation of N-[3-Methyl-1-(2-methyl-4-oxo-tetrahydro-furan-3-ylcarbamoyl)-butyl]-3-(4-methyl-piperazin-1-yl)-benzamide

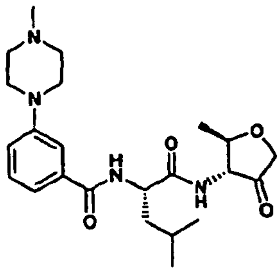

Following the procedure of Example 1 step d) except substituting "3-methylpiperazin-1-yl-benzoic acid" for "2-benzofuran-carboxylic acid."

a. 3-Methyl-piperazin-1-yl-benzoic acid methyl ester

Following the procedure above except substituting "4-methyl-piperazine" for "pyrrolidine" and "methyl-3-bromobenzoate" for "methyl-4-bromobenzoate" gave the title compound: MS (M+H+): 235.

b. 3-Methyl-piperazin-1-yl-benzoic acid salt

Following the procedure above except substituting "3-methyl-piperazin-1-yl-benzoic acid methyl ester" for "4-pyrrolidin-1-yl-benzoic acid methyl ester" gave the title compound: MS (M+H+): 221.

Preparation of N-[3-Methyl-1-(2-methyl-4-oxo-tetrahydro-furan-3-ylcarbamoyl)-butyl]-3-(2-morpholin-4-yl-ethylamino)-benzamide

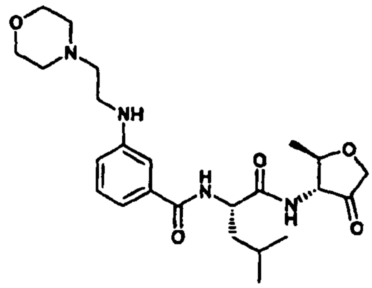

Following the procedure of Example 1 step d) except substituting "3-(2-morpholin-4-yl-ethylamino)-benzoic acid" for "2-benzofuran-carboxylic acid."

a. 3-(2-Morpholin-4-yl-ethylamino)-benzoic acid methyl ester

Following the procedure above except substituting "2-morpholin-4-yl-ethylamine" for "pyrrolidine" and "methyl-3-bromobenzoate" for "methyl-4-bromobenzoate" gave the title compound: MS (M+H+): 265.

b. 3-(2-Morpholino-4-yl-ethylamino)-benzoic acid salt

Following the procedure above except substituting "3-(2-Morphotin-4-yl-ethylamino)-benzoic acid methyl ester" for "4-pyrrolidin-1-yl-benzoic acid methyl ester" gave the title compound: MS (M+H+): 251.

Preparation of N-[3-Methyl-1-(2-methyl-4-oxo-tetrahydro-furan-3-ylcarbamoyl)-butyl]-3-piperazin-1-yl-benzamide

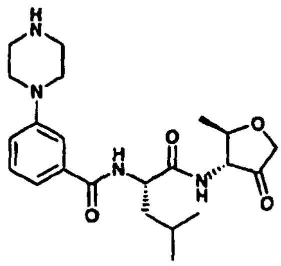

Following the procedure of Example 1 step d) except substituting "4-(3-carboxy-phenyl)-piparazine-1-carboxylic acid tert-butyl ester"

b. 3-(4-Methoxycarbonyt-phenyl)-piperazine-1-carboxylic acid tert-butyl ester

Following the procedure above except substituting "piperazine-1-carboxylic acid tert-butyl ester" for "pyrrolidine" and "methyl-3-bromobenzoate" for "methyl-4-bromobenzoate" gave the title compound: MS (M+H+): 321.

b. 4-(4-Carboxy-phenyl)-piperazine-1-carboxylic acid tert-butyl ester salt

Following the procedure above except substituting "4-(4-methoxycarbonylphenyl)-piperazine-1-carboxylic acid tert-butyl ester" for "4-pyrrolidin-1-yl-benzoic acid methyl ester" gave the title compound: MS (M+H+): 307.

Biological Examples

Determination of cathepsin K proteolytic catalytic activity

Convenient assays for cathepsin K are carried out using human recombinant enzyme. Standard assay conditions for the determination of kinetic constants used a fluorogenic peptide substrate, typically H-*D*-Ala-Leu-Lys-AMC, and were determined in either 100 mM Mes/Tris, pH 7.0 containing 1 mM EDTA and 10 mM 2-mercaptoethanol or 100

mM Na acetate, pH 5.5 containing 5 mM EDTA and 20 mM cysteine. The enzyme concentration used was 5 nM. The stock substrate solution was prepared at 10 mM in DMSO. Screens were carried out at a fixed substrate concentration of 60 μM and detailed kinetic studies with doubling dilutions of substrate from 250 μM. The total DMSO concentration in the assay was kept below 3%. All assays were conducted at ambient temperature. Product fluorescence (excitation at 390 nm, emission at 460 nm) was monitored with a Labsystems Fluoroskan Ascent fluorescent plate reader. Product progress curves were generated over 15 minutes following generation of AMC product.

Inhibition Studies

Potential inhibitors are screened using the above assay with variable concentrations of test compound. Reactions were initiated by addition of enzyme to buffered solutions of substrate and inhibitor. $K_i$ values were calculated according to equation 1

$$v_0 = \frac{VS}{K_M\left(1 + \frac{I}{K_i}\right) + S} \qquad (1)$$

where $v_0$ is the velocity of the reaction, $V$ is the maximal velocity, $S$ is the concentration of substrate with Michaelis constant of $K_M$, and I is the concentration of inhibitor.

In this assay the compounds depicted in Table I have a $K_i$ values at pH 7 in the range 10 nM to 250nM and are thus have utility in the treatment or prophylaxis of disorders in which cathepsin K is implicated, such as osteoporosis, gingival diseases such as gingivitis and periodontitis, Paget's disease, hypercalcaemia of malignancy, metabolic bone disease, osteoarthritis, rheumatoid arthritis, and metastatic neoplastias.

Cloning and expression of falcipain II

Generation of Faicipain 2

Cloning

The deoxyoligonucleotide primers:

(SEQ ID NO.: 1) 5'CGCGGATCCGCCACCATGGAATTAAACAGATTTGCCGAT-3' and
(SEQ ID NO.: 2) 5'CGCGTCGACTTAATGATGATGATGATGATGTTCAATTAATGGAATGAAT GCATCAGT-3' were designed based on sequences deposited at the Sanger Centre, Cambridge, UK (http://www.sanger.ac.uk/Projects/P falciparum/blast server.shtml). These primers were designed to amplify a portion of the cDNA sequence of the cysteinyl proteinase now known as Falcipain 2 and to include relevant terminal cloning enzymes sites and a carboxy-terminal hexahistidine coding sequence immediately upstream of the stop codon.

Polymerase chain reaction was performed with the above primers and *Plasmodium falciparum* phage library DNA as a template using the following conditions; 94°C for 2 minutes then 35 cycles of 94°C for 10 seconds, 50°C for 1 minute, and 60°C for 2 minutes, this was followed by a 60°C 5 minute incubation. The 880bp PCR amplicon was purified and phosphorylated using T4 polynucleotide kinase. This DNA was then ligated into EcoRV cleaved, dephosphorylated Bluescript II cloning vector and transformed into DH5 alpha *E.coli*. The DNA sequence of the plasmid inserts in isolated recombinant *E.coli* clones were determined using an Amersham Megabace sequencing instrument. To create an authentic ORF a three-way ligation was conducted bringing together the N-terminus of truncated falcipain-2 (Ncol/Ndel), the C-terminus of falcipain-2 (Ndel/BamH1) and the vector pQE-60 (Ncol/BamHl).

Nucleotide Sequence of TF2.10 (SEQ ID NO.: 3):

CCATGGAATTAAACAGATTTGCCGATTTAACTTATCATGAATTTAAAAACA
AATATCTTAGTTTAAGATCTTCAAAACCATTAAAGAATTCTAAATATTTATT
AGATCAAATGAATTATGAAGAAGTTATAAAAAAATATAGAGGAGAAGAAA
ATTTCGATCATGCAGCTTACGACTGGAGATTACACAGTGGTGTAACACCT
GTAAAGGATCAAAAAAATTGTGGATCTTGCTGGGCCTTTAGTAGTATAGG
TTCCGTAGAATCACAATATGCTATCAGAAAAAATAAATTAATAACCTTAAG
TGAACAAGAATTAGTAGATTGTTCATTTAAAAATTATGGTTGTAATGGAGG
TCTCATTAATAATGCCTTTGAGGATATGATTGAACTTGGAGGTATATGTCC

AGATGGTGATTATCCATATGTGAGTGATGCTCCAAATTTATGTAACATAG
ATAGATGTACTGAAAAATATGGAATCAAAAATTATTTATCCGTACCAGATA
ATAAATTAAAAGAAGCACTTAGATTCTTGGGACCTATTAGTATTAGTGTAG
CCGTATCAGATGATTTTGCTTTTTACAAAGAAGGTATTTTCGATGGAGAAT
GTGGTGATGAATTAAATCATGCCGTTATGCTTGTAGGTTTTGGTATGAAA
GAAATTGTTAATCCATTAACCAAGAAAGGAGAAAAACATTATTATTATATA
ATTAAGAACTCATGGGGACAACAATGGGGAGAAAGAGGTTTCATAAATAT
TGAAACAGATGAATCAGGATTAATGAGAAAATGTGGATTAGGTACTGATG
CATTCATTCCATTAATTGAACATCATCATCATCATCATTAAGTCGACGCGA
TCGAATTCCTGCAGCCCGGGGATCC

Coding for the Protein Sequence (SEQ ID NO.: 4):

MELNRFADLTYHEFKNKYLSLRSSKPLKNSKYLLDQMNYEEVIKKYRGEENF
DHAAYDWRLHSGVTPVKDQKNCGSCWAFSSIGSVESQYAIRKNKLITLSEQ
ELVDCSFKNYGCNGGLINNAFEDMIELGGICPDGDYPYVSDAPNLCNIDRCT
EKYGIKNYLSVPDNKLKEALRFLGPISISVAVSDDFAFYKEGIFDGECGDELN
HAVMLVGFGMKEIVNPLTKKGEKHYYYIIKNSWGQQWGERGFINIETDESGL
MRKCGLGTDAFIPLIEHHHHHH.

The TF2.10 insert was excised from the pQE-60 vector using the restriction enzymes NcoI and BamHI, ligated into NcoI/BamHI cut expression vector pET-11D and transformed into DH5 alpha *E.coli.* The presence of a recombinant expression plasmid (pET-TF2.10) in an isolated *E.coli* colony was confirmed by restriction enzyme digest of plasmid DNA. BL21 (DE3) *E.coli* were transformed with pET-TF2.10 and used for expression of the recombinant cysteinyl proteinase.

Protein Expression

pET-TF2.10-Transformed BL21(DE3) *E.coli* (BLTF2.10) were grown up overnight at 200 rpm. 37°C in Luria broth containing 100 μg/ml ampicillin. Fresh medium was then inoculated and grown to an $OD_{600nm}$ of 0.8 before protein expression was induced using 1 mM IPTG. Induction was performed for 3 hours at 200 rpm, 37°C then the bacterial cells harvested by centrifugation and stored at -80°C until protein purification performed.

Protein Purification and Refolding

An *E.coli* cell pellet equivalent to 250ml culture was lysed by resuspension in solubilisation buffer (6M guanidine hydrochloride, 20mM Tris-HCl, 250mM NaCl, 20mM imidazole, pH8.0) for 30 minutes at room temperature. After centrifugation at 12000g for 10 minutes at 4°C the cleared lysate was applied to 1 ml nickel-NTA agarose, and agitated for 1 hour at room temperature.

Protein Refolding Method 1

The protein bound to nickel-NTA was batch washed with 6M guanidine hydrochloride, 20mM Tris-HCl, pH 8.0. 250mM NaCl then 8M urea, Tris-HCl, pH 8.0, 500mM NaCl then 8M urea, Tris-HCl, pH 8.0 including 30 mM imidazole and protein elution performed using 8M urea, Tris-HCl, pH 8.0 with 1 M imidazole. The eluted protein was then diluted 100 fold in refolding buffer (100mM Tris-HCl, 1mM EDTA, 20% glycerol, 250mM L-arginine, 1mM reduced glutathione, 0.1mM oxidised glutatione, pH8.0) and left stirring overnight at 4°C. The protein could then be concentrated either by filter centrifugation or repurification using a nickel-agarose column (after dialysis to remove the EDTA).

Protein Refolding Method 2

The protein bound to nickel-NTA was batch washed with 8M urea, Tris-HCl, 500mM NaCl, pH 8.0 then 8M urea, Tris-HCl, pH 8.0 including 20 mM imidazole, then 2M urea, Tris-HCl, pH 8.0. The protein was then refolded on the column by the addition of 100mM Tris-HCl, pH8.0, 250mM L-arginine, 1mM reduced glutathione, 0.1mM oxidised glutatione with incubation at 4°C and protein elution performed using, 100mM Tris-HCl, pH 8.0 with 0.5 M imidazole.

Immediately active (mature) proteinase was obtained using protein refolding method 1 and concentrating the dilute refolded enzyme by filter centrifugation.This method, however, did result in a large degree of enzyme loss due to autoproteolysis. Both concentrating the protein refolded using method 1 by nickel column purification and using refolding method 2 resulted in greater recovery of the enzyme in its stable inactive pro-form. The pro-form could also be used to generate mature active falcipain 2, after incubation at 37°C.

The C-tagged construct outlined above enables rapid concentration and recovery after protein refolding and circumvents problems with autoproteolysis. Unlike the N-tagged constructs described in Shenai et al J Biol Chem 275 37 29000-29010m the constructs described here can be refolded on the surface of an insoluble matrix, for instance bound to a purification column. Additionally the proregion can act as an enzyme inactivating sequence analogous to the native enzyme making work with the enzyme more predictable ie the stable inactive enzyme can be controllably activated when needed. An N-terminal tag would tend to prevent this normal functioning of the enzyme, as the proregion of the enzyme ought to be able to fold independently to direct the folding state of the mature enzyme domain. The tag described herein allows affinity purification to increase yields and enhance opportunities to isolate stable proforms of the enzyme.

Accordingly there is described an enzymatically active falcipain 2 construct comprising a covalently bonded C-terminal tag. The C-terminal tag may comprise polyhistidine, for example 4-8 residues, preferably 6. The construct described above has the tag at the C terminal of glutamic acid residue, but other constructs can shorten the enzyme by up to 10, for example 6-8 or 2-4 residues and retain a useful screening activity. Preferred constructs comprise the sequence enumerated above, optionally truncated at the C terminal as described herein.

Determination of falcipain 2 proteolytic catalytic activity

Convenient assays for falcipain 2 are carried out using recombinant enzyme prepared above. Alternatively, falcipain is assayed as described in Sijwali et al Prot Exp Purif 22, 128-134 (2001). Standard assay conditions for the determination of kinetic constants used a fluorogenic peptide substrate, typically Boc-Val-Leu-Lys-AMC, and were determined in either 100 mM Mes/Tris/acetate, pH 7.0 containing 1 M NaCl and 10 mM 2-mercaptoethanol or 100 mM Na phosphate, pH 5.5 containing 1 M NaCl and 10 mM 2-mercaptoethanol. The enzyme concentration used was 2 nM. The stock substrate solution was prepared at 10 mM in DMSO. Screens were carried out at a fixed substrate concentration of 80 μM and detailed kinetic studies with doubling dilutions of substrate from 250 μM. The total DMSO concentration in the assay was kept below 3%. All assays were conducted at ambient temperature. Product fluorescence (excitation

at 390 nm, emission at 460 nm) was monitored with a Labsystems Fluoroskan Ascent fluorescent plate reader. Product progress curves were generated over 15 minutes following generation of AMC product.

Inhibition Studies

Potential inhibitors were screened using the above assay with variable concentrations of the compounds in the table below. These compounds were prepared on solid phase using the methodology outlined above. Reactions were initiated by addition of enzyme to buffered solutions of substrate and inhibitor. $K_i$ values were calculated according to equation 1

$$v_0 = \frac{VS}{K_M\left(1+\frac{I}{K_i}\right)+S} \quad (1)$$

where $v_0$ is the velocity of the reaction, V is the maximal velocity, S is the concentration of substrate with Michaelis constant of $K_M$, and / is the concentration of inhibitor.

The compounds depicted in the table below above showed $K_i$ values (at pH 7) between 0.5 μM and 2.7μM and are thus useful in the prophylaxis or treatment of parasite infections or infestations, such as malaria.

Naphthalene-2-carboxylic-acid-[3-methyl-1S-(2R-methyl-4-oxo-tetrahydrofuran-3S-ylcarbamoyl)-butyl]-amide
Benzofuran-2-carboxylic-acid-[3-methyl-1S-(2R-methyl-4-oxo-tetrahydrofuran-3S-ylcarbamoyl)-butyl]-amide
5-Methoxy-benzofuran-2-carboxylic-acid-[3-methyl-1S-(2R-methyl-4-oxo-tetrahydro-furan-3S-ylcarbamoyl)-butyl]-amide
4-Acetylamino-*N*-[3-methyl-1S-(2R-methyl-4-oxo-tetrahydrofuran-3S-ylcarbamoyl)-butyl]-benzamide
4-Hydroxy-*N*-[3-methyl-1S-(2R-methyl-4-oxo-tetrahydro-furan-3S-ylcarbamoyl)-butyl]-3-morpholin-4-ylmethyl-benzamide
Biphenyl-4-carboxylic-acid-[3-methyl-1S-(2R-methyl-4-oxo-tetrahydrofuran-3S-ylcarbamoyl)-butyl]-amide
4-*tert*-Butyl-*N*-[3-methyl-1S-(2R-methyl-4-oxo-tetrahydro-furan-3S-ylcarbamoyl)-butyl]-benzamide
Benzothiazole-5-carboxylic-acid-[3-methyl-1S-(2R-methyl-4-oxo-tetrahydrofuran-3S-ylcarbamoyl)-butyl]-amide

## Claims

1. A compound of the formula (IVa):

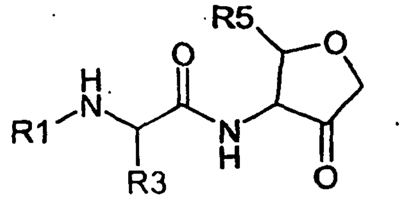

wherein
R1 = R'C(O), R' SO2,
R' = a bicyclic, saturated or unsaturated, 8-12 membered ring system containing 0-4 hetero atoms selected from S, O and N, which ring system is optionally substituted with up to four substituents independently selected from groups a), b) and c) below; or
R'= a monocyclic, saturated or unsaturated, 5-7 membered ring containing 0-3 hetero atoms selected from S, O and N, which monocyclic ring bears at least one substituent selected from group a) and c) below and which may optionally bear one or two further substituents selected from group b) below;

a) a cyclic group which may be linked direct to the R' ring or via an alkyl, alkylether, alkylthioether, alkylamine, alkylamide, alkylsulphonamide, alkylsulphone, alkylurea, alkylketone or alkylester linker comprising up to 6 C atoms; or
b) H, $C_{1-7}$alkyl, $C_{3-6}$-cycloalkyl, OH, SH, $NH_2$, $NHC_{1-3}$-alkyl, $N(C_{1-3}\text{-alkyl})_2$, halogen; or
c) O-$C_{1-4}$-alkyl, S-$C_{1-4}$-alkyl, $SOC_{1-4}$-alkyl, $SO_2C_{1-4}$-alkyl, $CO_2C_{0-4}$-alkyl, $NHCOC_{0-4}$-alkyl, $CONHC_{0-4}$-alkyl, $COC_{0-4}$-alkyl, $NHC(=NH)NH_2$;

R3 = $C_{1-7}$-alkyl, $C_2$-$C_7$-alkenyl, $C_{3-7}$-cycloalkyl, Ar-$C_{1-7}$-alkyl, Ar;
R5 = $C_{1-7}$-alkyl, Ar-$C_{1-7}$-alkyl, $C_{0-3}$-alkyl-CONR3R4 or $R^{iv}$;
$R^{iv}$=

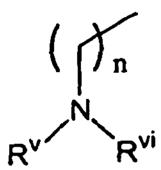
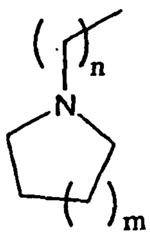
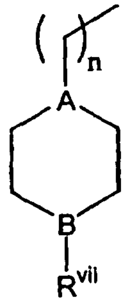
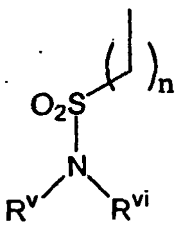
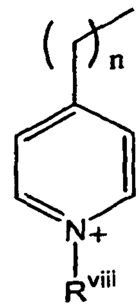

wherein
n = 1-3,
m = 1-3;
$R^v$, $R^{vi}$ = H, $C_{1-7}$-alkyl;
A = N, CH;
B = N, O, S, CH;
$R^{vii}$ = absent when B = O, S; or
$R^{vii}$ = H, $C_{1-7}$-alkyl when B = N, CH;
$R^{viii}$ = O, $C_{1-7}$-alkyl;
and pharmaceutically acceptable salts thereof, wherein
$C_1$-$C_7$ alkyl means straight and branched chain aliphatic carbon chains, optionally substituted by one or two halogens and/or a heteroatom S, O, NH, where the heteroatom at a chain terminus may be substituted with 1 or 2 hydrogen atoms or a sulphur oxidised to the sulphone;

$C_{3-6}$ or $C_{3-7}$ cycloalkyl means any variation of $C_1$-$C_7$ alkyl which additionally contains a $C_{3-6}$ or $C_3$-$C_7$ carbocyclic ring or the cycloalkyl is spiro bound to the adjacent carbon without an intervening $C_1$-$C_7$ alkyl;
Ar means phenyl, pyrazolyl, pyridyl, imidazolyl, oxazolyl, isoxazolyl, thiazinolyl, isothiazinolyl, thiazolyl, oxadiazolyl, 1,2,3-triazolyl, 1,2,4-triazolyl, furanyl or thienyl aromatic ring, optionally substituted with halogen, $C_{1-3}$-alkyl, OH, $OC_{1-3}$-alkyl, SH, $SC_{1-3}$-alkyl, amine;
$ArC_{1-7}$-alkyl means Ar attached to the respective carbon atom through a $C_1$-$C_7$ alkyl

**2.** A compound according to claim 1, wherein the R' bicyclic ring is selected from naphthyl, quinolyl, benzofuranyl, benzothienyl, indolyl, indolinyl.

**3.** A compound according to claim 2, wherein the linkage is the 2 position of the R' ring.

**4.** A compound according to claim 1, wherein R' is substituted with morpholine or N-methylpiperidine linked through an alkyl or alkylether linkage.

**5.** A compound according to claim 1, wherein R1 is R'C(O).

**6.** A compound according to claim 1, wherein R3 is 2-methylprop-1-enyl, benzyl or especially i-butyl.

**7.** A compound according to claim 1, wherein the stereochemistry at R3 corresponds to a natural or n on natural L-amino acid.

**8.** A compound according to claim 1, wherein R5 is $CH_3$, $C_2H_5$, $CH_2Ar$, $CH_2CONH_2$, $(CH_2)_2CONH_2$, $CH_2OH$,

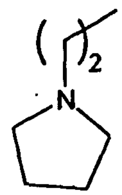
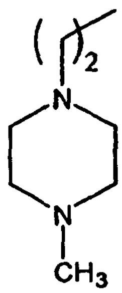
,

$CH_3$, $CH_2CH_3$, and $CH_2OH$ being particularly preferred.

**9.** A compound according to claim 1, wherein R5 and the $C_4$ bond both have (R) or both have (S) stereochemistry.

**10.** A compound according to claim 1 or 5-9, wherein R' is a monocyclic ring substituted with a cyclic substituent, preferably a non aromatic substituent, in particular wherein the nonaromatic cyclic substituent is selected from the group consisting of pyrrolidine-1-yl, piperidine-1-yl, morpholin-4-yl, 4-methylpiperazin-1-yl, 2-morpholin-4-yl-ethylamino, and piperazin-1-yl, the monocyclic ring ring preferably being pyridyl, pyrimidinyl or phenyl which is preferably substituted in the 3 or 4 position.

**11.** Use of a compound as defined in claim in the manufacture of a medicament for the treatment or prophylaxis of disorders dependent upon the activity of cathepsin K.

**12.** The use according to claim 11, wherein the disorder is a bone disorder such as periodontitis or osteoarthritis, a cartilage or matrix degradation disorder such as osteoarthritis or rheumatoid arthritis, or neoplasia.

**13.** Use of a compound as defined in claim 1 in the manufacture of a medicament for the treatment or prophylaxis of a parasite infection.

## Revendications

**1.** Composé de formule (IVa) :

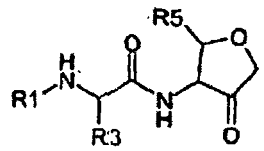

dans laquelle
R1= R'C(O), R'SO2,
R'= un système de noyau bicyclique à 8-12 chaînons, saturé ou insaturé, contenant 0-4 hétéroatomes choisis parmi S, O et N, lequel système de noyau est éventuellement substitué avec jusqu'à quatre substituants choisis de manière indépendante dans les groupes a), b) et c) ci-dessous ; ou
R'= un noyau monocyclique à 5-7 chaînons, saturé ou insaturé, contenant 0-3 hétéroatomes choisis parmi S, O et N, lequel noyau monocyclique porte au moins un substituant choisi dans le groupe a) et c) ci-dessous et qui peut éventuellement porter un ou deux substituants supplémentaires choisis dans le groupe b) ci-dessous ;

a) un groupe cyclique qui peut être lié directement au noyau R' ou via un lieur alkyle, alkyléther, alkylthioéther, alkylamine, alkylamide, alkylsulfamide, alkylsulfone, alkylurée, alkylcétone ou alkylester, comprenant jusqu'à 6 atomes de carbone ; ou
b) H, $C_{1-7}$alkyle, $C_{3-6}$-cycloalkyle, OH, SH, $NH_2$, $NHC_{1-3}$-alkyle, $N(C_{1-3}$-alkyl)2, halogène ; ou
c) O-$C_{1-4}$-alkyle, S-$C_{1-4}$alkyle, $SOC_{1-4}$-alkyle, $SO_2C_{1-4}$-alkyle, $CO_2C_{0-4}$-alkyle, $NHCOC_{0-4}$-alkyle, $CONHC_{0-4}$-alkyle, $COC_{0-4}$-alkyle, NHC(=NH)$NH_2$;

R3= $C_{1-7}$-alkyle, $C_2$-$C_7$-alkényle, $C_{3-7}$-cycloalkyle, Ar-$C_{1-7}$-alkyle, Ar;
R5= $C_{1-7}$-alkyle, Ar-$C_{1-7}$-alkyle, $C_{0-3}$-alkyle-CONR3R4 ou $R^{iv}$;
$R^{iv}$=

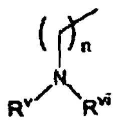 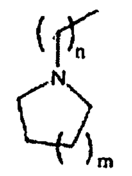 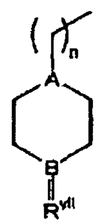 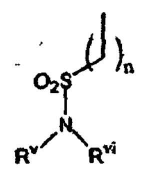 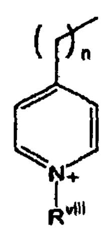

dans lequel
n= 1-3 ;
m=1-3 ;
$R^{v}$, $R^{vi}$= H, $C_{1-7}$-alkyle;
A= N, CH;
B= N, O, S, CH;
$R^{vii}$= absent lorsque B=O, S ; ou
$R^{vii}$= H, $C_{1-7}$-alkyle lorsque B=N, CH;
$R^{viii}$= O, $C_{1-7}$-alkyle;
et ses sels acceptables sur le plan pharmaceutique, dans lequel
$C_1$-$C_7$ alkyle indique des chaînes carbonées aliphatiques à chaîne linéaire ou branchée, éventuellement substituées par un ou deux halogènes et/ou un hétéroatome S, O, NH, où l'hétéroatome, à une terminaison de chaîne, peut être substitué par 1 ou 2 atomes d'hydrogène ou un soufre oxydé en sulfone ;
$C_{3-6}$ ou $C_{3-7}$ cycloalkyle indique toute variation d'un $C_1$-$C_7$ alkyle qui contient en plus un noyau carbocyclique en $C_{3-6}$ ou $C_3$-$C_7$ ou le cycloalkyle est lié en spiro au carbone adjacent sans $C_1$-$C_7$ alkyle intermédiaire ;
Ar indique un noyau aromatique phényle, pyrazolyle, pyridyle, imidazolyle, oxazolyle, isoxazolyle, thiazinolyle, isothiazinolyle, thiazolyle, oxadiazolyle, 1,2,3-triazolyle, 1,2,4-triazolyle, furannyle ou thiényle, éventuellement substitué par halogène, $C_{1-3}$-alkyle, OH, $OC_{1-3}$-alkyle, SH, $SC_{1-3}$-alkyle, amine ;

$ArC_{1-7}$-alkyle indique Ar attaché à l'atome de carbone correspondant par un $C_1$-$C_7$ alkyle.

2. Composé selon la revendication 1, dans lequel le noyau bicyclique R' est choisi parmi naphtyle, quinolyle, benzofuranyle, benzothiényle, indolyle, indolinyle.

3. Composé selon la revendication 2, dans lequel la liaison est en position 2 du noyau R'.

4. Composé selon la revendication 1, dans lequel R' est substitué par la morpholine ou la N-méthylpipéridine liée par une liaison alkyle ou alkyléther.

5. Composé selon la revendication 1, dans lequel R1 est R'C(O).

6. Composé selon la revendication 1, dans lequel R3 est le 2-méthylprop-1-ényle, le benzyle ou particulièrement l'i-butyle.

7. Composé selon la revendication 1, dans lequel la stéréochimie au niveau de R3 correspond à un L-amino acide naturel ou non naturel.

8. Composé selon la revendication 1, dans lequel R5 est $CH_3$, $C_2H_5$, $CH_2Ar$, $CH_2CONH_2$, $(CH_2)_2CONH_2$, $CH_2OH$,

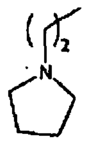

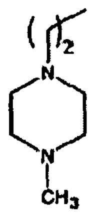,

$CH_3$, $CH_2CH_3$ et $CH_2OH$ étant particulièrement préférés.

9. Composé selon la revendication 1, dans lequel R5 et la liaison $C_4$ ont tous les deux une stéréochimie (R) ou ont tous les deux une stéréochimie (S).

10. Composé selon la revendication 1 ou 5-9, dans lequel R' est un noyau monocyclique substitué par un substituant cyclique, de préférence un substituant non aromatique, en particulier dans lequel le substituant cyclique non aromatique est choisi dans le groupe constitué de pyrrolidine-1-yl, pipéridine-1-yl, morpholin-4-yl, 4-méthylpipérazin-1-yl, 2-morpholin-4-yl-éthylamino et pipérazin-1-yl, le noyau monocyclique étant de préférence pyridyle, pyrimidinyle ou phényle qui est de préférence substitué à la position 3 ou 4.

11. Utilisation d'un composé tel que défini dans la revendication 1 dans la fabrication d'un médicament pour le traitement ou la prophylaxie de désordres dépendant de l'activité de la cathepsine K.

12. Utilisation selon la revendication 11, dans laquelle le désordre est un désordre osseux tel que la parodontite ou l'ostéo-arthrite, un désordre cartilagineux ou de dégradation de la matrice tel que l'ostéo-arthrite ou la polyarthrite rhumatoïde, ou une néoplasie.

13. Utilisation d'un composé tel que défini dans la revendication 1 dans la fabrication d'un médicament pour le traitement ou la prophylaxie d'une infection parasitaire.

**Patentansprüche**

1. Verbindung der Formel (IVa):

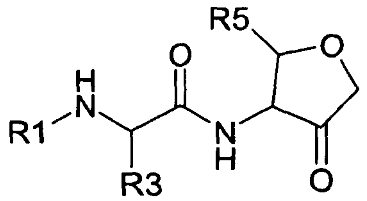

wobei
R1 = R'C(O), R'SO2,
R' = ein bizyklisches, gesättigtes oder ungesättigtes, 8-12-gliedriges Ringsystem, enthaltend 0-4 Heteroatome, die aus S, O und N ausgewählt sind, wobei das Ringsystem gegebenenfalls mit bis zu vier Substituenten substituiert ist, die unabhängig voneinander aus den Gruppen a), b) und c) unten ausgewählt sind; oder
R'= ein monozyklischer, gesättigter oder ungesättigter, 5-7-gliedriger Ring, enthaltend 0-3 Heteroatome, die aus S, O und N ausgewählt sind, wobei der monozyklische Ring wenigstens einen Substituenten trägt, der aus Gruppe a) und c) unten ausgewählt ist und der gegebenenfalls einen oder zwei weitere Substituenten trägt, die aus der Gruppe b) unten ausgewählt sind;

a) eine zyklische Gruppe, die direkt an den R'-Ring oder mittels eines Alkyl-, Alkylether-, Alkylthioether-, Alkylamin-, Alkylamid-, Alkylsulfonamid-, Alkylsulphon-, Alkylharnstoff , Alkylketon- oder Alkylester-Linker umfassend bis zu 6 C-Atome gebunden sein kann; oder
b) H, $C_{1-7}$-Alkyl, $C_{3-6}$-Cycloalkyl, OH, SH, $NH_2$, $NHC_{1-3}$-Alkyl, $N(C_{1-3}\text{-Alkyl})_2$, Halogen;
oder
c) O-$C_{1-4}$-Alkyl, S-$C_{1-4}$-Alkyl, $SOC_{1-4}$-Alkyl, $SO_2C_{1-4}$-Alkyl, $CO_2C_{0-4}$-Alkyl, $NHCOC_{0-4}$-Alkyl, $CONHC_{0-4}$-Alkyl, $COC_{0-4}$-Alkyl, $NHC(=NH)NH_2$;

R3 = $C_{1-7}$-Alkyl, $C_2$-$C_7$-Alkenyl, $C_{3-7}$-Cycloalkyl, Ar-$C_{1-7}$-Alkyl, Ar;
R5 = $C_{1-7}$-Alkyl, Ar-$C_{1-7}$-Alkyl, $C_{0-3}$-Alkyl-CONR3R4 oder $R^{iv}$;
$R^{iv}$=

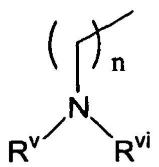

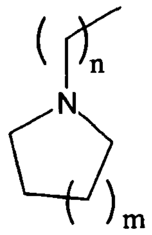

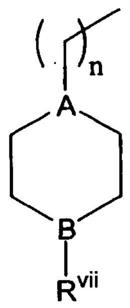

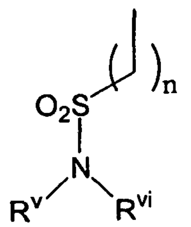

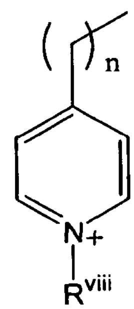

wobei
n = 1-3,
m =1-3;
$R^v$, $R^{vi}$ = H, $C_{1-7}$-alkyl;
A = N, CH;
B = N, O, S, CH;
$R^{vii}$ = fehlt wenn B = O, S; oder
$R^{vii}$ = H, $C_{1-7}$-Alkyl wenn B = N, CH;
$R^{viii}$ = O, $C_{1-7}$-Alkyl;
und pharmazeutisch verträgliche Salze davon, wobei,
$C_1$-$C_7$-Alkyl unverzweigte und verzweigt-kettige aliphatische Kohlenstoffketten bedeutet, gegebenenfalls substituiert mit einem oder zwei Halogenen und/oder einem Heteroatom S, O, NH, wo das Heteroatom an einem Kettenende mit 1 oder 2 Wasserstoffatomen oder einem Schwefel, der zum Sulfon oxidiert wurde, substituiert sein kann;

$C_{3-6}$ oder $C_{3-7}$-Cycloalkyl jede Variation von $C_1$-$C_7$-Alkyl bedeutet, das zusätzlich einen $C_{3-6}$- oder $C_3$-$C_7$-Carbozyklischen Ring beinhaltet, oder das Cycloalkyl ein Spirorest ist, der an das benachbarte Kohlenstoffatom ohne ein dazwischen liegendes $C_1$-$C_7$-Alkyl gebunden ist;
Ar bedeutet Phenyl-, Pyrazolyl-, Pyridyl-, Imidazolyl-, Oxazolyl-, Isoxazolyl-, Thiazinolyl-, Isothiazinolyl-, Thiazolyl-, Oxadiazolyl-, 1,2,3-Triazolyl-, 1,2,4-Triazolyl-, Furanyl- oder Thienyl-aromatischer Ring, gegebenenfalls substituiert mit Halogen, $C_{1-3}$-Alkyl, OH, $OC_{1-3}$-Alkyl, SH, $SC_{1-3}$-Alkyl, Amin;
$ArC_{1-7}$-Alkyl bedeutet, dass das Ar an das entsprechende Kohlenstoffatom durch eine $C_1$-$C_7$-Alkylgruppe angebracht ist.

**2.** Verbindung gemäß Anspruch 1, wobei der bizyklische Ring R' ausgewählt wird aus Naphthyl, Quinolyl, Benzofuranyl, Benzothienyl, Indolyl, Indolinyl.

**3.** Verbindung gemäß Anspruch 2, wobei die Bindung die 2-Position des R'-Rings ist.

**4.** Verbindung gemäß Anspruch 1, wobei R' durch Morpholin oder N-Methylpiperidin, das durch eine Alkyl- oder Alkylether-Bindung gebunden ist, substituiert ist.

**5.** Verbindung gemäß Anspruch 1, wobei R1 R'C(O) ist.

**6.** Verbindung gemäß Anspruch 1, wobei R3 2-Methylprop-1-enyl, Benzyl oder insbesondere i-Butyl ist.

**7.** Verbindung gemäß Anspruch 1, wobei die Stereochemie an R3 einer natürlichen oder nicht-natürlichen L-Aminosäure entspricht.

**8.** Verbindung gemäß Anspruch 1, wobei R5 $CH_3$, $C_2H_5$, $CH_2Ar$, $CH_2CONH_2$, $(CH_2)_2CONH_2$, $CH_2OH$,

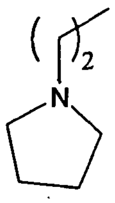

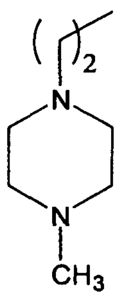
,

$CH_3$, $CH_2CH_3$, und wobei $CH_2OH$ insbesondere bevorzugt ist.

**9.** Verbindung gemäß Anspruch 1, wobei R5 und die $C_4$-Bindung beide (R) oder beide (S) Stereochemie aufweisen.

**10.** Verbindung gemäß der Ansprüche 1 oder 5-9, wobei R' ein monozyklischer Ring ist, der mit einem zyklischen Substituenten substituiert ist, vorzugsweise einem nichtaromatischen Substituenten, insbesondere wobei der nichtaromatische zyklische Substituent ausgewählt wird aus der Gruppe, die aus Pyrrolidin-1-yl, Piperidin-1-yl, Morpholin-4-yl, 4-Methylpiperazin-1-yl, 2-Morpholin-4-yl-ethylamino und Piperazin-1-yl ausgewählt wird, wobei der monozyklische Ring vorzugsweise Pyridyl, Pyrimidinyl oder Phenyl ist, das vorzugsweise an der 3- oder 4-Position substituiert ist.

**11.** Verwendung einer Verbindung wie in Anspruch 1 definiert für die Herstellung eines Medikaments zur Behandlung oder Prophylaxe von Erkrankungen, die von der Aktivität von Kathepsin K abhängig sind.

**12.** Verwendung gemäß Anspruch 11, wobei die Erkrankung eine Knochenerkrankung wie Periodontitis oder Osteoarthritis ist, eine Knorpel- oder Matrix-Degradationserkrankung wie Osteoarthritis oder rheumatoide Arthritis, oder Neoplasie ist.

**13.** Verwendung einer Verbindung wie in Anspruch 1 definiert, für die Herstellung eines Medikaments für die Behandlung

oder Prophylaxe einer Parasiteninfektion.

SEQUENCE LISTING

<110> Medivir AB
<120> Cysteine Protease Inhibitors
<130> 1718-196FPC

[illegible] 60/252,802

<151> 2000-11-17
<150> US 60/252.840

<151> 2000-11-17

<160> 4
<170> Patentin version 3.0
<210> 1

[illegible]

<212> DNA
<213> artificial sequence

<220>
<222>primer for cDNA of cysteinyl proteinase (Falcipain 2)
<400> 1

```
cgcggatccg ccaccatgga attaaacaga tttgccgat                              39
```

<211> 57
<212> DNA
<213> artificial sequence

<223> Primer for cDNA of cysteinyl proteinase (Falcipain 2)
<400> 2

```
cgcgtcgact taatgatgat gatgatgatg ttcaattaat ggaatgaatg catcagt          57
```

<210> 3
<211> 886
<212> DNA
<213> artificial sequence
<220>
<223> PCR product from amplification using primers for the cDNA sequenc e of cysteinyl proteinase (Falcipain 2)

<221> CDS
<222> (3)..(848)
<400> 3

```
cc atg gaa tta aac aga ttt gcc gat tta act tat cat gaa ttt aaa          47
   Met Glu Leu Asn Arg Phe Ala Asp Leu Thr Tyr His Glu Phe Lys
   1               5                   10                  15

aac aaa tat ctt agt tta aga tct tca aaa cca tta aag aat tct aaa         95
Asn Lys Tyr Leu Ser Leu Arg Ser Ser Lys Pro Leu Lys Asn Ser Lys
                20                  25                  30

tat tta tta gat caa atg aat tat gaa gaa gtt ata aaa aaa tat aga        143
Tyr Leu Leu Asp Gln Met Asn Tyr Glu Glu Val Ile Lys Lys Tyr Arg
            35                  40                  45

gga gaa gaa aat ttc gat cat gca gct tac gac tgg aga tta cac agt        191
Gly Glu Glu Asn Phe Asp His Ala Ala Tyr Asp Trp Arg Leu His Ser
        50                  55                  60

ggt gta aca cct gta aag gat caa aaa aat tgt gga tct tgc tgg gcc        239
Gly Val Thr Pro Val Lys Asp Gln Lys Asn Cys Gly Ser Cys Trp Ala
```

65 70 75

ttt agt agt ata ggt tcc gta gaa tca caa tat gct atc aga aaa aat 287
Phe Ser Ser Ile Gly Ser Val Glu Ser Gln Tyr Ala Ile Arg Lys Asn
80 85 90 95

aaa tta ata acc tta agt gaa caa gaa tta gta gat tgt tca ttt aaa 335
Lys Leu Ile Thr Leu Ser Glu Gln Glu Leu Val Asp Cys Ser Phe Lys
100 105 110

aat tat ggt tgt aat gga ggt ctc att aat aat gcc ttt gag gat atg 383
Asn Tyr Gly Cys Asn Gly Gly Leu Ile Asn Asn Ala Phe Glu Asp Met
115 120 125

att gaa tta gga ggt ata tgt cca gat ggt gat tat cca tat gtg agt 431
Ile Glu Leu Gly Gly Ile Cys Pro Asp Gly Asp Tyr Pro Tyr Val Ser
130 135 140

gat gct cca aat tta tgt aac ata gat aga tgt act gaa aaa tat gga 479
Asp Ala Pro Asn Leu Cys Asn Ile Asp Arg Cys Thr Glu Lys Tyr Gly
145 150 155

atc aaa aac tat tta tcc gta cca gat aat aaa tta aaa gaa gca ctt 527
Ile Lys Asn Tyr Leu Ser Val Pro Asp Asn Lys Leu Lys Glu Ala Leu
160 165 170 175

aga ttc ttg gga cct att agt att agt gta gcc gta tca gat gat ttt 575
Arg Phe Leu Gly Pro Ile Ser Ile Ser Val Ala Val Ser Asp Asp Phe
180 185 190

gct ttt tac aaa gaa ggt att ttc gat gga gaa tgt ggt gat gaa tta 623
Ala Phe Tyr Lys Glu Gly Ile Phe Asp Gly Glu Cys Gly Asp Glu Leu
195 200 205

aat cat gcc gtt atg ctt gta ggt ttt ggt atg aaa gaa att gtt aat 671
Asn His Ala Val Met Leu Val Gly Phe Gly Met Lys Glu Ile Val Asn
210 215 220

cca tta acc aag aaa gga gaa aaa cat tat tat tat ata att aag aac 719
Pro Leu Thr Lys Lys Gly Glu Lys His Tyr Tyr Tyr Ile Ile Lys Asn
225 230 235

tca tgg gga caa caa tgg gga gaa aga ggt ttc ata aat att gaa aca 767
Ser Trp Gly Gln Gln Trp Gly Glu Arg Gly Phe Ile Asn Ile Glu Thr
240 245 250 255

gat gaa tca gga tta atg aga aaa tgt gga tta ggt act gat gca ttc 815
Asp Glu Ser Gly Leu Met Arg Lys Cys Gly Leu Gly Thr Asp Ala Phe
260 265 270

att cca tta att gaa cat cat cat cat cat cat taagtcgacg cgatcgaatt 868
Ile Pro Leu Ile Glu His His His His His His
275 280

cctgcagccc ggggatcc 886

<210> 4
<211>282
<212> PRT
<213> artificial
<400> 4

Met Glu Leu Asn Arg Phe Ala Asp Leu Thr Tyr His Glu Phe Lys Asn
1 5 10 15

Lys Tyr Leu Ser Leu Arg Ser Ser Lys Pro Leu Lys Asn Ser Lys Tyr
20 25 30

Leu Leu Asp Gln Met Asn Tyr Glu Glu Val Ile Lys Lys Tyr Arg Gly
35 40 45